(19) **Europäisches Patentamt**

**European Patent Office**

**Office européen des brevets**

(11) **EP 1 043 334 A1**

(12) **EUROPEAN PATENT APPLICATION**
published in accordance with Art. 158(3) EPC

(43) Date of publication:
**11.10.2000 Bulletin 2000/41**

(21) Application number: **98961656.0**

(22) Date of filing: **28.12.1998**

(51) Int. Cl.⁷: **C07K 14/47**, G01N 33/15, G01N 33/68, C12N 15/12, C12P 21/02

(86) International application number:
**PCT/JP98/06022**

(87) International publication number:
**WO 99/33874 (08.07.1999 Gazette 1999/27)**

(84) Designated Contracting States:
**AT BE CH CY DE DK ES FI FR GB GR IE IT LI LU MC NL PT SE**

(30) Priority: **27.12.1997 JP 36883397**

(71) Applicant:
**Matsushita Electric Industrial Co., Ltd.
Kadoma-shi, Osaka-fu, 571-8501 (JP)**

(72) Inventors:
• **MAEDA, Yuichiro,
Matsushita Electric Ind. Co. Ltd.
Kadoma-shi, Osaka 571-8501 (JP)**

• **MAEDA, Kayo,
Matsushita Electric Ind. Co., Ltd.
Kadoma-shi, Osaka 571-8501   1 (JP)**
• **TAKEDA, Soichi,
Matsushita Electric Ind. Co., Ltd.
Kadoma-shi, Osaka 571-85018501 (JP)**
• **VASSYLYEV, Dmitry,
Matsushita Elect. Ind. Co., Ltd
Kadoma-shi, Osaka 571-8501 (JP)**

(74) Representative:
**Cornish, Kristina Victoria Joy et al
Kilburn & Strode,
20 Red Lion Street
London WC1R 4PJ (GB)**

(54) **TROPONIN CI COMPLEXES**

(57) A stereostructure of complexes composing troponin C and an N-terminal fraction of troponin I $TnI_{1-47}$ constituting troponin complexes which is clarified by the X-ray diffractometry; a troponin calcium switching mechanism which is obtained based on the above stereostructure; and a system for screening agonists affecting the troponin calcium switching. These agonists might be candidates for drugs for myocardial infarct with a novel function mechanism.

**EP 1 043 334 A1**

## Description

TECHNICAL FIELD

The present invention relates to a complex consisting of troponin C and a peptide comprising troponin $I_{1-47}$, wherein both troponin C and troponin I are components of the troponin complex known as the calcium switch for muscle contraction, wherein said complex consisting of troponin C and the peptide has the atomic coordinates revealed by the present invention, and a use thereof.

BACKGROUND ART

The regulation of muscle contraction is accomplished by calcium ion binding to the muscle thin filament protein, troponin (Ebashi, S. and Endo, M., Calcium ion and muscle contraction, Prog. Biophys. Mol. Biol. 18, 123-183 (1968)). Troponin (Tn) is composed of three subunits: troponin T (TnT), a subunit having the tropomyosin-binding activity; troponin C (TnC), a subunit having the calcium ion-binding activity; and troponin I (TnI), a subunit having the inhibitory activity (Leavis, P. C. and Gergely, J., Thin filament proteins and thin filament-linked regulation of vertebrate muscle contraction, CRC Crit. Rev. Biochem. 16, 235-305 (1984); Zot, A. S. and Potter, J. D., Structural aspects of troponin-tropomyosin regulation of skeletal muscle contraction, Annu. Rev. Biophys. Biophys. Chem. 16, 535-559 (1987); Tobacman, L. S., Thin filament-mediated regulation of cardiac contraction, Annu. Rev. Physiol. 58, 447-481 (1996)). The concomitant presence of all of the three subunits and tropomyosin is required for the calcium-regulation of muscle contraction.

Although structural details of the whole Tn complex are not yet known, the crystal structure of the TnC subunit alone has been obtained. The crystal structure of TnC revealed a dumbbell-shape structure with two globular domains connected by a central helix (Herzberg, O. and James, M. N. G., Structure of the calcium regulatory muscle protein troponin-C at 2.8Å resolution, Nature 313, 653-659 (1985); Sundaralingam, M., Bergstrom, B., Strasburg, G., Rau, S. T., Roychowdhury, P., Greaser, M. and Wang, B. C., Molecular structure of troponin C from chicken skeletal muscle at 3 Å resolution, Science 227, 945-948 (1985)).

Each globular domain contains a helix-turn-helix motif which is a pair of calcium ion-binding sites known as EF hand. It has been shown that EF hand (which is composed of two calcium ion-binding sites) in the N-terminal domain is specific for calcium ions and responsible for regulation of muscle contraction, while EF hand (which is composed of two calcium ion-binding sites) in the C-terminal domain shows higher affinity for calcium ions and also binds to magnesium ions (Grabarek, Z., Tao, T. and Gergely, J., Molecular mechanism of troponin-C function, J. Muscle Res. Cell Motil. 13, 383-393 (1992); Farah, C. S. and Reinach, F. C., The troponin complex and regulation of muscle contraction, FASEB J. 9, 755-767 (1995)) and is capable of binding to calcium ions only when calcium ions are released in sacroplasm.

Recently, by multidimensional NMR, the calcium ion-saturated form of the TnC structure has been solved (Slupsky, C. M. and Sykes, B. D., NMR solution structure of calcium-saturated skeletal muscle troponin C, Biochemistry 34, 15953-15964 (1995)). As proposed by Herzberg et al. (Herzberg, O., Moult, J. and James, M. N. G., A model for the $Ca^{2+}$-induced conformational transition of troponin C, J. Biol. Chem. 261, 2638-2644 (1986)), in the calcium ion-bound form, a hydrophobic patch is exposed in the N-terminal domain by relative movement of helices B and C with respect to helices N, A and D.

Although the tertiary structures of other subunits still remain to be solved, a low-resolution model of TnC/TnI complex has been proposed (Olah, G. A., Rokop, S. E., Wang, C.-L. A., Blechner, S. L. and Trewhella, J., Troponin I encompasses and extended troponin C in the $Ca^{2+}$-bound complex, Biochemistry 33, 8233-8239 (1994); Olah, G. A. and Trewhella, J., A model structure of the muscle protein complex $4Ca^{2+}$-troponin C -troponin I derived from small-angle scattering data, Biochemistry 33, 12800-12806 (1994)).

Calcium ion-binding to the regulatory sites of TnC causes a change in the protein-protein interaction within the Tn complex, which transmits to thin filament components and ultimately changes the interaction of actin with myosin. In order to understand the molecular mechanism of the regulation by Tn, conformational transitions in Tn upon binding of calcium ions to the regulatory sites of TnC have been investigated by various methods, including chemical modification of Lys side chains (Hitchcock, S. E., Zimmerman, C. J. and Smalley, C., Study of the structure of troponin-T by measuring the relative reactivities of lysine with acetic anhydride, J. Mol. Biol. 147, 125-151 (1981); Hitchcock, S. E., Study of the structure of troponin-C by measuring the relative reactivities of lysine with acetic anhydride, J. Mol. Biol. 147, 153-173 (1981); Hitchcock-DeGregori, S. E., Study of the structure of troponin-I by measuring the relative reactivities of lysine with acetic anhydride, J. Biol. Chem. 257, 7372-7380 (1982)), chemical cross-linking (Sutoh, K., Direct evidence for the calcium-induced change in the quaternary structure of troponin in situ, Biochemistry 19, 1977-1983 (1980); Wang, Z., Sarker, S., Gergely, J. and Tao, T., $Ca^{2+}$-dependent interaction between the C-helix of troponin C and troponin I, J. Biol. Chem. 265, 4953-4957 (1990)), and fluorescence resonance energy transfer (Schulzki, H.-D.,

Kramer, B., Fleschhauer, J., Mercola, D. A. and Woller, A., Calcium-dependent distance changes in binary and ternary complexes of troponin, Eur. J. Biochem. 189, 683-692 (1990); Zhao, X., Kobayashi, T., Malak, H., Gryczynski, I., Lakowicz, J., Wade, R. and Collins, J. H., Calcium-induced troponin flexibility revealed by distance distribution measurements between engineered sites, J. Biol. Chem. 270, 15507-15514 (1995)).

Using limited proteolysis of the Tn complex under different conditions, the present inventors found that not only TnT but also TnI was susceptible to chymotryptic digestion and that cleavage sites in TnI were dependent on the calcium ion occupancy of TnC, suggesting the calcium ion-dependent conformational transition of TnI (Takeda, S., Kobayashi, T., Taniguchi, H., Hayashi, H., and Maeda, Y. Eur. J. Biochem. 246, 611-617 (1997)). The present inventors also investigated the effects of the depletion of C-terminal segments of TnI on acto-S1 ATPase activity. Our results indicate that the residues 117-134 or 117-140 of TnI are necessary for the calcium ion-dependent inhibitory action of Tn complex on acto-S1 ATPase activity, while the residues 135-181 or 141-181 of TnI are involved in the interaction with the tropomyosin-actin filament. Since the accessibility to proteases is thought to be related to flexibility of the protein substrate (Fontana, A., Fassina, G., Vita, C., Dalzoppo, D., Zamai, M. and Zambonin, M., Correlation between sites of limited proteolysis and segmental mobility in thermolysin, Biochemistry 25, 1847-1851 (1986)), namely, since it is thought that the hardly cleaved parts compose single domains and the easily cleaved parts are linkages between the plural domains in the whole troponin molecule, the complex consisting of three kinds of subunits, the results obtained here also indicate the structural domains of the Tn ternary complex.

The present status of study on troponin as described above is summarized as follows:

(1) Troponin is composed of troponin T (TnT), a subunit having the tropomyosin-binding activity, troponin C (TnC), a subunit having the calcium ion-binding activity and troponin I (TnI), a subunit having the inhibitory activity.
(2) The regulation of skeletal and cardiac muscle contraction is associated with a calcium ion-dependent structural transition in the muscle thin filament, the complex of actin, tropomyosin and troponin (Tn, TnC/TnI/TnT complex). The signals induced by the variations of the calcium ion concentration are transferred to the other components of the thin filament through the calcium ion-receptor, Tn. The binding of calcium ions to TnC facilitates the release of inhibitory region of TnI from the actin-tropomyosin, which relieves the inhibition of actomyosin magnesium ion-ATPase.
(3) The entire structure of the Tn complex has not been elucidated yet. Although ten years have passed since the structure of TnC alone, a component of Tn was known, it is, even now, impossible to understand the mechanism of the calcium switch from the structure of TnC alone. There are two reasons for this. First, how the two domains of TnC forming a dumbbell-shape structure interact with each other in the troponin complex has not been elucidated yet. Second, how TnC binds to TnI has not been found yet. There are two similar domains, N domain and C domain in TnC. And TnI also has two hydrophobic regions (TnI1-47 and $TnI_{reg}$). Which domain of TnC binds to which region of TnI has not been determined yet.
(4) As described above, it is clear that troponin plays an important role in the calcium switch for muscle contraction. However, since the entire structure of troponin complex has not been determined yet, the action of mechanism of the calcium switch can not be understood and hence, it is also difficult to investigate the drugs acting muscle contraction. These situations would be owing to the quite different conformational structure of TnC in muscle tissues from the elucidated, isolated structure of TnC.

DISCLOSURE OF INVENTION

The first object of the present invention is to elucidate a three-dimensional structure of the troponin complex, the calcium switch regulating muscle contraction, in particular the complex consisting of TnC and TnI, and to reveal a relationship between the three dimensional structure of the complex and the mechanism of calcium switch for muscle contraction. The second object of the present invention is to provide a protein complex, which is a minimum unit of the calcium switch structure for muscle contraction. The third object of the present invention is to provide an assay system for screening a drug capable of exerting an influence on the calcium switch for muscle contraction.

In the course of endeavor to find a minimum unit composing the calcium switch among the troponin components, the present inventors have succeeded in determining the atomic coordinates of crystals of the complex consisting of troponin C and a N-terminal fragment of troponin I (amino acid residues 1 to 47) at 2.3Å resolution and further elucidating the interrelationship between the obtained structure of the complex and the mechanism of calcium switch for muscle contraction.

The first aspect of the present invention provides a complex or crystals thereof consisting of troponin C and a peptide fragment of troponin $I_{1-47}$ and having the atomic coordinates set forth in the description. Furthermore, it provides a complex consisting of troponin C and a peptide that contains the troponin $I_{1-47}$ fragment and that is capable of binding to troponin C wherein the complex has the atomic coordinates substantially as set forth in the description. In the above peptide that contains the troponin $I_{1-47}$ fragment and that is capable of binding to troponin C, troponin I itself is also included. Further, it also provides a complex consisting of troponin C and any peptide fragment obtained by single or multiple deletions, additions, insertions or substitutions of one or more amino acids in troponin $I_{1-47}$ or a peptide containing the peptide fragment, wherein said complex has the atomic coordinates substantially as set forth in this description.

The second aspect of the present invention relates to a screening system for drugs capable of exerting an influence on the calcium switch for muscle contraction, said screening system comprising the complex consisting of troponin C and troponin $I_{1-47}$ which can be replaced by troponin I, a peptide containing troponin $I_{1-47}$ fragment and which is capable of binding to troponin C, or a peptide containing a peptide fragment obtained by single or multiple deletions, additions, insertions or substitutions of one or more amino acids in troponin $I_{1-47}$ and which is capable of binding to troponin C.

Such a drug screening system, for example, includes an appropriate buffer solution containing troponin C, equimolar troponin $I_{1-47}$ to troponin C, a drug candidate and various concentrations of calcium ion. In the screening system, troponin $I_{1-47}$ can be replaced by troponin I, a peptide containing troponin $I_{1-47}$ fragment and which is capable of binding to troponin C, or a peptide containing a peptide fragment obtained by single or multiple deletions, additions, insertions or substitutions of one or more amino acids in troponin $I_{1-47}$ and which is capable of binding to troponin C.

The drug screening system is useful for selecting compounds which may have a function of strengthening or weakening the binding of calcium ion to troponin C. In the screening system, any usable method for calcium binding measurement includes, for example, an indicator method using methyl murexide (Ogawa, Y. and Ebashi, S., "Measurements for Calcium Binding", In Ebashi ed. "Development of Techniques for Heart and Blood Vessel Study", Gakkai-shuppan Center (1983)).

Using a similar drug screening system, compounds which are capable of exerting an influence on the association between troponin C and troponin $I_{1-47}$ or troponin I may be selected. These are, for example, the compounds capable of strengthening or weakening the association between troponin C and troponin $I_{1-47}$ or troponin I. For determination of the strength of association between troponin C and troponin $I_{1-47}$ or troponin I, for example, a method monitoring a strength change of fluorescence of fluorescence-labeled troponin C upon complex formation (Pearlstone, J. R., Sykes, B. D. and Smillie, L. B., Biochemistry <u>36</u>, 7601-7606 (1997)) can be used.

BRIEF DESCRIPTION OF DRAWINGS

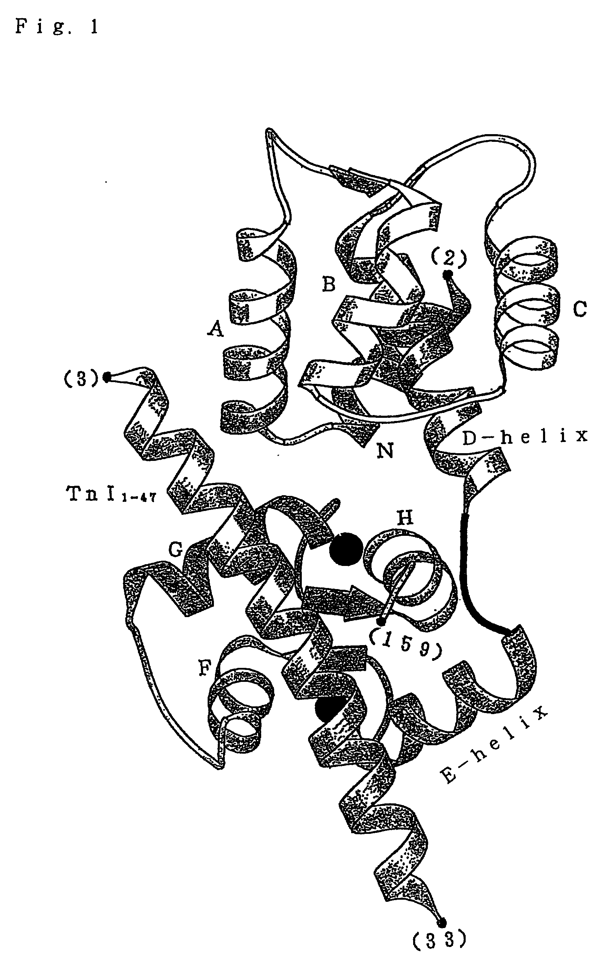

Fig. 1 shows a ribbon diagram of the structure of TnC/TnI$_{1-47}$ complex. The $\alpha$ helices, $\beta$ strands and loops of TnC are represented by spirals labeled with N or A to H, arrow plates and thin lines, respectively. The helices N, A, B, C and D altogether constitute N domain and helices E, F, G and H constitute C domain. A dark part linking D-helix to E-helix is a linker part linking N domain with C domain and the $\alpha$ helix in the part is unwound and kinked. The two calcium ions in the C domain of TnC are represented as black balls. The $\alpha$ helix of TnI$_{1-47}$ is labeled with TnI$_{1-47}$. The diagram was drawn by using the MOLSCRIPT program (Kraulis, P. J., MOLSCRIPT: a program to produce both detailed and schematic plots of protein structures, J. Appl. Cryst. <u>24</u>, 946-950 (1991)).

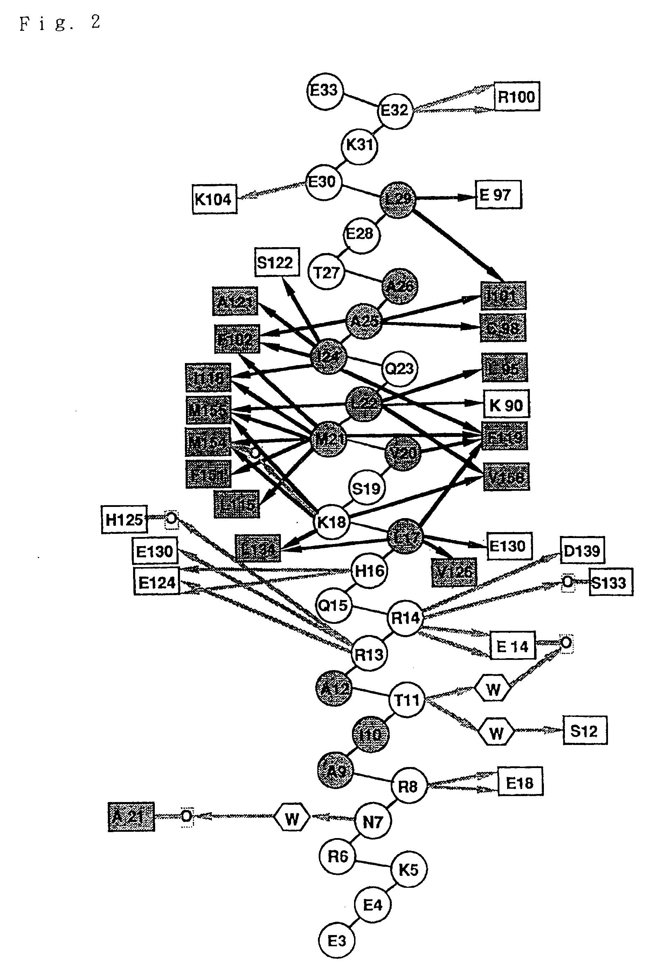

Fig. 2 shows hydrophobic (closed arrows) and polar (gray arrows) interactions between TnI$_{1-47}$ residues (circles) and TnC residues (squares) in the TnC/TnI$_{1-47}$ complex. Amino adds are shown in the single letter abbreviation. Hydrophobic and polar residues are shown with gray and outline types, respectively. Water molecules are shown as hexagonal outline types.

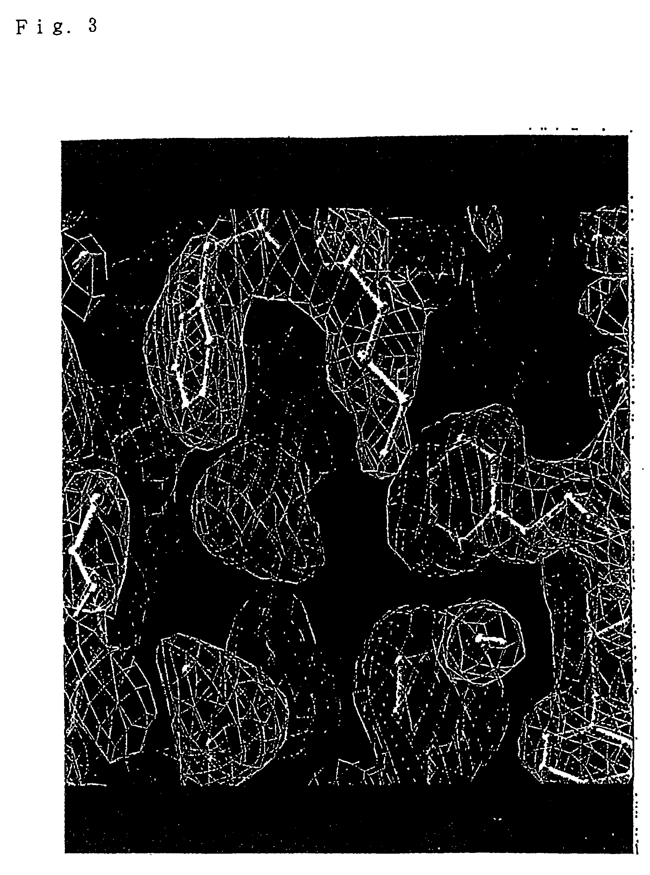

Fig. 3 shows the final electron density map (2Fo-Fc) partially superimposed by TnC/TnI$_{1-47}$ model (a ball-and-stick model) in the hydrophobic pocket of C domain of TnC. TnI and TnC residues are shown with cyan colors and each atom is shown with each color (C, N, O and S are shown with yellow, blue, red and green colors, respectively). Met21 protruding into the hydrophobic pocket of TnC is shown with red color. The figure was drawn by O program (Jones, T. A., Zou, J. Y., Cowan, S. E. and Kjeldgaard, M., Improved methods for building protein models in electron density maps and location of errors in these models, Acta Crystallogr. <u>A47</u>, 110-119 (1991)).

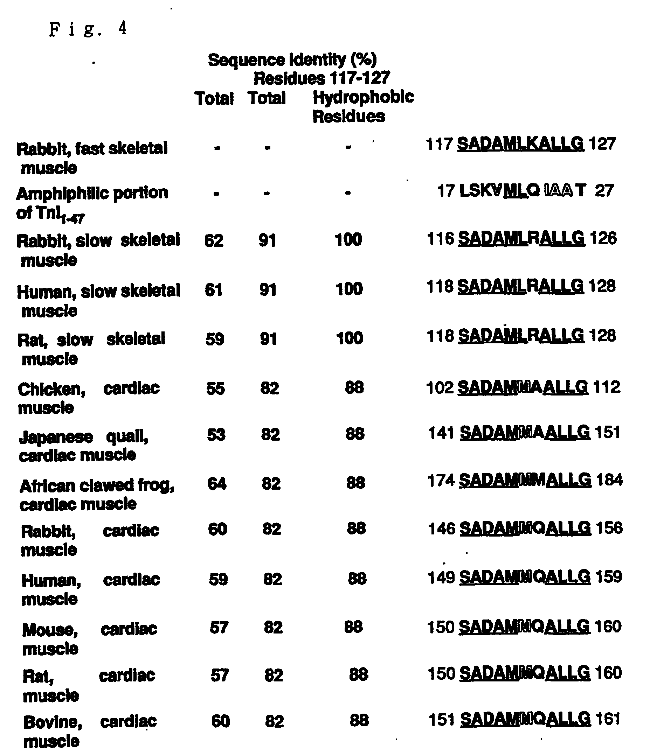

Fig. 4 shows the TnI sequence at the C terminal end (residues 117-127, rabbit skeletal muscle) of the inhibitory fragment (residues 96-116) aligned with the amphiphilic portion of TnI$_{1-47}$ and with the TnIs from the other species. The amino acids are shown in the single letter abbreviation. Only the TnIs with less than 65% overall homology were taken for comparison. The rabbit sequence and the identical residues in the other sequences are underlined, the distinct residues are shown with usual letters, and the amphiphilic residues corresponding to the rabbit hydrophobic amino acids are shown with outline types.

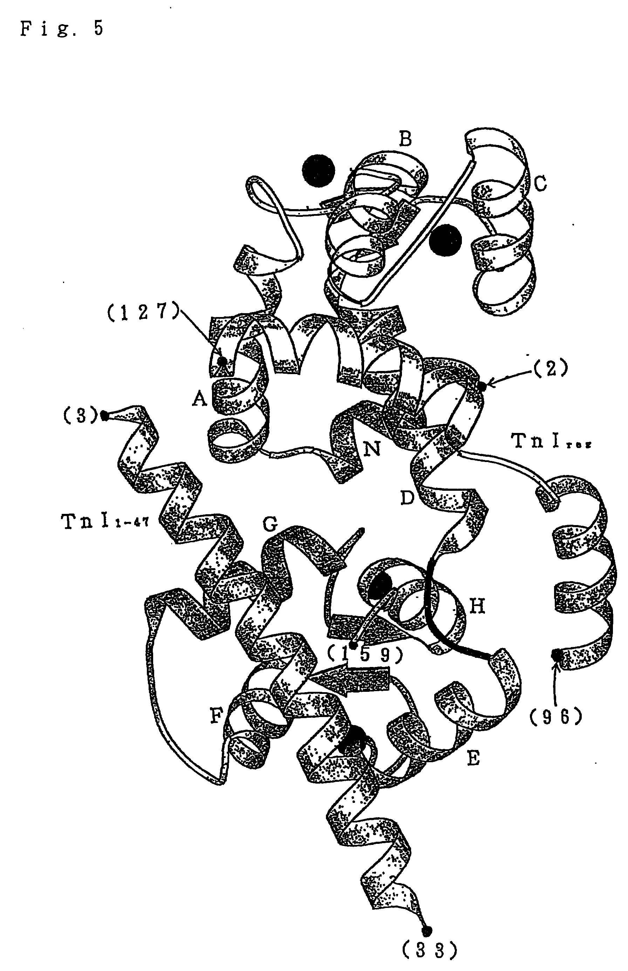

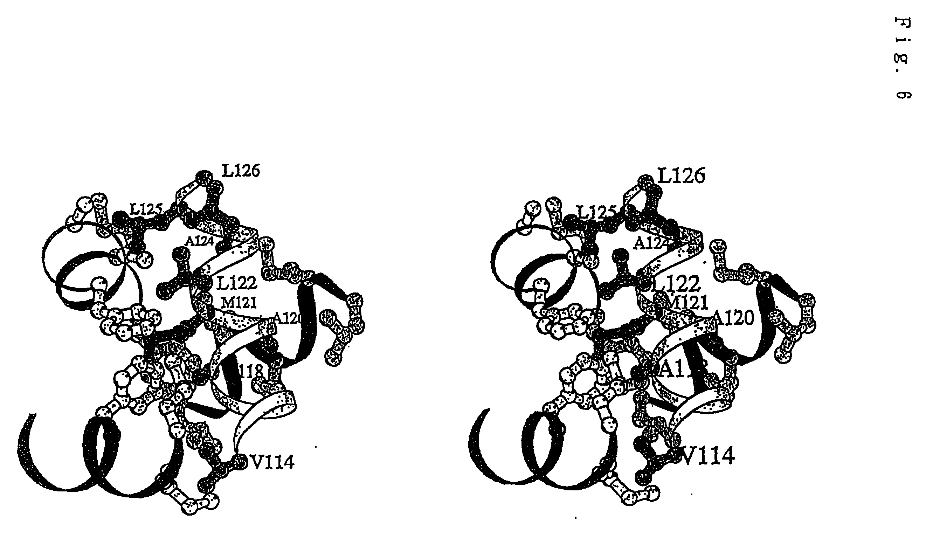

Fig. 5 shows a ribbon diagram of the structure of TnC/TnI$_{reg}$ model. This figure and Fig. 6 were drawn by the MOLSCRIPT. The two "open" N- and C-domains of TnC, namely, N-domain consisting of N, A, B, C and D helices and C-domain consisting of E, F, G and H helices, respectively and the linker part connecting the D helix with E helix (shown with black color) are shown in the same manner as in Fig. 1. Two calcium ions (shown with black balls) bind to each of the N- and C-domains. The $\alpha$ helices of TnI$_{1-47}$ and TnI$_{reg}$ are indicated as TnI$_{1-47}$ and TnI$_{reg}$, respectively.

Fig. 6 shows a stereo view of the hydrophobic core of the TnC/TnI$_{reg}$, model structure. The $\alpha$ helices of TnC and TnI$_{reg}$ are shown as black and outline type spirals, respectively. The side chains of the residues in the hydrophobic pocket of the "open" C-domain of TnC and the complementary hydrophobic residues of TnI$_{reg}$ (including Met121) are shown with gray and black ball-and-stick models, respectively.

The reference letters used in Fig. 1 to Fig. 6 have the following means:
A, B, C, N, F, G and H show $\alpha$ helices, respectively. (2) means the amino acid residue at the 2nd position in the N-terminus of TnC, (159) means the amino acid residue at the 159th position in the C-terminus of TnC, (3) means the amino acid residue at the 3rd position in the N-terminus of TnI$_{1-47}$, (33) means the amino acid residue at the 33rd position in the C-terminus of Tn$_{1-47}$, (96) means the amino add residue at the 96th position in the N-terminus of TnI$_{reg}$, and (127) means the amino acid residue at the 127th position in the C-terminus of TnI$_{reg}$.
E3 means glutamic acid at the 3rd position wherein E represents single letter abbreviation of amino acid (glutamic acid) and 3 means the position of the residue in the peptide chain. Other reference letters in Figs. 2 and 6 have the same meanings.

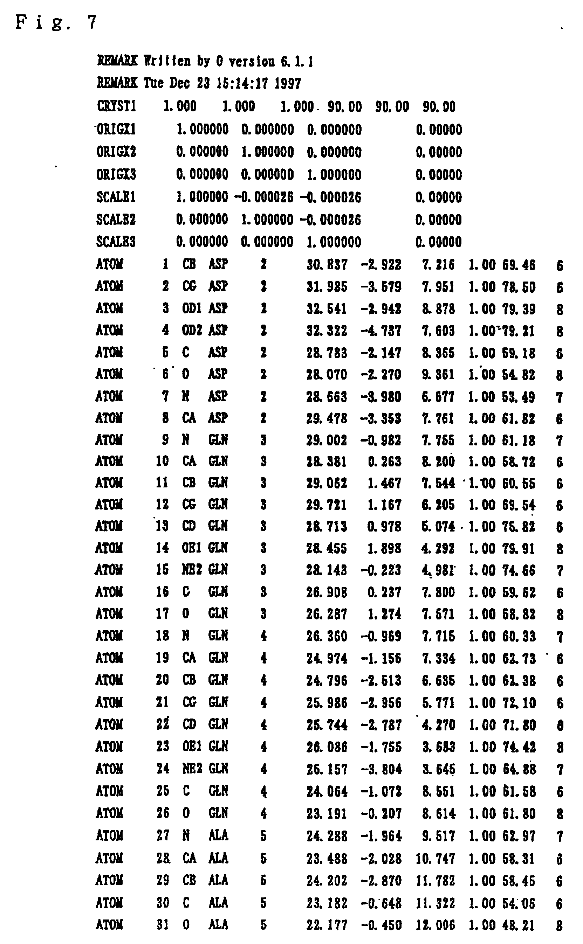

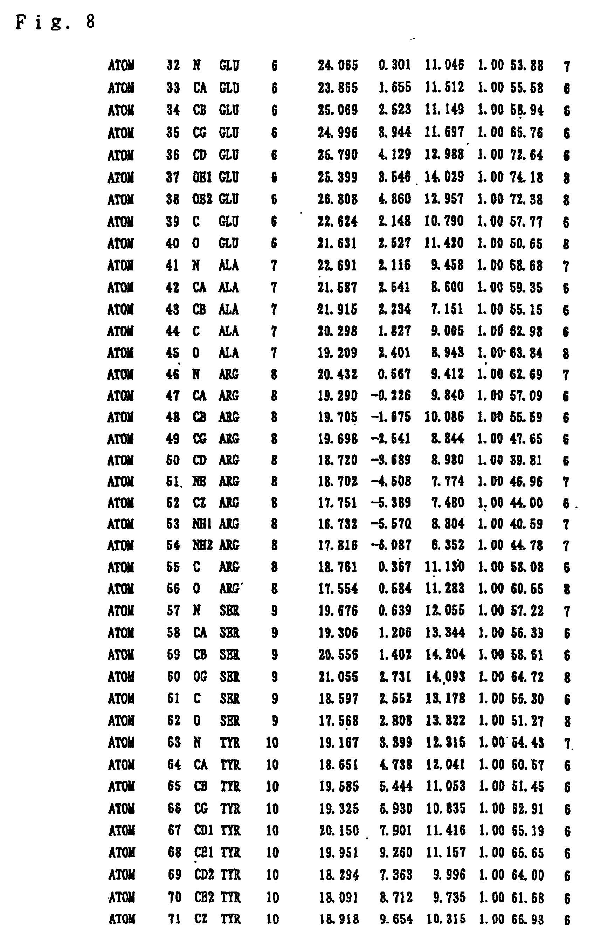

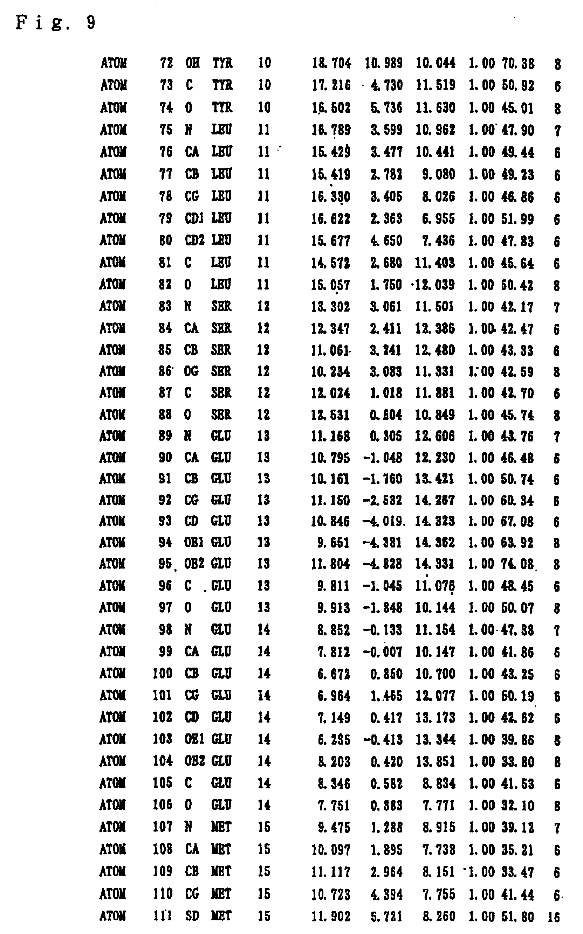

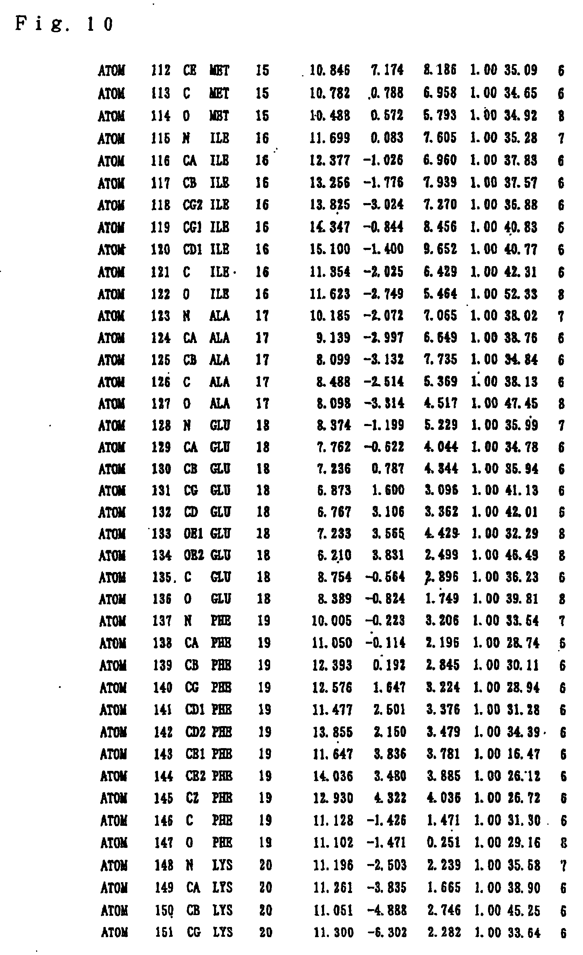

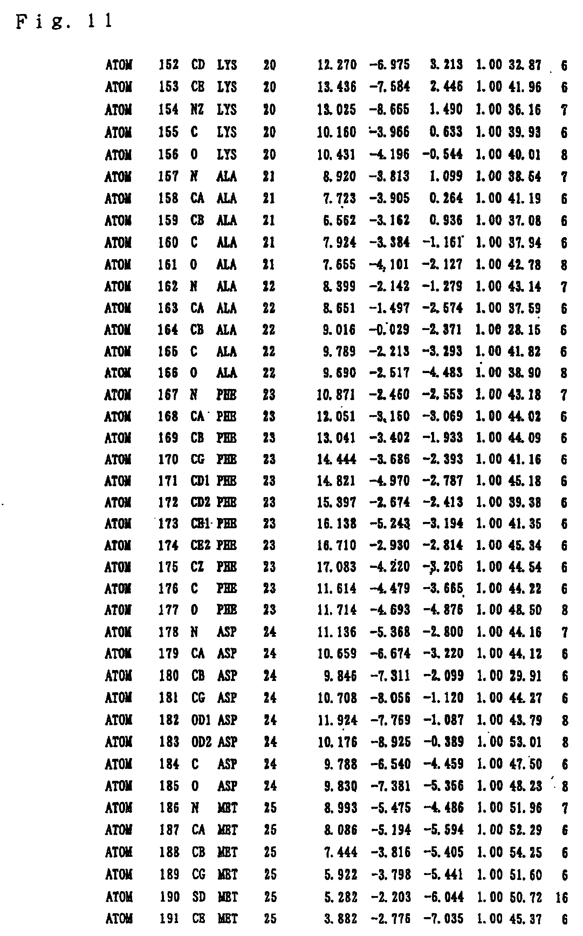

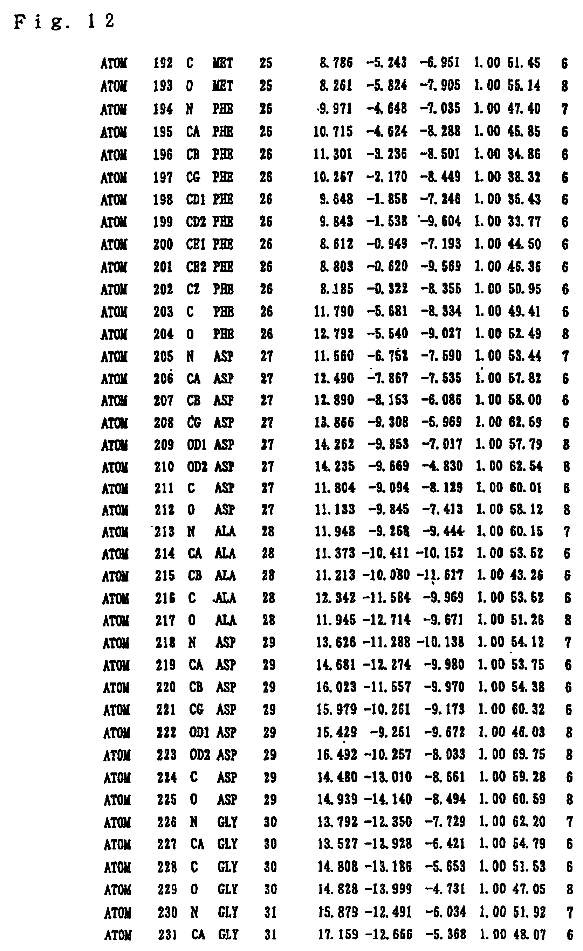

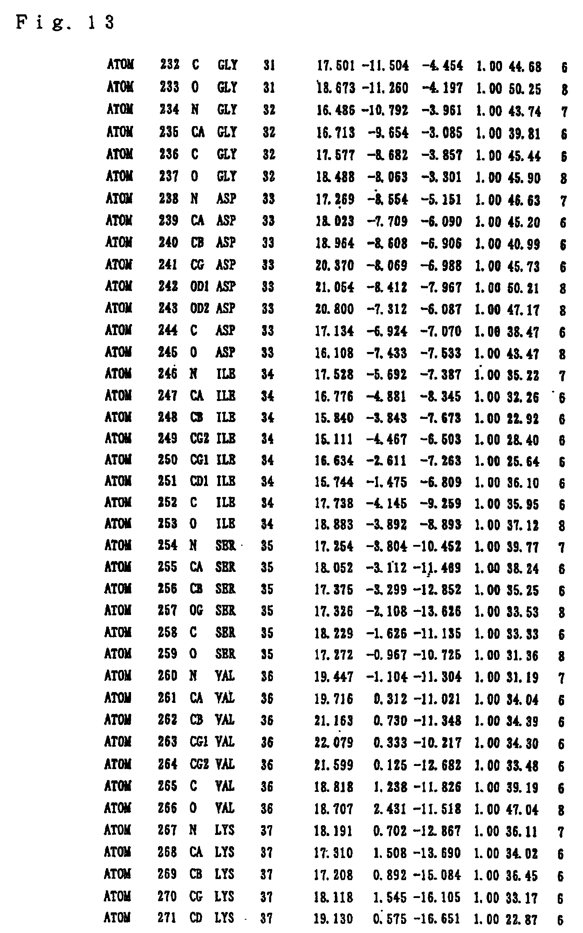

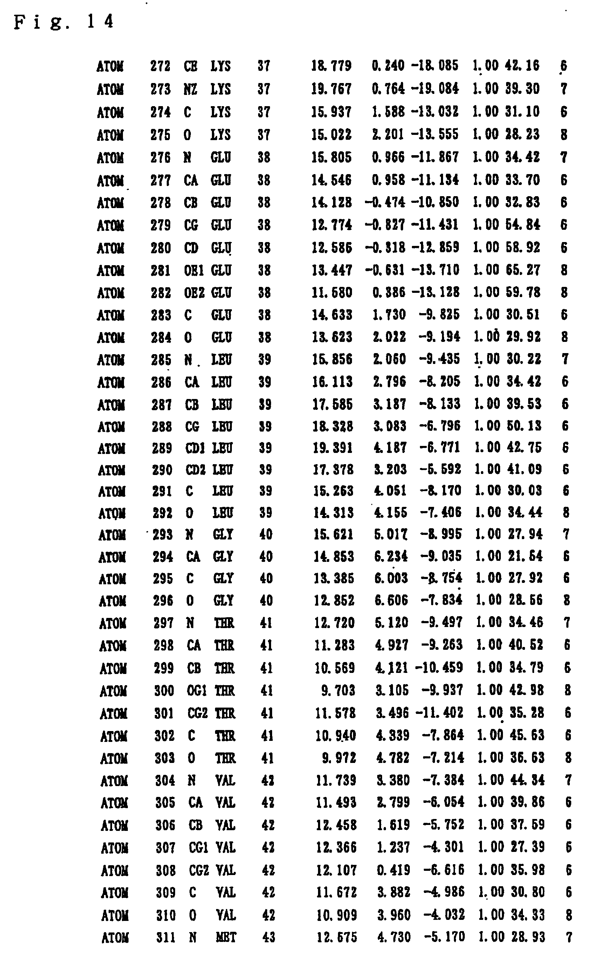

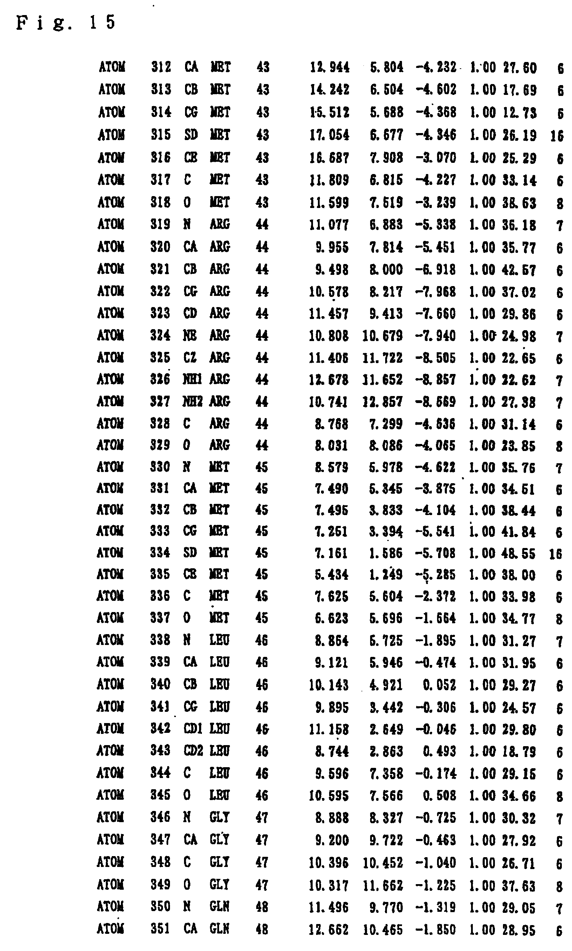

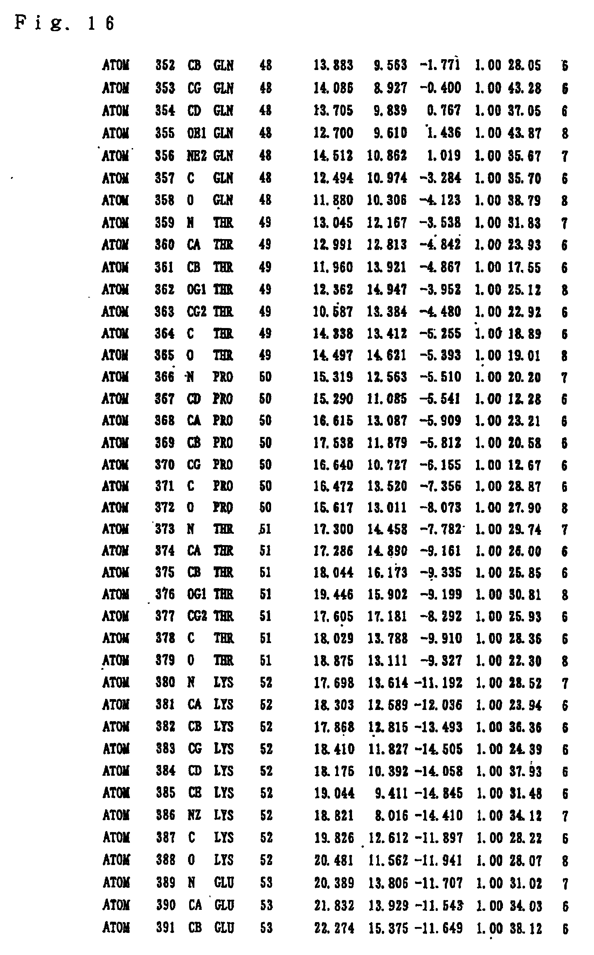

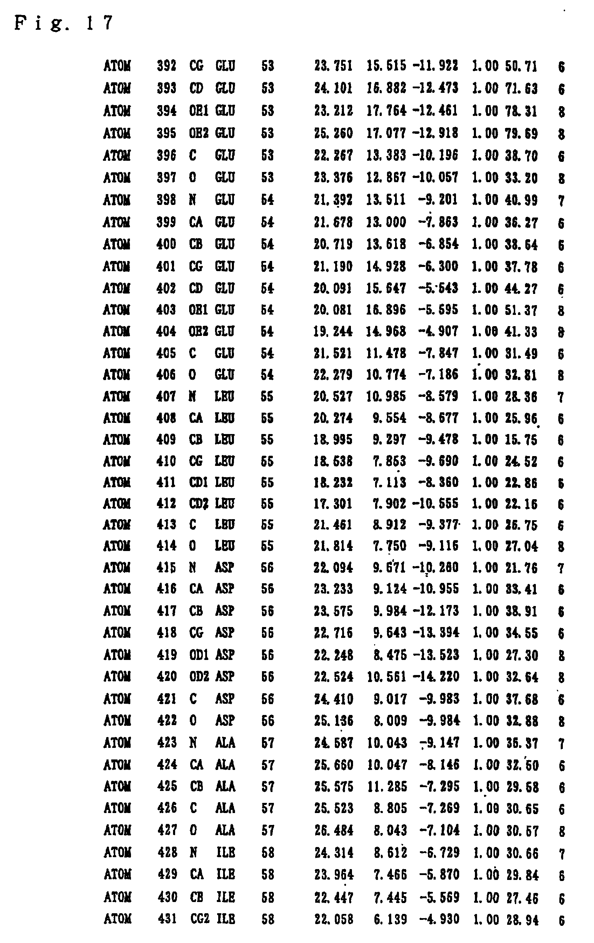

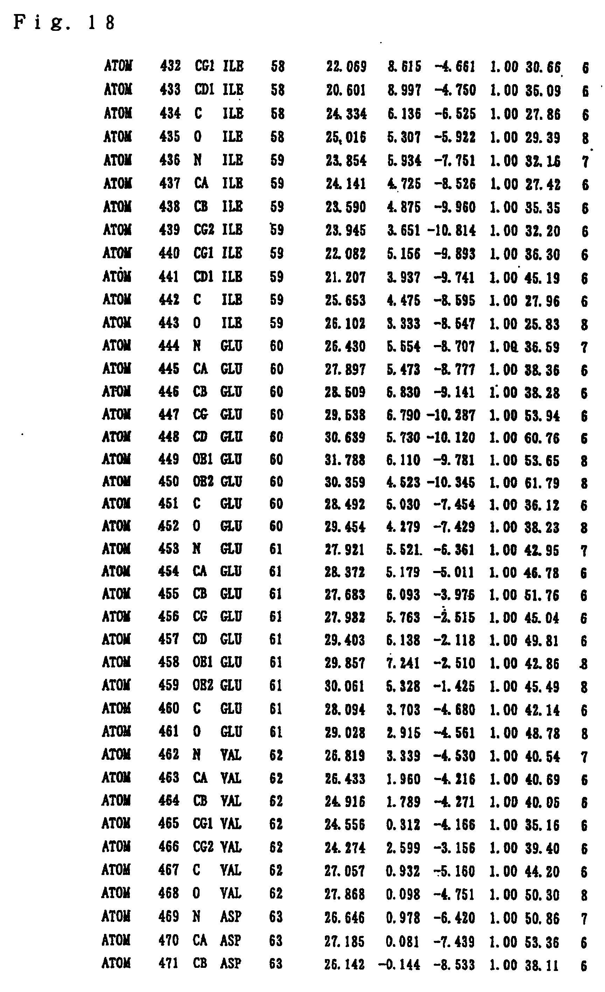

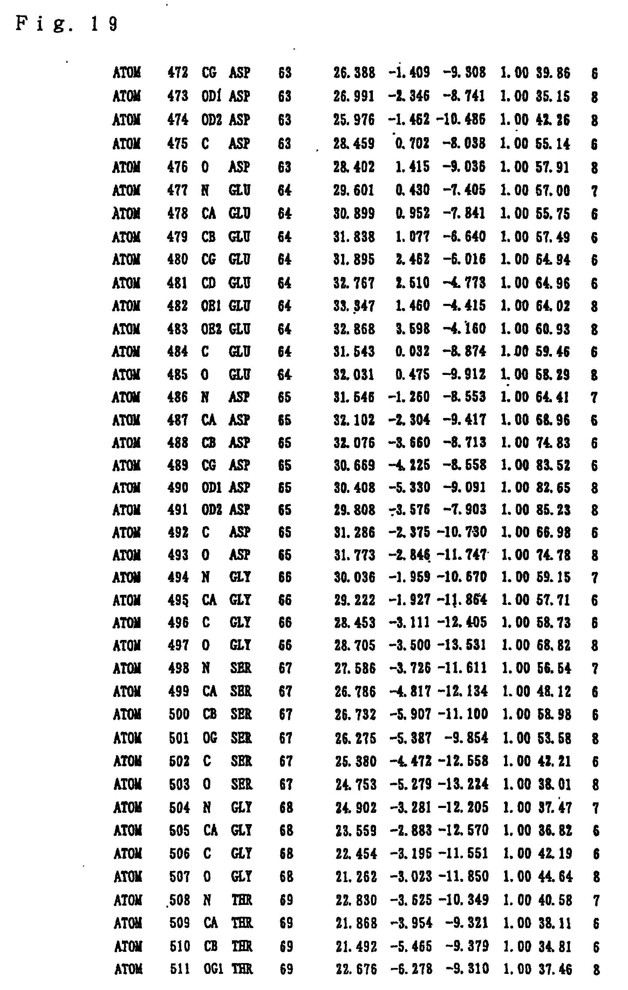

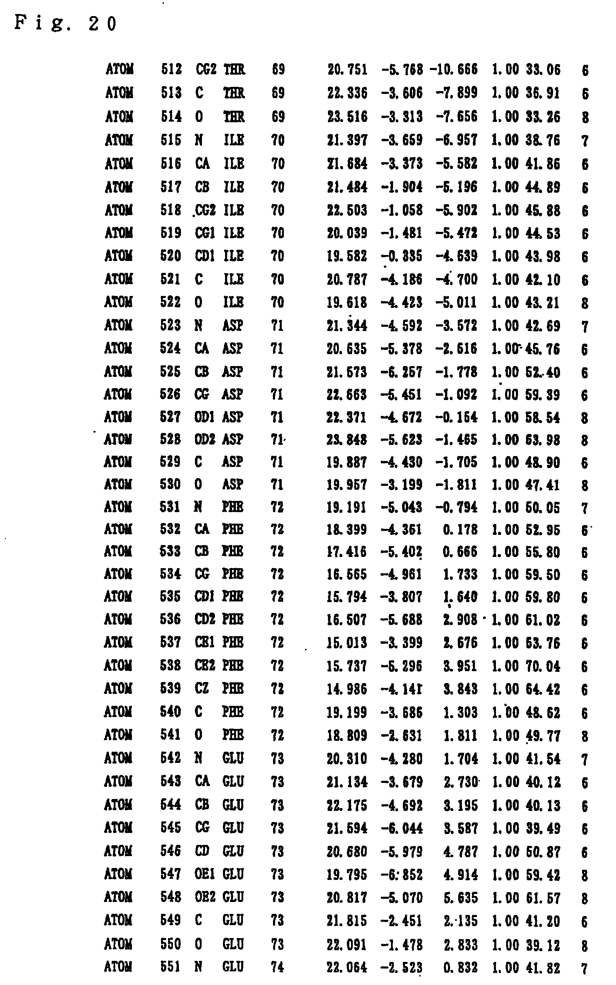

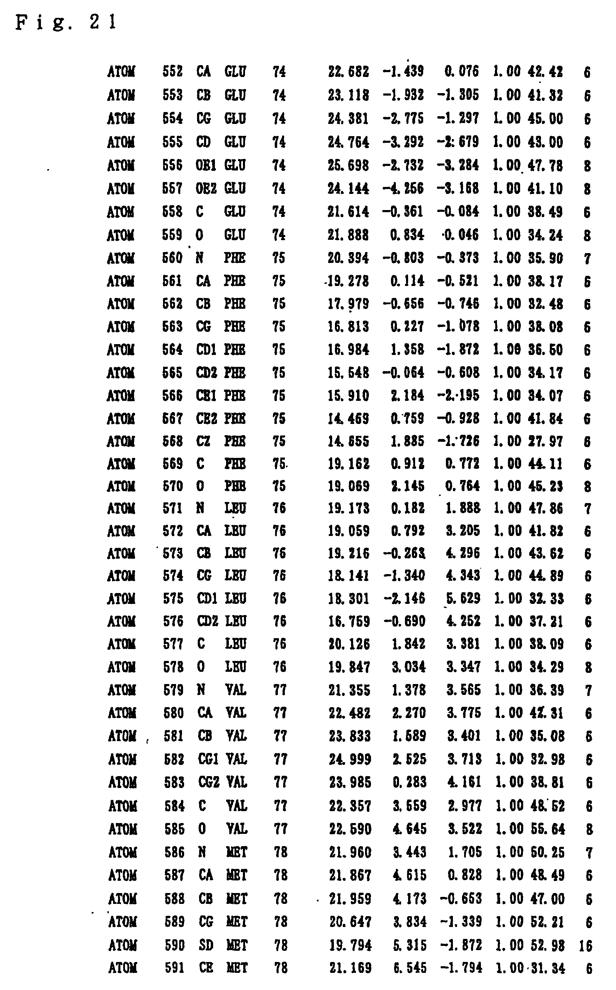

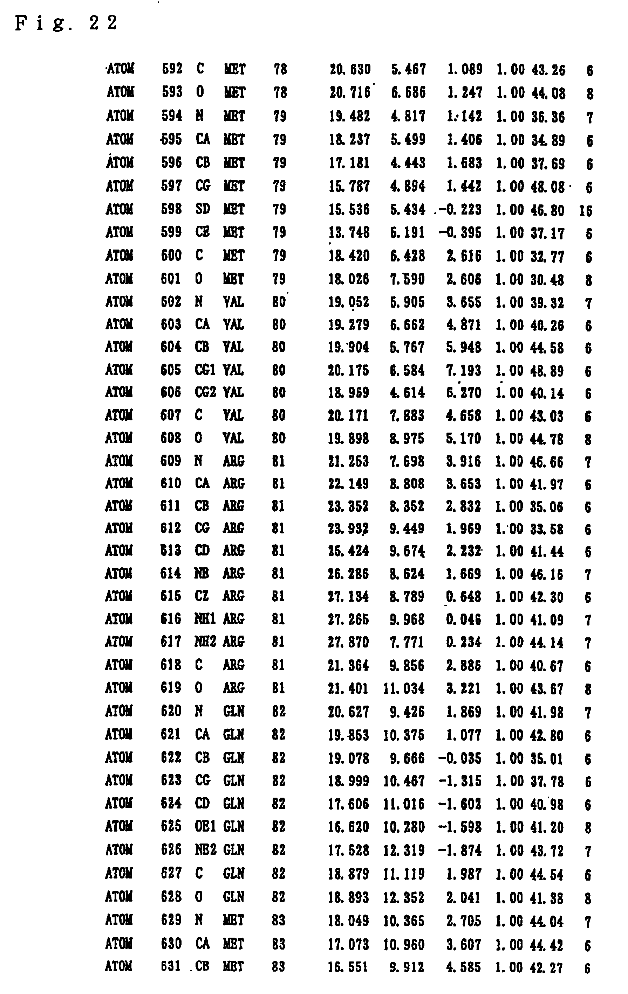

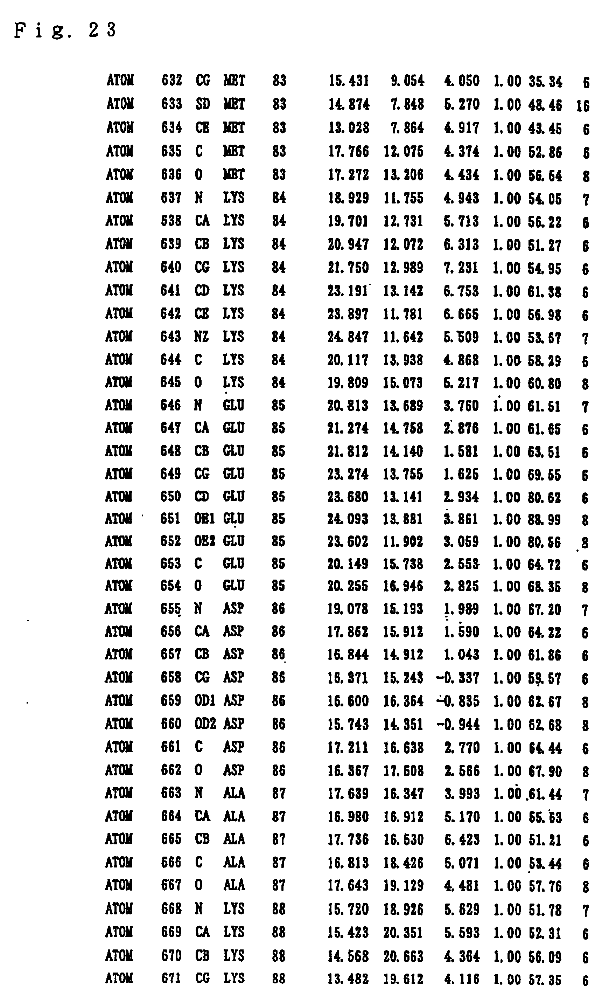

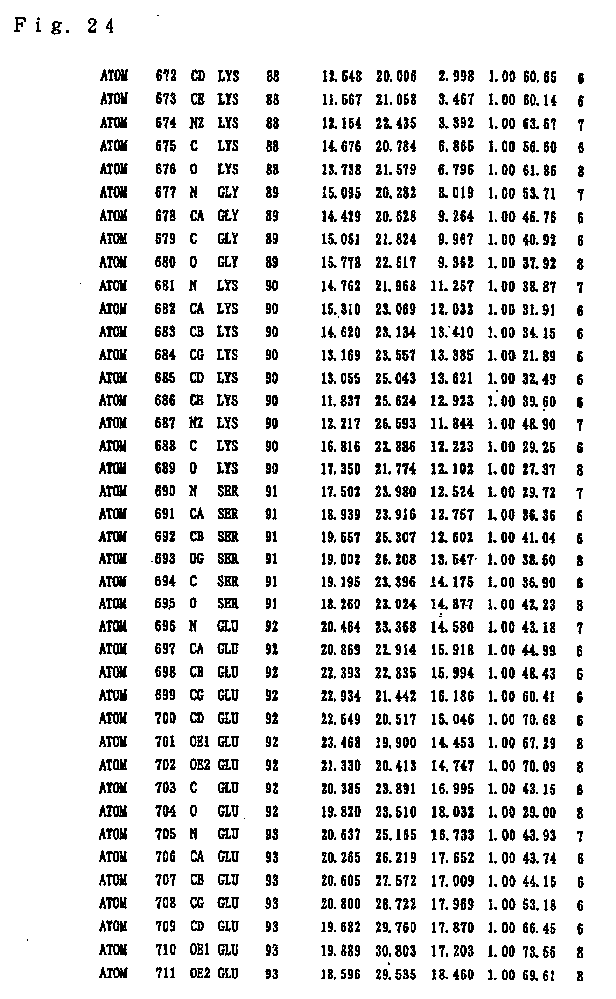

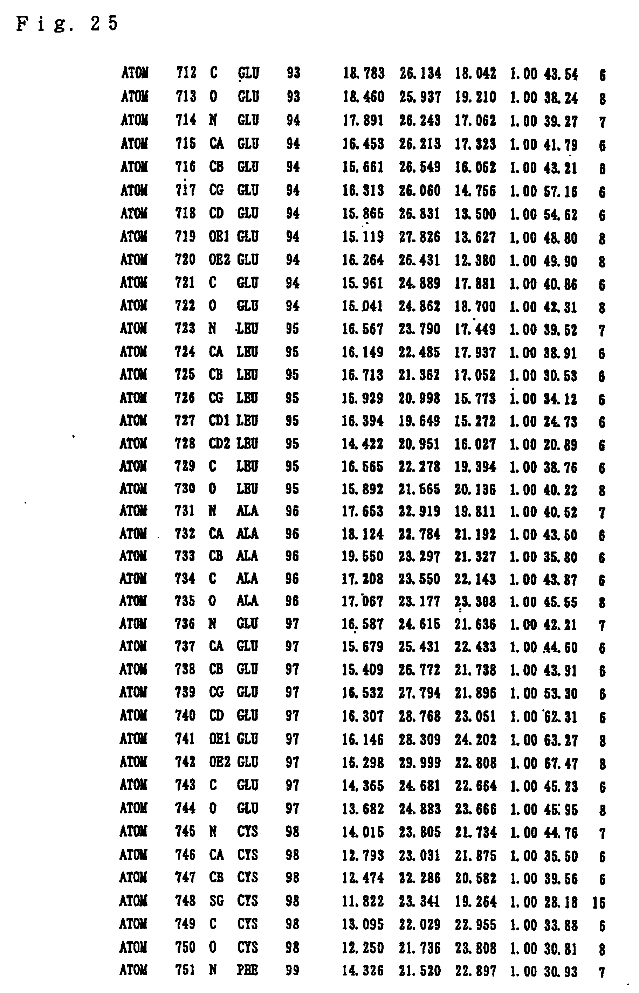

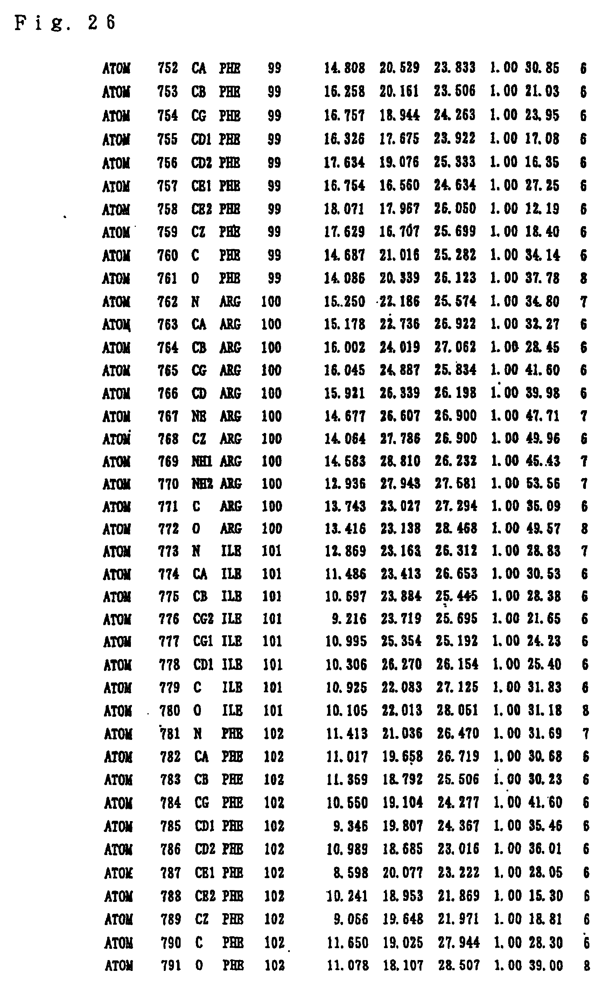

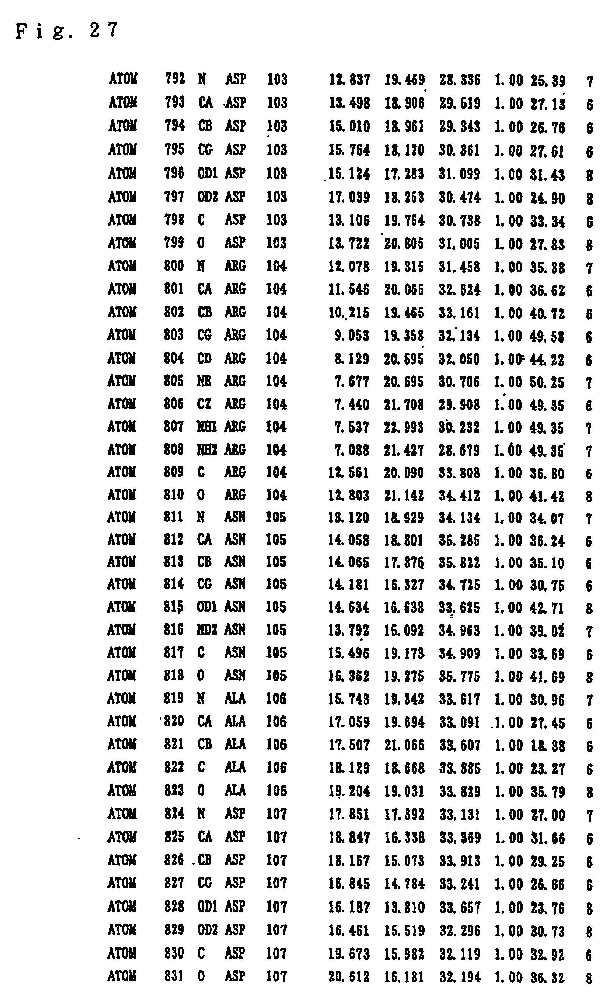

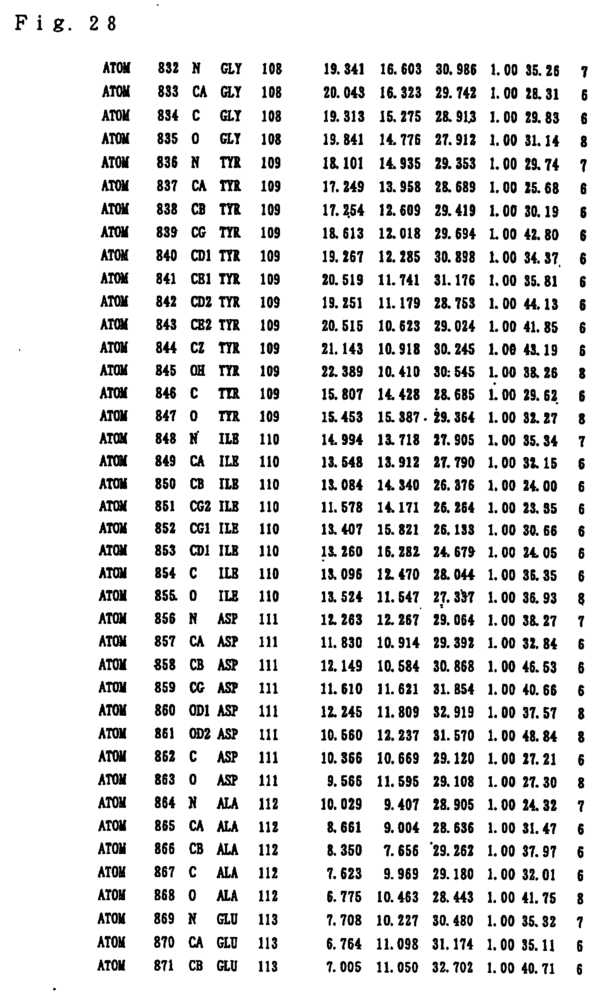

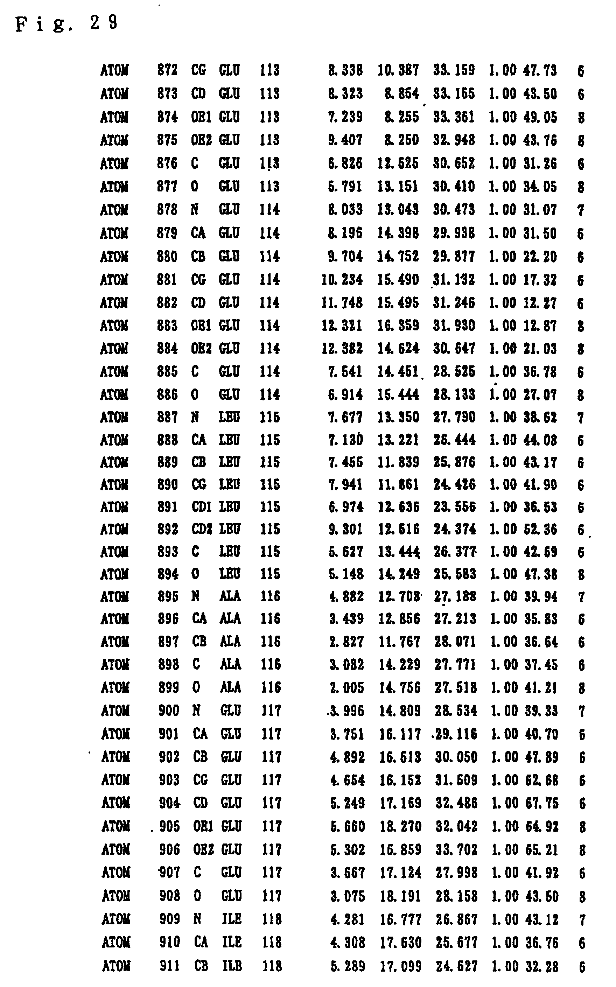

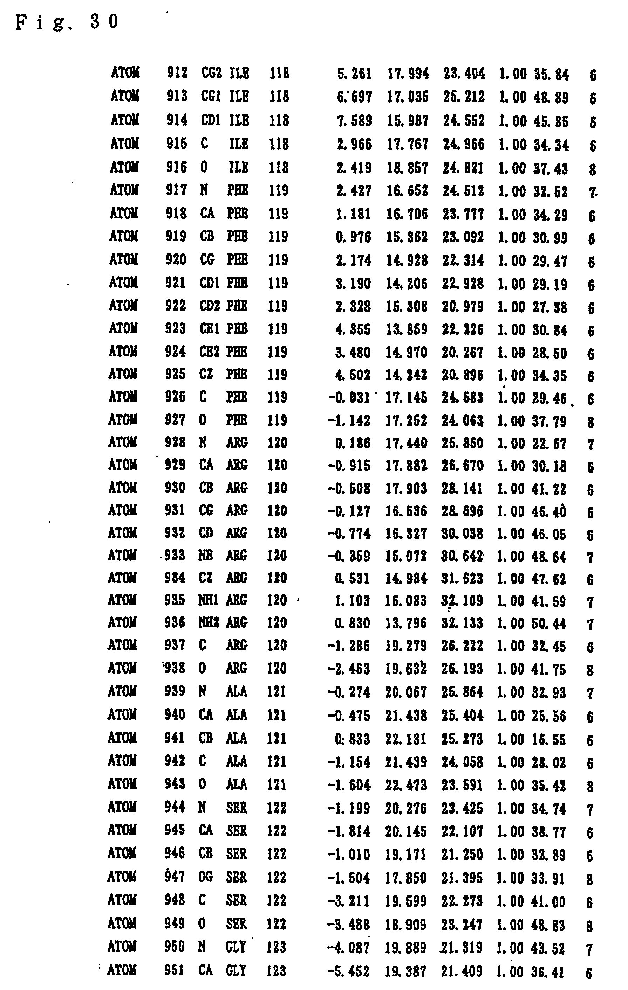

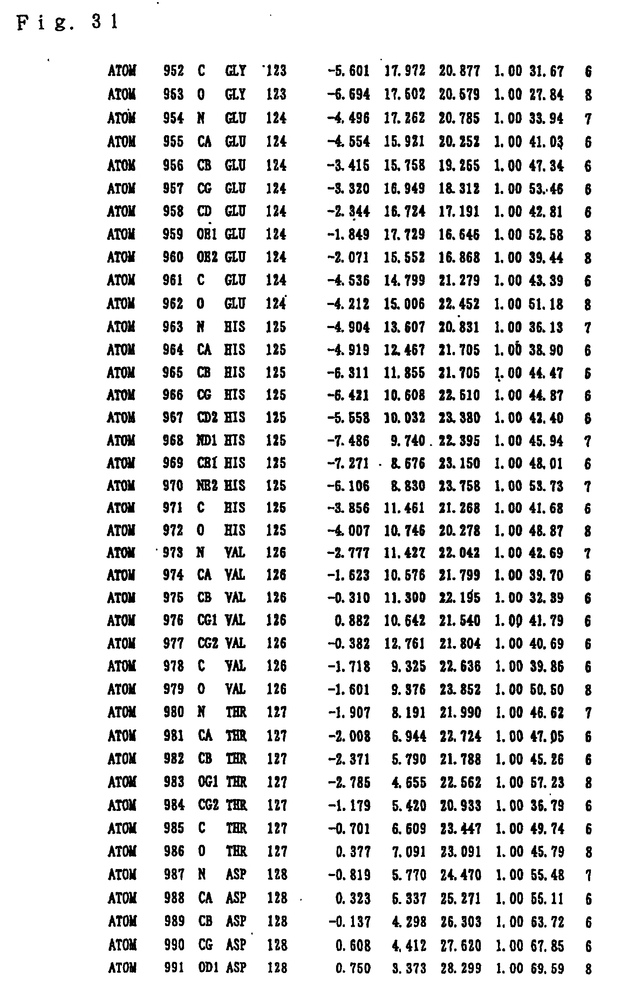

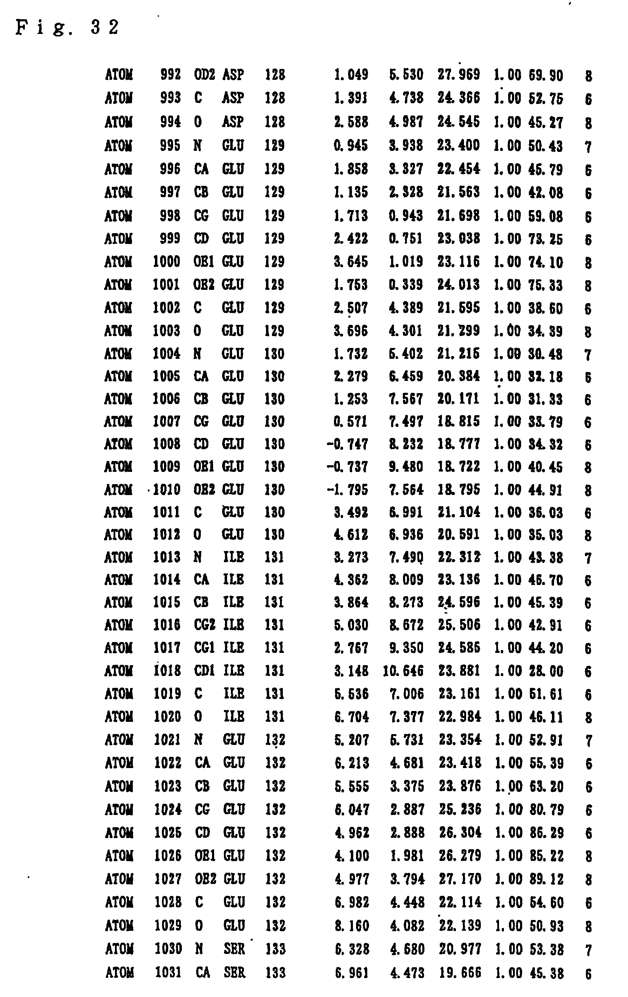

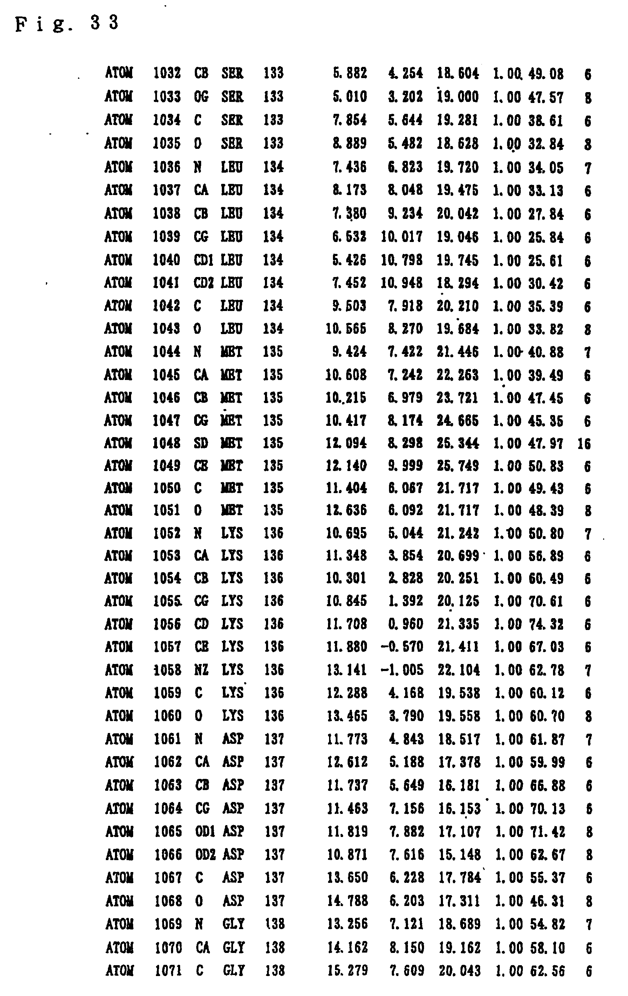

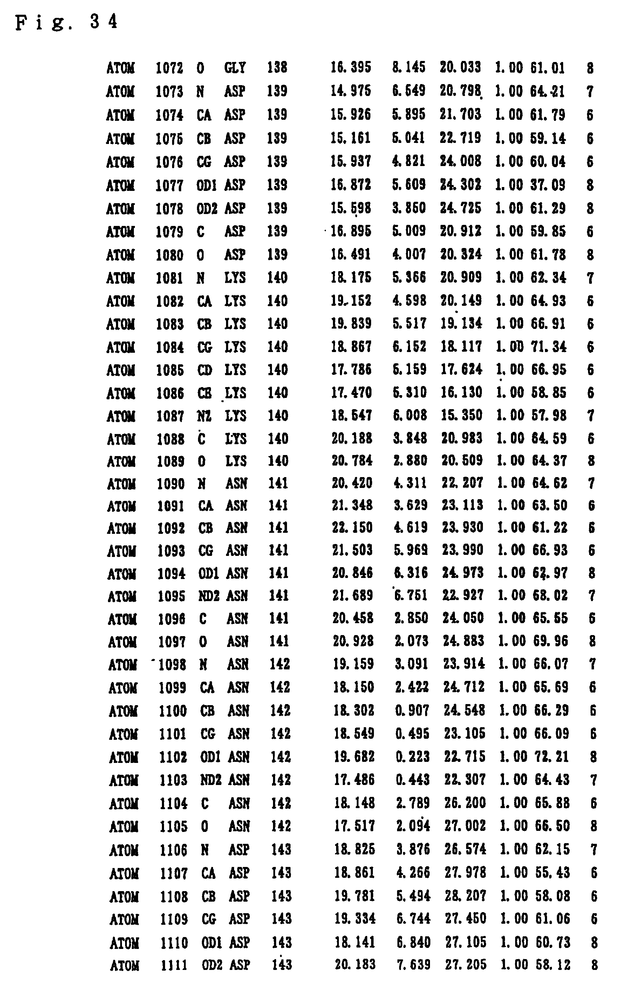

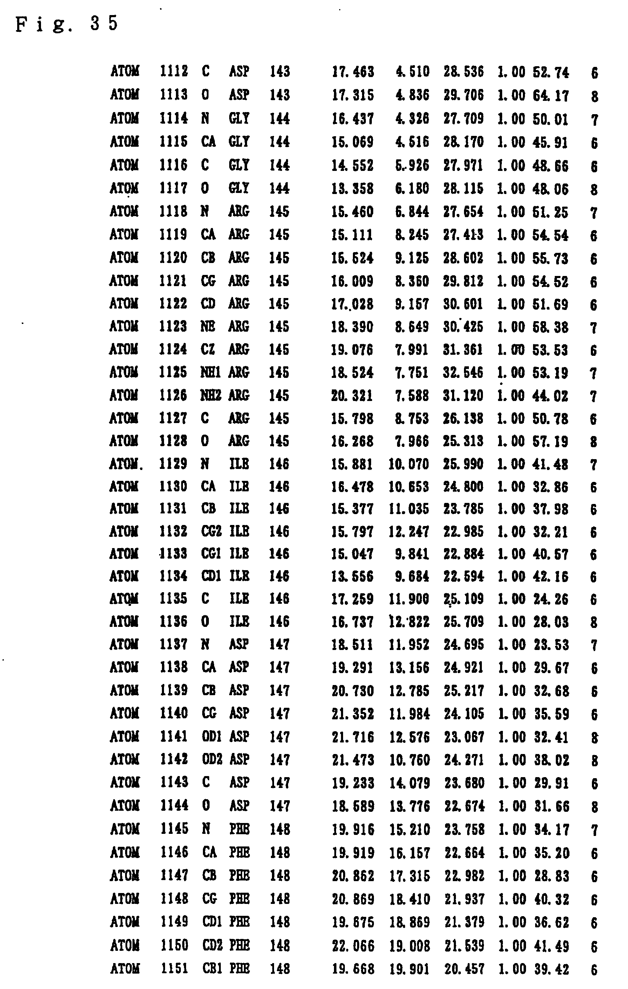

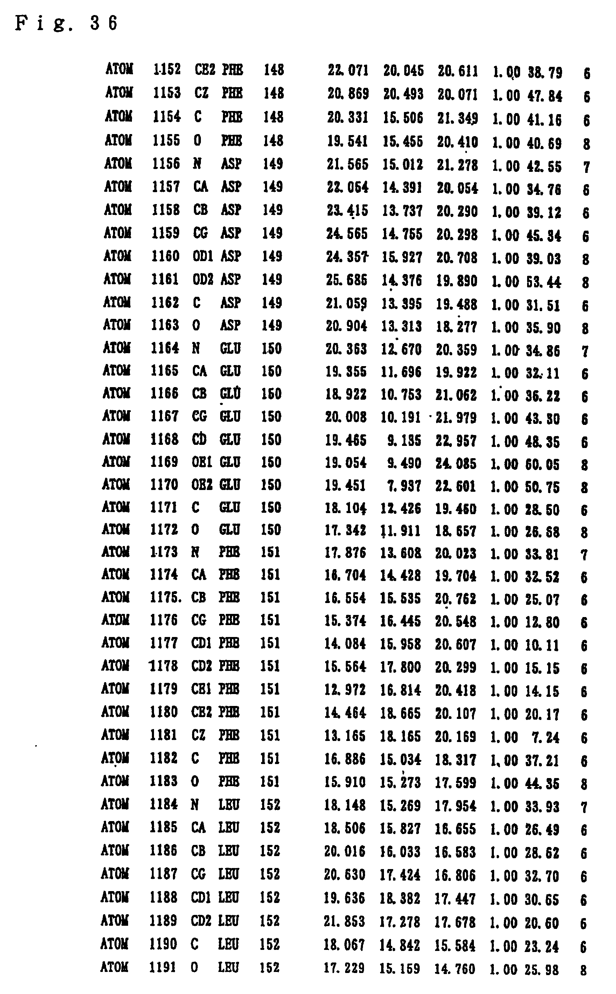

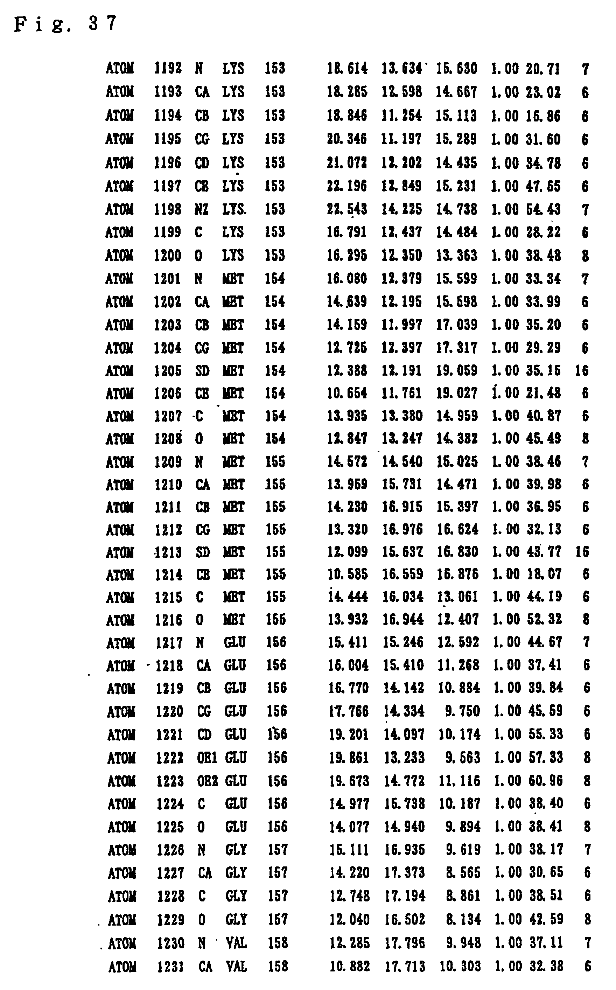

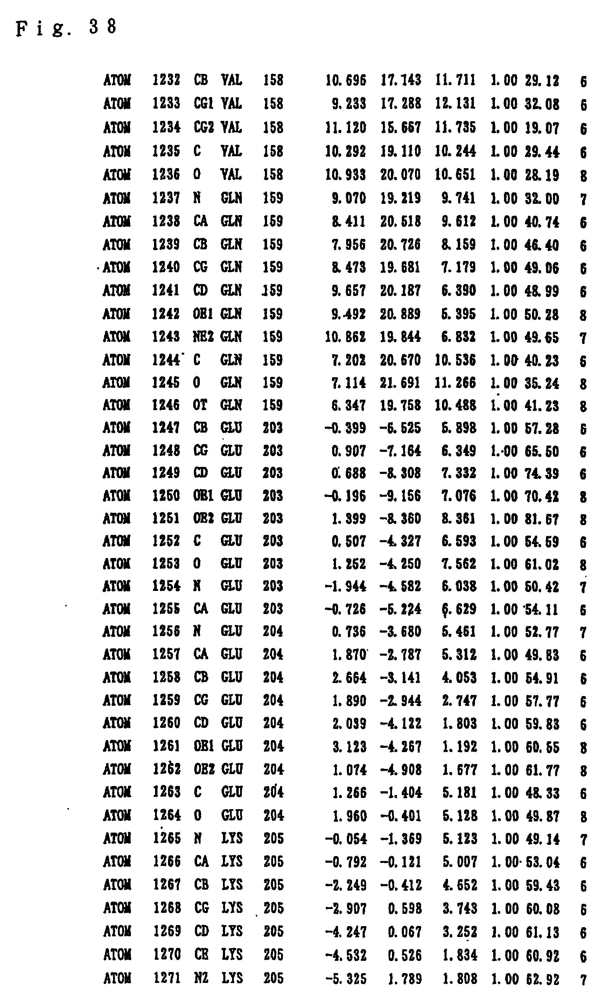

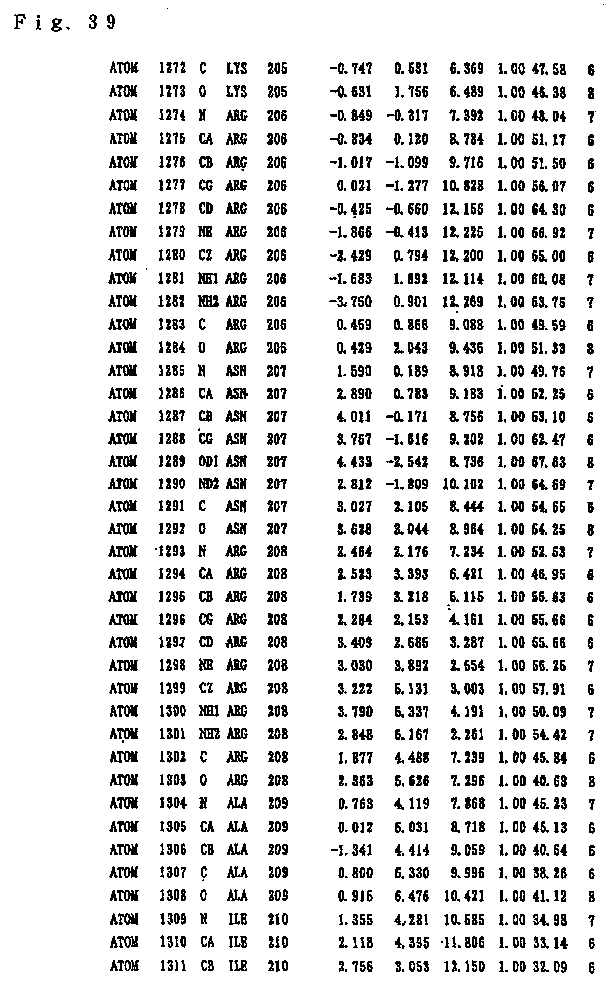

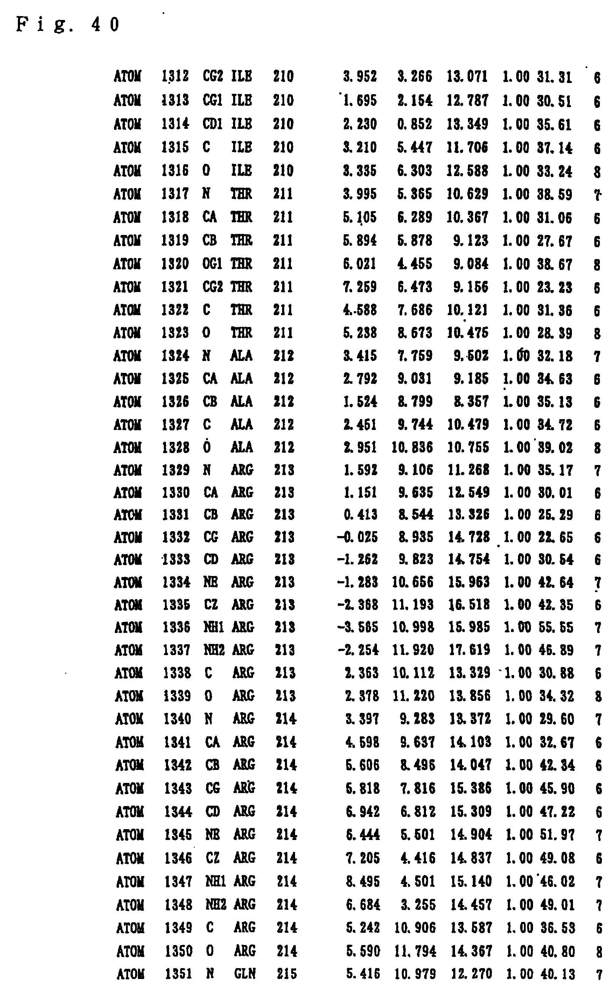

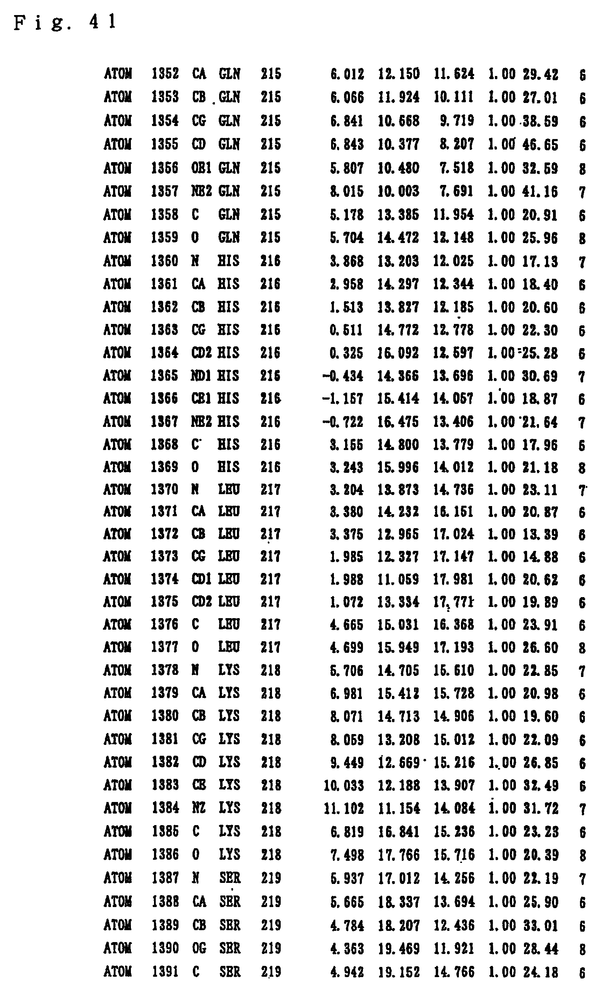

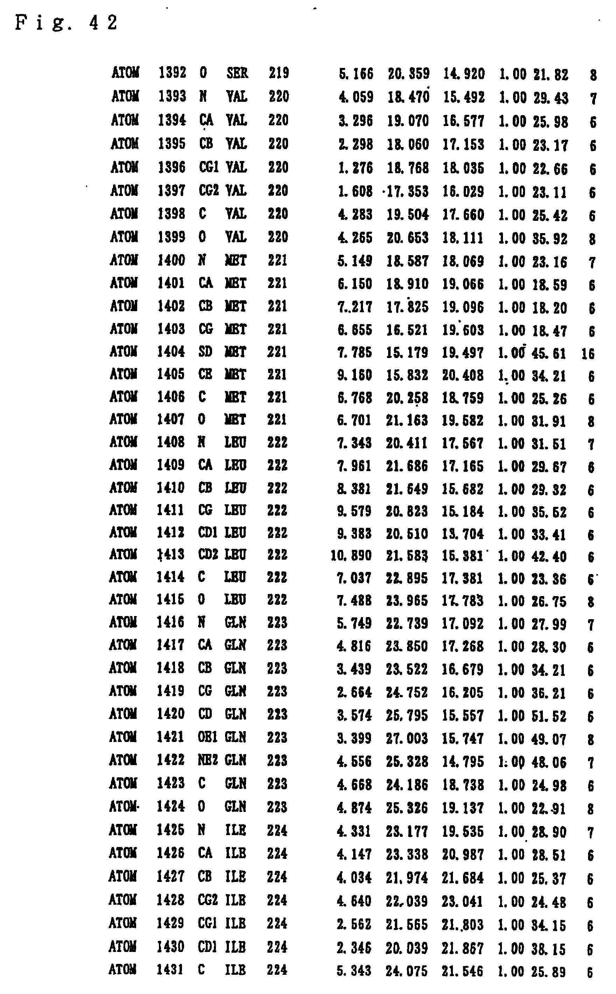

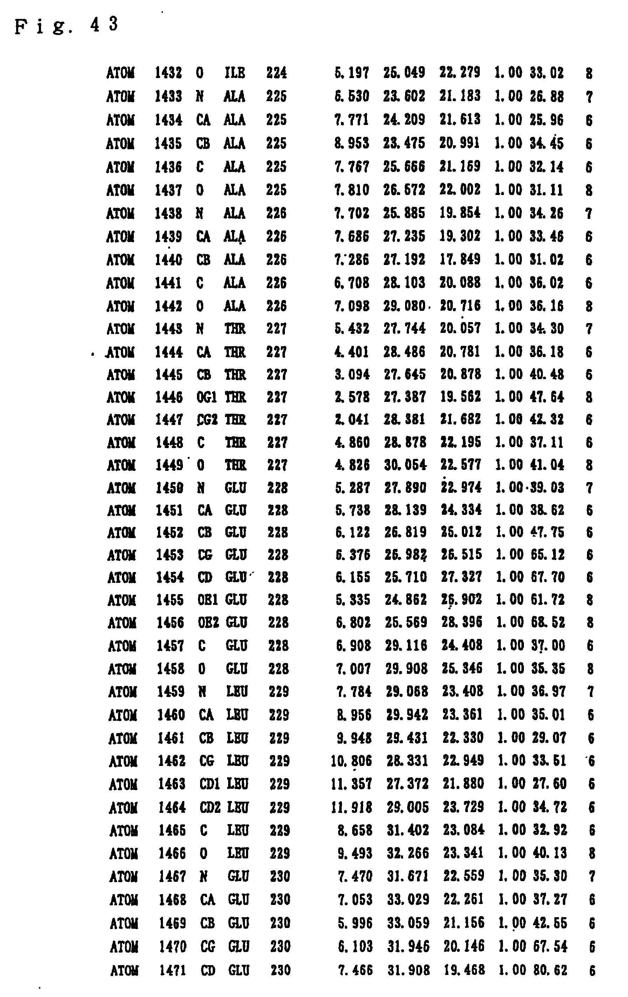

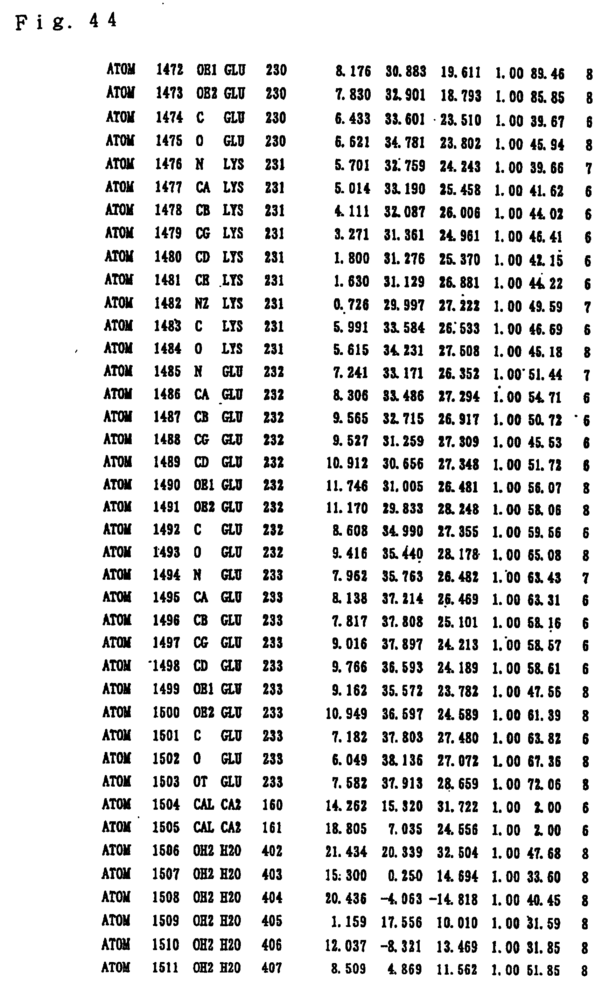

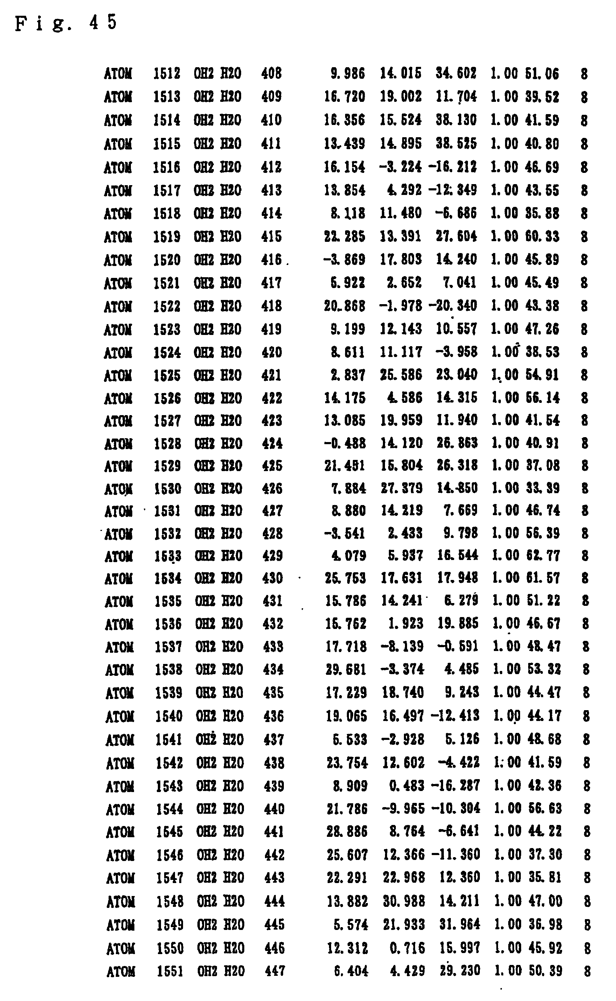

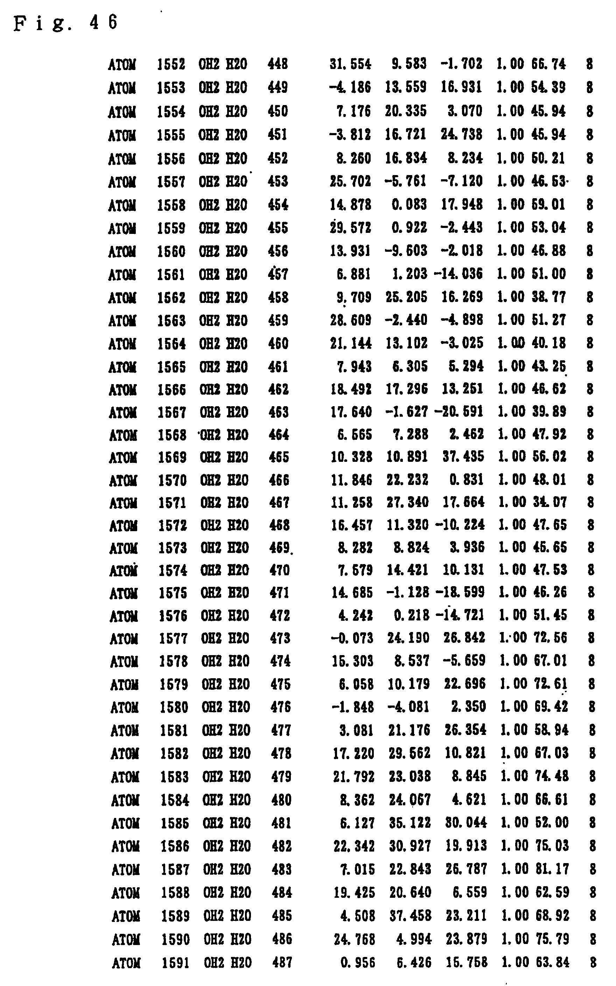

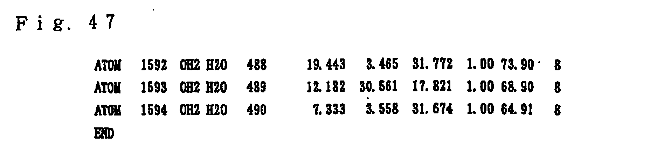

Figures. 7 to 47 show the atomic coordinates obtained from the results of X-ray analysis of the TnC/TnI$_{1-47}$ complex. Figures. 7 to 47 are described by using the format of Protein Data Bank (PDB). The PDB format is one where the coordinates (X, Y, Z) of each of atoms constituting a protein molecule and temperature factors are described in the same manner as those described in Figs. 7 to 47 and which is one of the standard formats treating the coordinates of biopolymers. The rows (from left to right) in Figs. 7 to 47 show atom's serial number, a kind of atoms (including information about bindings), a kind of residues to which the atom belongs (shown by three-letter abbreviation of amino adds), the residual number from the N-terminus, X-coordinate (Å unit), Y-coordinate (Å unit), Z-coordinate (Å unit), an occupation ratio, and an isotropic temperature factor, respectively. The name of atoms are described according to the nomenclature by IUPAC-IUB except that $\alpha,\beta,\gamma,\delta,\varepsilon,\zeta$ and $\eta$ are replaced by A, B, C, D, E, Z and H. Additional oxygen atoms in the C-terminus are represented by OXT (OT).
In Figs. 7 to 47, the serial number of atoms from 1 to 1,246 show the information of the atoms belonging to the TnC peptide chain, and from 1,247 to 1,503 show that of the atoms belonging to the TnI peptide chain, and 1,504 and 1,505 show that of calcium ions, and from 1,506 to 1,594 show that of the atoms of combined water.

THE BEST MODE FOR CARRYING THE INVENTION

The present invention will be described below in details.

X-ray analysis of the TnC/TnI$_{1-47}$ complex

The crystal structure of the complex between rabbit skeletal muscle TnC (160 residues, 18 kDa) and TnI$_{1-47}$ has been solved by the single isomorphous replacement (SIR) method in combination with the multiple anomalous dispersion (MAD) technique (Table 1).
The crystallographic R factor of crystallography was calculated including or excluding the refinement reflections. The free reflections constituted 7% of the total number of reflections.
The final model lacks two N-terminal residues of TnC, and two and 14 amino-acids from the N- and C-termini of TnI$_{1-47}$, respectively, probably owing to disorder of these residues in the crystal.

Table 1

| Summary of the TnC/TnI$_{1-47}$ complex structure determination | | | | |
|---|---|---|---|---|
| | | PCMBS derivative | | |
| Data collection | Native | λ1 | λ2 | λ3 |
| Resolution (Å) | 25.0-2.3 | 25.0-2.8 | 25.0-2.8 | 25.0-2.8 |
| Reflections | | | | |
| Total (N) | 37,214 | 22,050 | 21,747 | 23,003 |
| Unique (N) | 8,870 | 4,998 | 4,989 | 5,091 |
| Rsym* (%) | 5.0 | 5.3 | 6.0 | 5.1 |
| Completeness (%) | 96.3 | 94.1 | 94.4 | 94.8 |
| Derivatives | | | | |
| Riso**(%) | - | 22.0 | 23.2 | 24.0 |
| Phasing power*** | - | 3.0 | 3.1 | 3.0 |
| Refinement statistics | | Stereochemistry | | |
| Resolution(Å) | 10.0-2.3Å | RMSD bond length (Å) | | 0.01 |
| Number of reflections | 8,736 | RMSD bond angles (°) | | 1.28 |
| Rcryst#(%) | 22.2 | RMSD improper angles(°) | | 0.72 |
| Rfree #(%) | 32.5 | | | |
| Number of protein atoms | 1,502 | | | |
| Number of water molecules | 89 | | | |
| Number of calcium ions | 2 | | | |

The symbols in Table 1 will be explained below:

* R$_{sym}$ is calculated according to the following equation:

$$R_{sym} = \Sigma_{hkl} \Sigma_j | I_j (hkl) - < I (hkl) > | / \Sigma_{hkl} \Sigma_j | < I (hkl) > |,$$

where I$_j$ (hkl) and <I (hkl) > are the intensity of measurement j and the mean intensity for the reflection with indices hkl, respectively;
**R$_{iso}$ is calculated according to the following equation:

$$R_{iso} = \Sigma_{hkl} | | F_{der} (hkl) | - | F_{nati} (hkl) | | / \Sigma_{hkl} | F_{nati} (hkl) |,$$

where F$_{der}$ (hkl) and F$_{nati}$ (hkl) are the structure factors of the heavy atom derivative and the native for the reflection with indices hkl, respectively;
*** Phasing power is calculated according to the following equation:

$$\text{Phasing power} = < F_h > / E,$$

where < F$_h$ > is the root mean square of heavy-atom structure factor and E is the residual lack of closure error:
#R$_{cryst}$, free is calculated according to the following equation:

$$R_{cryst, free} = \Sigma_{hkl} | | F_{calc} (hkl) | - | F_{obs} (hkl) | | / \Sigma_{hkl} | F_{obs} |.$$

Results of X-ray analysis of the TnC/TnI$_{1-47}$

The atomic coordinates obtained from the X-ray analysis of the TnC/TnI$_{1-47}$ complex are shown in Figs. 7 to 47.

How to read the Figures is the same as those in described in the Brief Description of Drawings.

In Figs. 1 to 47, each section with the serial number of atoms from 1 to 1,246 shows the information of each atoms belonging to the peptide chain of TnC, from 1,247 to 1,503 show those of the atoms belonging to the peptide chain of TnI, 1,504 and 1,505 show those of calcium ions, and from 1,506 to 1,594 show those of combined water.

Explanation of the results of X-ray analysis of the TnC/TnI$_{1-47}$ complex

The conformation of TnC in the TnC/TnI$_{1-47}$ complex is strikingly different from that of uncomplexed TnC (TnC$_{free}$), in which the two domains are connected by a long $\alpha$-helix, and thus are 20Å apart from each other. In the complex, this connecting D/E $\alpha$-helix is unwound at the center (four residues 88-91) and is transformed into an extended linker between the two TnC domains. The 120° relative rotation of the TnC domains accompanies the unwinding of the D/E $\alpha$-helix, which results in a 90° bend between its D- and E-portions. As a result of these conformational changes, the TnC molecule in the TnC/TnI$_{1-47}$ complex has a compact globular shape, with approximate dimensions of 50x30x30Å (Fig. 1). The N- and C-domains come into dose proximity and form a deep narrow interdomain pocket.

There are two direct interdomain hydrogen bonds in TnC within the TnC/TnI$_{1-47}$ complex. Only two calcium ion-binding sites (EF-hands) in the C-domain of TnC in the complex are occupied by calcium ions, while the two other EF-hands in the N-domain are empty, as was observed in the TnC$_{free}$ structure (Fig. 1). Thus, the conformation of each TnC domain itself in the TnC/TnI$_{1-47}$ complex is very similar to that in the TnC$_{free}$ structure. The root mean square deviations between all C $\alpha$ atoms of TnC$_{free}$ and TnC in the TnC/TnI$_{1-47}$ complex are 1.0Å and 1.2Å for the N-and C-domains, respectively.

The TnI$_{1-47}$ fragment in the TnC/TnI$_{1-47}$ complex is represented by a 31-residue-long $\alpha$-helix that is slightly bent toward the concave surface of the TnC molecule (Fig. 1). Fourteen residues at the C-terminal end of TnI$_{1-47}$ are disordered in the crystal, and probably do not have contacts with TnC. The TnI$_{1-47}$ $\alpha$-helix makes multiple polar and van der Waals interactions with both domains of TnC. According to these interactions, the TnI$_{1-47}$ $\alpha$-helix may be divided into two parts. The N-terminal (residues 3-12) and C-terminal (residues 13-33) portions of this $\alpha$-helix are bound to the N- and C-domains of TnC, respectively. Arg14 of TnI$_{1-47}$ is located at the interface between the domains of TnC and bridges them by four strong hydrogen bonds (Fig. 2). The C-terminal part of the TnI $\alpha$-helix is tightly bound to the C-terminal domain of TnC by 11 hydrogen bonds. In addition, there are 38 van der Waals interactions (<4Å) between the amphiphilic C-terminal end of TnI$_{1-47}$ $\alpha$-helix and the TnC residues in the hydrophobic pocket of the C-domain (Fig. 2). Thus, the hydrophobic residues of TnI$_{1-47}$ and TnC complement each other and constitute the hydrophobic core of the TnC/TnI$_{1-47}$ complex.

The present inventors believe that these hydrophobic interactions play a major role in the recognition between TnC and TnI$_{1-47}$. It is worthwhile to mention that Met21 of TnI$_{1-47}$ seems to be of central importance for the recognition; its bulky side chain deeply protrudes into the hydrophobic pocket of the TnC C-domain and makes a total of seven strong van der Waals contacts with the TnC residues (Figs. 2 and 3).

The interactions of TnI$_{1-47}$ with the N-domain of TnC are weaker than those with the C-domain. There are only four direct and three water-mediated hydrogen bonds (Fig. 2). It is likely that these interactions are required mostly to anchor the TnC N-domain to TnI to stabilize the compact conformation of TnC, and do not play an essential role in recognition.

At first sight, the structure of the TnC/TnI$_{1-47}$ complex suggests that the number of unwound residues in the central $\alpha$-helix, and hence, the length of the entire D/E $\alpha$-helix, must be very important to achieve the proper orientation of the TnC domains upon the binding to TnI. Thus, the retention of activity by TnC mutants with deletions in the central $\alpha$-helix is surprising (Dobrowolski, Z., Xu, G. Q., Chen, W. and Hitchcock, D. S., Analysis of the regulatory and structural defects of troponin C central helix mutants, Biochemistry 30, 7089-7096 (1991)). In brief, the deletion of three, four or seven amino acids in the D/E $\alpha$-helix (residues 83-94) did not seriously impair the calcium ion regulation of muscle contraction, while the deletion of more than seven residues caused a substantial loss of the Tn regulatory activity.

To explain the small effect of the length of the TnC D/E $\alpha$-helix on the regulatory activity of Tn, the present inventors built models of seven- and twelve-residues deletion mutants of TnC on the basis of the TnC/TnI$_{1-47}$ structure (data not shown). The analysis of the TnC structure in the complex showed that the residues 83 to 94 in the D/E $\alpha$-helices, including the linker region (residues 88-91), have no strong polar interactions with either TnI$_{1-47}$ or the TnC domains. Therefore, it is likely that upon the deletion, any of these residues could be easily unwound to compensate for the deletions, so that the intra- and inter-domain conformations of TnC are retained. Our modeling showed that the deletion of seven residues would require unwinding of the five residues in the D-helix (exactly residues 83-94), which should not strongly alter the TnC conformation. In contrast, the deletion of twelve residues would need either the unwinding of eight residues in the central α-helix (residues 83-102), which would extend to functional residues in the C-domain, or a substantial change in the interdomain orientation. In either case, the deletion would probably disturb the interactions between the subunits in Tn and result in a loss of activity.

A mechanism of the TnC/TnI$_{1-47}$ complex formation, derived from the results of the X-ray structure analysis of the TnC/TnI$_{1-47}$ complex

What is a mechanism of the TnC/TnI$_{1-47}$ complex formation ?

The TnI$_{1-47}$ α-helix would probably bind first to the C-domain of elongated TnC$_{free}$, as the interactions of TnI$_{1-47}$ with the C-domain are much stronger in the complex than those with the N-domain of TnC. At this stage, no contact would occur between TnI$_{1-47}$ and the N-domain of TnC. Thus, the question is how TnC can adopt the compact conformation observed in the TnC/TnI$_{1-47}$ complex.

The NMR studies of the TnC molecule itself at neutral pH (7.0) show that the domain orientation is not fixed, and that the central portion of the connecting D/E α-helix is flexible in solution (Slupsky, C.M. & Sykes, B.D., NMR solution structure of calcium-saturated skeletal muscle troponin C, Biochemistry 34, 15953-15964 (1995)).

The proper conformation of TnC in the TnC/TnI$_{1-47}$ complex is induced, therefore, by the binding of TnI$_{1-47}$ to its C-domain, and by the free motions of the TnC domains in a concerted manner. The binding of TnI$_{1-47}$ to the TnC C-domain facilitates its rotation in a favorable direction and increase the flexibility of the D/E α-helix. Once the proper conformation of TnC is achieved, it is fixed by interdomain interactions, together with hydrogen bonds of the TnC N-domain to TnI$_{1-47}$ (Fig. 2).

The overall conformation of TnC and the rearrangement of the TnC domains induced by TnC/TnI$_{1-47}$ complex formation resemble another calcium ion-receptor protein, calmodulin, in the complex formation with its target peptides (Ikura, M., Clore, G. M., Gronenborn, A. M., Zhu, G., Klee, C. B. and Bax, A., Solution structure of a calmodulin-target peptide complex by multidimensional NMR, Science 256, 632-638 (1992); Meador, W. E., Means, A. R. and Quiocho, F. A., Modulation of calmodulin plasticity in molecular recognition on the basis of x-ray structures, Science 262, 1718-1721 (1993)).

The extended dumbbell-shaped molecules of both proteins undergo substantial conformational changes upon binding to other proteins. In both cases, the dramatic reorientation of the domains accompanies the unwinding and the bend of the central connecting α-helix, resulting in the compact globular protein structure. Moreover, the target peptides of both proteins adopt α-helical conformations, and the recognition mechanism involves multiple hydrophobic interactions between the proteins and the amphiphilic portions of the bound peptides. The latter feature also resembles the binding between the myosin light and heavy chains (Xie, X. et al., Structure of the regulatory domain of scallop myosin at 2.8Å. Nature 368, 306-312 (1994)).

However, there is an essential difference between TnC and calmodulin (or myosin light chain) in the mode of binding to the substrates. In the calmodulin/peptide complexes, the hydrophobic binding sites of both domains are turned toward each other and toward the center of the molecule. This orientation allows both domains of calmodulin to interact simultaneously with two region of the same target α-helix. In contrast, in the TnC/TnI$_{1-47}$ complex, the hydrophobic pockets in the C-and N-domains are turned out of the molecular center and are located on opposite sides of the molecular surface. As a result, the binding of a single α-helix to the hydrophobic pockets of both domains of TnC, as in the calmodulin complexes, is impossible.

A mechanism of the calcium switch for muscle contraction, derived from the results of the X-ray structure analysis of the TnC/TnI$_{1-47}$ complex

How the inhibition of acto-myosin ATPase is released is very important in the studies of mechanisms of regulation of the muscle contraction. Therefore, in the past years many biochemical and biophysical works were focused on the inhibitory segment of TnI (residues 96-116) (Syska, H., Wilkinson, J. M., Grand, R. J. A., Perry, S. V., The relationship between biological activity and primary structure of troponin I from white skeletal muscle of the rabbit, Biophys. J. 153, 375-387 (1976); Farah, C. S. and Reinach, F. C., The troponin complex and regulation of muscle contraction, Faseb. J. 9, 755-767 (1995)).

The inhibitory segment was shown to bind to either actin-tropomyosin or TnC, thus switching on and off the activity of acto-myosin ATPase. On the other hand, the conformational changes between "closed" and "open" forms of TnC regulatory N-domain induced by calcium ion-binding are also involved in the regulation of muscle contraction. The C- and N-domains of TnC are very similar to each other in both sequence and structure in calcium ion-bound states (Herzberg, O., Moult, J. and James, M. N., A model for the Ca$^{2+}$-induced conformational transition of troponin C, A trigger for muscle contraction, J. Biol. Chem. 261, 2638-2644 (1986); Gange, S. M., Tsuda, S., Li, M. X., Smillie, L. B. and Sykes, B. D., Structures of the troponin C regulatory domains in the apo and calcium-saturated states, Nat. Struct. Biol. 2, 784-789 (1995)). Thus, the TnC/TnI$_{1-47}$ structure suggests that "open" N-domain of TnC would bind to another

amphiphilic $\alpha$-helix in a symmetrical manner.

In view of important regulatory role of TnI inhibitory segment, it is likely that this $\alpha$-helix is located in the vicinity of the inhibitory peptide in the TnI. Indeed, such a hydrophobic segment exists at the C-terminal end of the TnI inhibitory sequence, and was predicted to have an $\alpha$-helical conformation (Pearlstone, J. R, Sykes, B. D. and Smillie, L. B., Interactions of structural C and regulatory N domains of troponin C with repeated sequence motifs in troponin I, Biochemistry 36, 7601-7606 (1997)). This region is highly conserved among the TnIs from different species (Fig. 4).

The N-terminal and "inhibitory" sequences are similar to each other in the general hydrophobicity of the amino acids, although the sequence homology is low (Fig. 4). Further, the methionine residue (Met121), corresponding to Met21 at the N-terminus, which was proposed to play an important role in the recognition between TnC and $TnI_{1-47}$, is conserved in the "inhibitory" sequence (Fig. 4). The measurements of the binding constants of two TnI inhibitory fragments to TnC showed that $TnI_{96-116}$ preferably interacts with the C-domain of TnC, while the longer fragment in C-terminal direction, $TnI_{96-148}$, is most tightly bound to the TnC N-domain (Pearlstone, J. R, Sykes, B. D. and Smillie, L. B., Interactions of structural C and regulatory N domains of troponin C with repeated sequence motifs in troponin I, Biochemistry 36, 7601-7606 (1997)).

At high calcium ion concentrations, the sequence at the C-terminal end of the TnI inhibitory sequence was protected against proteolysis (Takeda, S., Kobayashi, T., Taniguchi, H, Hayashi, H. and Maeda, Y., Structural and functional domains of troponin I revealed by limited digestion of the troponin ternary complex, Eur. J. Biochem. 246, 611-617 (1997)) and was shown to be involved in the regulatory activity of Tn (Farah, C. S. et al., Structural and regulatory functions of the $NH_2$-and COOH-terminal regions of skeletal muscle troponin I, J. Biol. Chem. 269, 5230-5240 (1994)).

The biochemical data and the results of X-ray structure of the $TnC/TnI_{1-47}$ complex indicate that the TnI inhibitory region could extend to the regulatory TnI fragment ($TnI_{reg}$, residues 96-127), in which the amphiphilic C-terminal end (residues 117-127) plays a major regulatory role in the relief of the inhibition of the actomyosin magnesium ion-ATPase, through binding to the hydrophobic pocket of the TnC N-domain in its "open" state.

Construction of Three-dimensional Model For The $TnC/TnI_{reg}$ Complex

The present inventors have constructed the three-dimensional model of $TnC/TnI_{reg}$ complex (Fig. 5, see Methods). The $TnI_{reg}$ model can be divided into two parts; the N-terminal part (residues 96-107) interacts with the C-domain of TnC, while the C-terminal region (residues 108-127) is strongly bound to the TnC N-domain (Fig. 5). In the $TnC/TnI_{reg}$ model, seven hydrophobic residues at the C-terminal region of $TnI_{reg}$ interact with the thirteen TnC residues in the hydrophobic pocket of the N-domain (Fig. 6). Interestingly, according to our model, Phe 100 of $TnI_{reg}$ forms multiple hydrophobic contacts with Phe145 and Leu155 of TnC, which increases the affinity of $TnI_{reg}$ to the TnC C-domain.

The $TnC/TnI_{reg}$ model demonstrates the mechanism of regulation of muscle contraction by Tn complex. Upon the binding of calcium ion, the conformation of regulatory N-domain of TnC is changed from "closed" to "open" state. This structural transformation of TnC facilitates the relief of inhibition of acto-myosin ATPase by $TnI_{reg}$. Thus, $TnI_{reg}$ preferably binds to TnC and its amphiphilic C-terminal end is locked in the hydrophobic pocket of TnC N-domain through the multiple van der Waals interactions.

The release of calcium ion from N-domain of TnC initiates its conformational change into the "closed" form, which pushes the amphiphilic $\alpha$-helix of $TnI_{reg}$ out of the pocket. After "closing" of TnC N-domain, this hydrophobic $\alpha$-helix can not bind on the surface of TnC covered with hydrophilic residues. Thus, the amphiphilic $TnI_{reg}$ $\alpha$-helix is able to bind to a part of hydrophobic site in the actin molecule, which increases the affinity of $TnI_{reg}$ to the actin, and facilitate its release from the TnC and the binding to the actin-tropomyosin.

The $TnC/TnI_{1-47}$ complex structure and the $TnC/TnI_{reg}$ model have several important implications.

(1) Within Tn, the TnC molecule has a compact globular shape with direct polar interactions between the N- and C-domains.

(2) The N-terminal regions of TnI and $TnI_{reg}$ have $\alpha$-helical structures and are bound to TnC in an anti-parallel pseudo-symmetrical manner.

(3) The recognition of $TnI_{1-47}$ and $TnI_{reg}$ by TnC is accomplished by hydrophobic interactions, in which Met21 ($TnI_{1-47}$) and Met121 ($TnI_{reg}$) are probably the key residues.

(4) $TnI_{reg}$ binds to both domains of TnC in the presence of calcium ion, and hence, it alone may induce the functional, compact conformation of TnC. Consistent with this, an N-terminal deletion mutant, $TnI_{58-182}$, can still constitute a functional Tn, but only at high calcium ion concentrations (Potter, J. D., Sheng, Z., Pan, B-S. and Zhao, J., A direct regulatory role for troponin T and a dual role for troponin C in the $Ca^{2+}$ regulation of muscle contraction, J. Biol. Chem. 270, 2557-2562 (1995)).

(5) The two-proline kink in the $TnI_{reg}$ $\alpha$-helix is probably required to direct the N-terminal end of $TnI_{reg}$ towards the TnC C-domain.

(6) The difference between calmodulin and TnC in the binding to their targets accounts for their distinct biological functions. Calmodulin functions as an isolated protein; thus, both of its domains are involved in signal transduction. In contrast, TnC is integrated into the Tn complex through its C-domain, and only the N-domain possesses regulatory activity.

In conclusion, the binding of $TnI_{1-47}$ to the TnC C-domain is likely to play a primarily structural role in the formation of Tn, while the amphiphilic C-terminal end of $TnI_{reg}$ forms the actual switch that moves between actin-tropomyosin and the hydrophobic pocket of the N-domain of TnC in a calcium ion dependent manner, thereby regulating the process of muscle contraction.

Use Of TnC/TnI Complex

As described in details above, the present inventors have prepared the complex between troponin C and an N-terminal fragment of troponin I ($TnI_{1-47}$), crystallized it, and determined the atomic coordinates of the crystal at 2.3 Å resolution, in order to elucidate interactions between troponin subunits as well as a conformation of troponin C in the troponin complex.

As a result, it has become evident that the structure of TnC in the complex has a compact globular shape in contrast to the elongated dumbbell-shaped molecule of uncomplexed TnC. A long $\alpha$-helix stretches on the surface of TnC and it has multiple contacts with both domains of TnC. The amphiphilic C-terminus of $TnI_{1-47}$ is firmly bound in the hydrophobic pocket of the C-domain of TnC. The results indicate the major difference between the complexed TnC and the isolated calcium ion receptor (calmodulin).

The steric structure of the $TnC/TnI_{1-47}$ complex revealed by the present invention remains unchanged, even when $TnI_{1-47}$ is replaced by TnI itself, a peptide containing $TnI_{1-47}$ and which is capable of forming a complex with TnC, or a peptide obtained by a single or multiple deletion, addition, insertion or substitution of one or more amino add residues in $TnI_{1-47}$ fragment and which is capable of forming a complex with TnC, as far as these peptide are capable of forming a complex with TnC.

A peptide containing $TnI_{1-47}$ and which is capable of forming a complex with TnC may be, for example, prepared by cloning a polynucleotide obtained by cleavage of the polynucleotide encoding TnI with a restriction enzyme having a restriction site at the 48 position or thereafter of the corresponding amino acid sequence and expressing it in an Escherichia coli strain according to a conventional method. Such restriction enzymes include, for example, Pvu II and Eag I. Alternatively, a polypeptide having 63 or 132 amino add residues and which is capable of binding to TnC may be prepared by the chemical cleavage by Swenson & Fredricson (Swenson & Fredricson, Biochemistry 31, 3420-3429 (1992)) of a mutant polypeptide of TnI whose cysteins are substituted with other amino acids except for those at 64 or 133 positions, respectively.

A peptide containing a derivative obtained by a single or multiple deletion, addition, insertion or substitution of one or more amino acid residues in the $TnI_{1-47}$ fragment mentioned above and which is capable of binding to TnC to form a complex may be prepared by conducting a mutation of the polynucleotide encoding TnI, leading to deletion, addition, insertion or substitution of amino acid residues in the corresponding amino acid sequence according to the conventional site-directed mutagenesis (Sambrook, Fritch & Maniatis, Molecular Cloning, A laboratory manual, 2nd Edition, Chapter 15) and then expressing the mutant polynucleotide thus obtained in an Escherichia coli strain according to a conventional method.

Further, investigation of compounds that exert on influence on calcium ion binding to the TnC/TnI complex makes it possible to screen drugs capable of exerting an influence on muscle contraction. These drugs include active agents capable of exerting an influence on the binding between TnC and TnI and those capable of exerting an influence on the binding between TnC and calcium ion and the like. The present invention also provides for such screening systems for active substances capable of exerting an influence on the muscle contraction.

The "active substances capable of exerting an influence on the muscle contraction" thus obtained offer potential medicaments for heart disorders on the basis of a novel type of action mechanism mentioned above such as those for heart failure, cardiomyopathy and myocardial infarction. There may be also a possibility that the active substances would include an effective medicament for neuromuscular disorders such as myasthenia gravis and myodystrophia which are known as intractable diseases and for which no effective medicament have been found so far.

Such screening systems, for example, include a method comprising a dissolving an appropriate amount of the TnC/TnI complex of the present invention and a substance to be tested in a suitable buffer dose to physiological conditions followed by measuring the strength of calcium ion binding to the complex by the equilibrium dialysis or methyl murexide method (Ogawa, Y. and Ebashi, S., "Measurements for Calcium Binding", In Ebashi ed. "Development of Techniques for Heart and Blood Vessel Study", Gakkai-syuppan Center (1983)) and a method wherein a strength of binding between TnC and TnI is determined by introducing a fluorescent pigment into a suitable position of one of both proteins and recording the change of the fluorescence strength (Pearlstone, J. R., Sykes, B. D. & Smillie, L. B., Interactions of structural C and regulatory N domains of troponin C with repeated sequence motifs in troponin I, Biochemistry 36, 7601-7606 (1997)). In the above method, the fluorescent pigment may be bound to, for example, cystein residues introduced by the genetic engineering techniques, and introduction of a fluorescent amino add residue also can be performed by the genetic engineering techniques.

EXAMPLES

The present invention will be described, hereafter, by means of the following examples and reference examples which should not any way be construed as limiting.

The genetic engineering techniques are, unless otherwise specified, conducted primarily according to Sambrook, Fritch & Maniatis, A Laboratory Manual: Molecular Cloning, 2nd ed., Cold Spring Harbor Laboratory Press.

Reference Example 1

Construction of expression vectors of troponin C and troponin I

The cDNA of troponin C, a component of the troponin complex of the rabbit muscle was given by Dr. J. D. Potter of Miami Medical College. The cDNA of troponin I was cloned by Dr. L. Kluwe who created also mutant TnIs (C64 and C133) used in this experiment (L. Kluwe et al., FEBS Letters 323, 83-88 (1993)).

Construction of an expression vector for troponin C

The cDNA of troponin C was amplified by a polymerase chain reaction (PCR) (15 cycles of denaturation at 94°C, annealing at 54°C and extension reaction at 72°C on a Zymoreactor II model AB-1820, Atto), which was carried out using cDNA of troponin C as a template and oligonucleotides as set forth in SEQ ID NO:1 and SEQ ID NO:2 as primers in 100 μl size. The amplified troponin C cDNA was digested with restriction enzymes, PvuI and BamHI (Takara) and the fragments thus obtained were separated by electrophoresis. The resulting troponin C fragment, an expression vector pMAL-c (Biolabs, New England) digested with restriction enzymes, XmnI and BamHI (Takara) and annealed oligonucleotides as set forth in SEQ ID NO:3 and SEQ ID NO:4, respectively were incubated with T4 DNA ligase (Takara) at 16°C for 6 hours in a 10 μl of a reaction solution (66 mM Tris-HCl (pH 7.6), 6.6 mM $MgCl_2$ , 10 mM dithiothreitol, 0.1 mM ATP) for ligation to construct an expression vector for troponin C (pMALTnC).

(2) Construction of an expression vector for troponin I

The wild type of troponin I has 3 cysteins (at the 48, 64 and 133 positions). In order to prepare $TnI_{1-47}$, a mutant polynucleoticle having mutations of cysteines at the 64 and 133 positions while the cysteine at the 48 position unchanged was used.

The polynucleotide in which Cys at the 64 position was changed into Ala and Cys at the 133 position into Ser (cDNA-TnI (C64A, C133S)) was digested with restriction enzymes, NcoI (Takara) and EcoRI (Takara), harvested and ligated to an expression vector pTrc99c (Amann, E. et al., Gene 69, 301-315 (1988)) which was previously digested with NcoI and EcoRI in the same manner above by T4DNA ligase to form pTrcTnI.

Reference Example 2

Expression of troponin C and troponin I in E. coli cells

E. coli AD202 strain (ompT- strain) (Akiyama, Y. and Ito, K., B.B.R.C. 167, 711-715 (1990)) was transformed with either the troponin C expression vector, pMALTnC or troponin I expression vector, pTrcTnI. The transformants were incubated in 10 ml of the LB medium (GIBCO BRL) at 37°C over night, then inoculated in 1 liter of the LB medium and allowed to grow. When the OD at 600 nm of the culture broth reached 0.8-1.0, 0.1 mM isopropylthiogalactoside (IPTG) was added to the culture broth to induce the expression of a maltose-conjugated troponin C fusion protein or maltose-conjugated troponin I fusion protein. After further cultivation for additional 18 hours, the cells were collected by a centrifugation (on a Beckman JS-4.2, for 20 min. at 4000 rpm).

Reference Example 3

Preparation of TnC

About 40 g of the cells obtained in Reference Example 2 were suspended in 200 ml of a buffer solution containing 20 mM Tris-HCl (pH 8.0), 20 % sucrose, 1 mM EDTA. To the suspension lysozyme (Sigma) was added to final concentration of 0.5 mg/ml and allowed to stand at the room temperature for 30 min. After that the cells were ultrasonicated (on a Tommy Seiko sonicator model UD-201, at 4°C, 50 % interval, total 10 min. sonication). The sonicates obtained were centrifuged using a rotor JA20 on a Beckman centrifuge (Model J2M1) at 4°C and 30000 rpm for 20 min. To the obtained supernatant, ammonium sulfate was added to give a final concentration of 35% saturated at 4°C, and centrifugation was conducted by using the same rotor as described above at 4°C, 30000 rpm for 20min. The supernatant was harvested and chromatographed on a hydrophobic column.

The supernatant was charged on a column (2.5 x 10 cm, phenyl-sepharose CL-4B, Pharmacia) previously equilibrated with a buffer solution containing 20 mM Tris-HCl (pH 8.0), 50 mM NaCl, 5 mM $CaCl_2$, 1 mM $MgCl_2$, 1 mM dithiothreitol and washed well with the same buffer. The column was then washed with another buffer with a high salt concentration containing 20 mM Tris-HCl (pH 8.0), 1 M NaCl, 0.1 mM $CaCl_2$, 1 mM dithiothreitol, followed by elution of proteins with an elution buffer containing 20 mM Tris-HCl (pH 8.0), 1 mM EDTA and 1 mM dithiothreitol. The eluted fractions of proteins were collected. Detection of proteins was carried out using a peristaltic pump at a flow rate of about 2 ml/min while monitoring absorption at 280 nm by means of a UV spectrophotometer.

After confirmation of the presence of the maltose-conjugated TnC, a fusion protein by a SDS electrophoresis, the maltose-conjugated TnC fractions were collected and incubated with Factor Xa (Behringer-Manheim) (1/10 (w/w) of Tnc) at 25°C for 2 hours in a buffer solution containing 100 mM NaCl, 50 mM Tris-HCl (pH 8.0) and 1 mM $CaCl_2$ to cleave the fusion protein, resulting in release of TnC. And then a hydrophobic chromatography was carried out twice under the same conditions described above to give the TnC fraction. The fraction was dialyzed against 2 liters of an outer solution containing 20 mM Tris-HCl (pH 8.0), 0.1 M NaCl, 1 mM EDTA and 1 mM dithiothreitol.

To the inner solution of the dialysis was added solid urea to give a final concentration of 6M and the resulting solution was charged onto an anion exchange column (2.5 x 10 cm, Q-Sepharose fast-flow, Pharmacia) previously equilibrated with a buffer solution containing 20 mM Tris-HCl (pH 8.0), 6 M urea, 0.1 M NaCl, 1 mM EDTA and 1 mM dithiothreitol. The column was washed with a buffer solution containing 20 mM Tris-HCl (pH 8.0), 6 M urea, 0.1 M NaCl, 1 mM EDTA and 1 mM dithiothreitol, followed by elution of proteins with an NaCl salt concentration gradient of from 0.1 M to 0.5 M.

After confirmation of the fractions containing TnC by means of an SDS electrophoresis, the fractions were further purified by a reverse phase high performance liquid chromatography. It was carried out using an Aquapore butyl column (Brownlee) (10 x 300 mm), eluted with an acetonitrile concentration gradient (0-100 %) in the presence of 0.1 % trifluoroacetic acid at flow rate of 3 ml/min., and detected the elution of TnC by absorbance at 280 nm using a UV absorbance detector. The fractions containing TnC were dried using a Tommy Seiko centrifugal concentrator (Model CC101) and stored at -20°C.

Reference Example 4

Preparation of $TnI_{1-47}$

(1) Extraction and purification of TnI

About 40 g of the cells obtained in Reference Example 2 were suspended in 200 ml of a STET solution containing 8% sucrose, 50 mM Tris-HCl (pH 8.0), 50 mM EDTA and 5% Triton X-100. Lysozyme was added to the suspension to give a final concentration of 0.5 mg/ml. The suspension was allowed to stand at the room temperature for 30 min and then ultrasonicated (on a Tommy Seiko sonicator model UD-201, at 4°C, 50% interval, total 10 min). The sonicates were centrifuged at 30,000 rpm and 4°C for 20 min. using a Beckman centrifuge Model J2M1 and a rotor Model JA20. The obtained precipitates were suspended in the STET solution, ultrasonicated again and centrifuged again under the same conditions described above. The same procedure was repeated again and then the same procedure was repeated twice using a different solution containing 50 mM Tris-HCl (pH 8.0) and 5 mM EDTA. Obtained precipitates were suspended in a urea-buffer solution containing 6 M urea, 0.1 M NaCl, 20 mM Tris-HCl (pH 8.0), 1 mM EDTA and 1 mM dithiothreitol, ultrasonicated (on a Tommy Seiko sonicator model UD-201, at 4°C, 50 % interval, total 5 min), and centrifuged again under the same conditions described above to give a supernatant.

The obtained supernatant was charged onto a DE52 column (Whatman, 5 x 5 cm) equilibrated with the urea-containing buffer solution containing 6 M urea, 0.1 M NaCl, 20 mM Tris-HCl (pH 8.0), 1 mM EDTA, 1 mM dithiothreitol. The flow-through fraction was adsorbed on a SP-sepharose fast-flow column (Pharmacia, 2.5 x 10 cm) equilibrated with the same buffer solution and developed with an NaCl concentration gradient of 0.1 to 0.4 M. The fractions containing TnI were detected by SDS electrophoresis and pooled.

The obtained fractions were further purified through reverse phase HPLC using an Aquapore butyl column (Brownlee) (10 x 300 mm) and eluting at a flow rate of 3 ml/min with an acetonitrile concentration gradient of 0 to 100% in the presence of 0.1% trifluoroacetic acid. Elution of TnI was confirmed with a UV absorption detector at 280 nm absorbance. The obtained fraction were dried on a centrifugal concentrator and stored at -20°C.

### (2) Preparation of TnI$_{1-47}$ fragment

The TnI$_{1-47}$ fragment was obtained by a chemical treatment of a TnI mutant (C64A, C133S) in which a cystein residue resides only in the 48 position. The chemical cleavage was essentially carried out by the method of Swenson and Fredricson, Biochemistry 31, 3420-3429 (1992). Briefly, 70 mg of dried TnI (C64A, C133S) was dissolved in 40 ml of a guanidine-containing buffer (0.2 M Tris-HCl (pH 8.0), 4 M guanidine hydrochloride and 0.2 mM dithiothreitol) and allowed to stand at 37°C. To the solution, nitrothiocyanobenzoic add (NTCB) was added in about 10 fold amounts (10 mg) of -SH groups contained in the solution and the mixture was pre-incubated for 20 min. The pH of the solution was increased to 9.0 by addition of aqueous NaOH solution and the reaction was allowed to take place by incubation at 37°C for 6 hours. Obtained reaction products were separated by a gel-filtration chromatography using Sephacryl S-100 column (2.5 x 100 cm, Pharmacia) previously equilibrated with a buffer solution containing 20 mM Tris-HCl (pH 8.0) and 0.3 M NaCl. The fractions containing N-terminus fragment$_{1-47}$ were detected by SDS electrophoresis and further purified by reverse-phase HPLC using an Aquapole Butyl column (10 x 300 mm, Brownlee) and eluting with an acetonitrile concentration gradient of 0 to 100% in the presence of 0.1% trifluoroacetic acid at a flow rate of 3 ml/min. Elution of TnI$_{1-47}$ was detected by a UV absorbance detector at 280 nm absorbance. The obtained fractions were dried on a centrifugal concentrator and stored at -20°C.

### Reference Example 5

### Purification and crystallization of TnC/TnI$_{1-47}$ complex

### (1) Purification of TnC/TnI$_{1-47}$ complex

Dried preparations of TnC and TnI$_{1-47}$ which were separately purified as in Reference Examples 3 and 4, respectively were dissolved in a solution containing 6 M urea, 1 M NaCl, 20 mM Tris-HCl (pH 8.0), 5 mM CaCl$_2$ and 1 mM dithiothreitol in about 30 % by mole excess of TnI$_{1-47}$ over TnC. The solution was dialyzed against an outer solution containing 1 M NaCl, 20 mM Tris-HCl (pH 8.0), 5 mM CaCl$_2$ and 1 mM dithiothreitol and then sequentially dialyzed against outer solutions containing 0.7, 0.5, 0.3 and 0.1M NaCl, respectively. After that, the resulting complex was purified by an anion exchange chromatography as shown below.
After dialysis, the sample was applied onto a Q-sepharose fast-flow column (1.5 x 10 cm, Pharmacia) previously equilibrated with a buffer solution containing 20 mM Tris-HCl (pH 8.0), 0.1 M NaCl, 5 mM CaCl$_2$ and 1 mM dithiothreitol and eluted with a NaCl salt gradient of 0.1 to 0.6 M. The fractions containing the complex were detected by SDS electrophoresis and the obtained fractions were dialyzed against an outer solution containing 10 mM Tris-HCl (pH 8.0) and 5 mM CaCl$_2$. The inner solution of the dialysis was concentrated to a protein concentration of 25-30 mg/ml using Centriprep (Amicon) to give a sample for crystallization.

### (2) Crystallization of TnC/TnI$_{1-47}$ complex

Crystallization was carried out by means of a vapor phase crystallization method, in particular, a hanging drop method using a 24 well dish for tissue culture (McPherson, A., Eur. J. Biochem. 189, 1-23 (1990)). A mixture of 5 μl of crystallization sample described above and 5 μl of a reservoir solution containing 1.5 M sodium citrate, 0.1 M Tris-HCl (pH 8.0) and 15% trehalose as a mother liquor was placed in a high temperature box at 16°C against 1 ml of the reservoir solution and vapor diffusion was carried out. Crystals of 0.6 to 1 mm size grew within 1 to 2 weeks.

### Example 1

### X-ray analysis of TnC/TnI$_{1-47}$ complex

### (1) Preparation and crystallization of the proteins

In order to avoid heterogeneity of the N-terminus, TnC was expressed as a fusion protein using pMal-c2 (New England Bio Labs). The fusion protein was purified and then cleaved by Xa factor. The recombinant TnC molecule used here has an extra Met residue in the N-terminus, which does not exist in the peptide sequence of the rabbit skeletal muscle TnC and hence the Met was excluded from numbering of amino acid residues of TnC. The TnC/TnI$_{1-47}$ complex was prepared and crystallized in the same manner as those described previously (Saijo, Y. et al., Production, crystallization, and preliminary X-ray analysis of rabbit skeletal muscle troponin complex consisting of troponin C and fragment (1-47) of troponin I, Protein Sci. 6, 916-918 (1997)) with minor modifications (Reference Example 5 (2)). The crystals belong to the space group P3$_2$21 with the unit cell dimensions a=b=46.9Å , c=152.3 Å and with a solvent content of 35%.

(2) Data collection

In order to alter the environment of the solution of the crystals, the mother liquor containing crystals was stepwise diluted with the same solution containing no protein (crystallizing solution) and the solution was completely exchanged for the crystallizing solution. The resulting solution containing crystals was sealed in a thin glass tube. The thin tube in ice was transported to Grenoble in France. In Grenoble the thin tube was cut to remove the crystals.

In order to prepare crystals substituted with heavy metals, the crystals were further soaked in a crystallizing solution containing 1 mM PCMBS-Na (sodium p-chloromercuribenzensulfonate) at the room temperature for 8 hours and then the crystals were soaked in the crystallizing solution to remove the excess heavy atom compounds.

Each crystal was taken in a molt quartz thin tube (0.7 mm diameter, Hilgenberg, Germany) immediately before measurement with X-ray. After removal of excess solution, the thin tube was sealed at both ends with wax and put on a mount for crystal freezing (Cryocup, Hampton Research, San Diego, California, USA). The thin tube together with the mount was installed on a goniometer head (Rigaku Denki, Japan) and a cooled air blowing type crystal freezing device (Oxford Cryo Systems, Oxford, UK) was manually slowly moved against the crystal in the quartz thin tube to freeze the crystal. During the freezing, the temperature lowering rate was adjusted to take about 5 seconds to fall from the room temperature to -100°C. The beam line BM14 of European Synchrotron Radiation Facility (ESRF) in Grenoble city was used for measurement of X-ray.

The data of natural TnC/TnI$_{1-47}$ complex and its PCMBS derivative were collected using 3 different wave lengths (which contain one of absorbance ends of the mercury atom) and with the beam line BM14 (ESRF research laboratories, Grenoble, France) and for two frozen single crystals (100°K). Diffraction data were treated using programs DENZO and SCALEPACK (Otwinowski, Z. and Minor, W., in methods in Enzymology (Carter C. W. J.& Sweet, R. M. ed.) 276, 307-326 (Academic Press, San Diego, 1997).

(3) Structure determination

All initial attempts to determine the TnC/TnI$_{1-47}$ structure by molecular replacement by using the structure of uncomplexed TnC as either a whole or its domains failed. The structure was solved using the single isomorphous replacement (SIR) method in combination with the multiple wavelength anomalous dispersion (MAD) technique. The major heavy atom site of the PCMBS derivative was located in both isomorphous and anomalous difference Patterson maps with the RSPS program (Collaborative Computational Project No 4, Acta Crystallogr. D 50, 760-763 (1994)). The heavy atom site was refined using the MLPHARE program (Otwinowski, Z., in Proceedings of the CCP4 Study Weekend, eds. Sawyer N. I. L. & Borley, S., 56-62 (SERC Daresbury, Daresbury, UK) (1993)) modified by D.G.V., and three additional minor heavy atom sites were found in the difference Fourier maps. The overall figure of merit was 0.85 at 2.8 Å resolution.

The SIR/MAD electron density map was then calculated and the phases were improved by solvent flattening and histogram matching using the DM program (Collaborative Computational Project No 4, Acta Crystallogr. D 50, 760-763 (1994)). The rough orientation of the TnC domains in the final electrron density map was determined manually using the O program (Jones, T. A., Zou, J. Y., Cowan, S.E. & Kjeldgaard, M., Improved methods for building protein models in electron density maps and location of errors in these models. Acta Crystallogr. A47, 110-119 (1991)) on the basis of 1TOP registered at Protein Data Bank (PDB). The TnC model was then rebuilt to achieve the best fit to the electron density, and the interdomain linker region and the TnI$_{1-47}$ $\alpha$-helix were modeled with the O program.

(4) Refinement.

The R-factor for initial model was 48%. The model was refined with the program X-PLOR v3.1 Manual (Yale Univ. Press, New Haven, 1992). During the course of the refinement, the fraction of residues in the most favorable region of the Ramachandran plot increased from 80% to 88.3%, as indicated by the program PROCHECK (Laskowski, R.A., MacArthur, M.W., Moss, D.S. and Thornton, J.M., PROCHCK: program to check the stereochemistry of protein structure, J. Appl. Cryst. 26, 283-291 (1993)). No residues were found in the disallowed region of the Ramachandran plot. The average B-factor of the final model is 41 Å$^2$. Almost all of the modeled residues, including their side chains, were well defined by the electron densities in the final (2Fo-Fc) map.

Example 2

Model building of TnI$_{reg}$

The three-dimensional model of the TnC/TnI$_{reg}$ complex was constructed in four steps. Firstly, the "closed" N-domain of TnC in the TnC/TnI$_{reg}$ complex was replaced by a calcium ion bound "open" N-domain (PDB entry 1TNQ) (Gagne, S.M., Tsuda, S., Li, M.X., Smillie, L.B. & Sykes, B.D. , Structures of the troponin C regulatory domains in the apo and calcium-saturated states, Nat. Struct. Biol. 2, 784-789 (1995)). Secondly, the C-domain bound to the TnI$_{1-47}$ α-helix was superimposed on the "open" N-domain, and the resulting orientation of the α-helix was taken as a starting model for TnI$_{reg}$. Thirdly, the TnI$_{1-47}$ sequence was replaced by that of TnI$_{reg}$ in the model. At this stage, the conserved Met121 of TnI$_{reg}$ fit very well into the hydrophobic pocket of the N-domain of TnC. Finally, a two-proline kink (residues 109-110) was introduced in the TnI$_{reg}$ α-helix, on the basis of an NMR structure of the inhibitory peptide (residues 104-115) bound to TnC (Campbell, A.P. & Sykes, B.D., Interaction of Troponin C. Use of the two-dimensional nuclear magnetic resonance transferred nuclear Overhauser effect to determine the structure of the inhibitory troponin I peptide when bound to skeletal troponin C, J. Mol. Biol. 222, 405-421 (1991)) (Table 2).

In addition, the N-terminal end of the α-helix was extended to the TnI residue 96 in order to make it more consistent with the biochemical data. The model was then refined by energy minimization with the X-PLOR program (Brunger, A.), X-PLOR v3.1 Manual (Yale Univ. Press, New Haven, 1992). At the high calcium ion concentration, the distances between Trp 106 (TnI) and Met25, Cys89 (TnC) are 21.6 Å and 20.2 Å, respectively, which are in good agreement with the respective distances of 24.2 Å and 23.2 Å measured in the proposed model (Personal communication, Zhao, X., Kobayashi, T., Malak, H., Gryczynski, L, Lakowicz, J., Wade, R. & Collins, J.H.).

Table 2

| Comparison of the TnC/TnI$_{reg}$ Model with NMR structure of TnI inhibitory peptide (residues 104-116) bound to TnC | | | | |
|---|---|---|---|---|
| Residues model (TnI) | Main chain torsion angles ($\Phi,\Psi,(°)$) | | | |
| | NMR structure | | TnC/TnI$_{reg}$ | |
| | $\Phi$ | $\Psi$ | $\Phi$ | $\Psi$ |
| Lys$^{107}$ | - 70 | - 25 | - 66 | - 25 |
| Asp$^{108}$ | - 59 | - 38 | - 44 | - 34 |
| Pre$^{109}$ | - 80 | 174 | - 58 | 149 |
| Pro$^{110}$ | - 78 | 161 | - 89 | 152 |
| Leu$^{111}$ | - 59 | - 23 | - 56 | - 26 |
| Arg$^{112}$ | - 66 | - 43 | - 55 | - 38 |

Example 3

Use of Troponin C/Troponin I complex

(1) Construction of a screening system for active agents capable of exerting some influence on the binding between calcium ion and Troponin C/Troponin I complex.

A reaction solution (total 3 ml) is prepared by dissolving 13 μM of TnC prepared in the same manner as described in Reference Example 3 and 13 μM of either TnI$_{1-47}$ or TnI prepared in the same manner as described in Reference Example 4 in a buffer solution containing 70 mM KCl, 20 mM MOPS-KOH, pH 6.80, 10 mM MgCl$_2$, 0.13 mM tetramethylmurexide. To the reaction solution, each 3 μl (5 μM as concentration) of 5.0 mM CaCl$_2$ solution is added repeatedly and after every addition the calcium ion-concentration of the solution is measured as a difference in absorbance at 554 nm and 507 nm. The calcium ion-concentrations thus obtained are plotted as the horizontal axis against color developments of tetramethylmurexide as the vertical axis, yielding a standard curve (usually a sigmoidal curve) for the binding between calcium ions and the TnC/TnI$_{1-47}$ or TnC/TnI complex.

Next, to the reaction solution described above, 1 nM to 1 mM of a compound to be tested is added and the mixture is incubated at 36°C for an appropriate period of time. After that, the calcium ion-concentrations of the reaction solution are determined in the same manner as described above and the calcium ion-concentrations are plotted as the horizontal axis against the color developments of tetramethylmurexide as the vertical axis.

The compounds are selected which show a significant change in the curve described above (namely, in the bound calcium ion concentrations) before and after addition of the compound to be tested.

<u>(2) Construction of a screening system for active agents capable of exerting some influence on the association between TnC and TnI</u>

Troponin C is prepared in the same manner described in Reference Example 3, using a mutant in which tryptophan is substituted for the 23 and 102 position residues according to a method of Pearlstone et al. (Pearlstone, J. R, Sykes, B. D. & Smillie, L. B., Biochemistry $\underline{36}$, 7601-7606 (1997)). $TnI_{1-47}$ and TnI are dissolved in a buffer solution containing 100 mM KCl, 50 mM POMS-KOH (pH 7.1), 1 mM EDTA and 1 mM DTT.

A solution containing only TnC (at a concentration of 0.75 $\mu$M) and a solution containing both TnC and $TnI_{1-47}$ or TnI (at a concentration of several folds excess over TnC) are irradiated with a UV-light at 282 nm wavelength and the fluorescence spectra of the solution are measured. The measurement is conducted under the conditions of high concentration of calcium ion (in the presence of 2 mM $CaCl_2$) and low concentration (in the absence of 2 mM $CaCl_2$).

Next, a compound to be tested is added at from 1 nM to 1 mM. After incubation at 36°C for an appropriate period of time, the fluorescence spectra of the reaction solutions are measured again.

The compounds are chosen which cause a significant change in the wave shapes or intensities of the fluorescence spectra before and after addition of the compound to be tested.

INDUSTRIAL APPLICABILITY

According to the present invention, the atomic coordinates, namely, stereo structure of the $TnC/TnI_{1-47}$ complex which has not been elucidated so far has been revealed, thereby the mechanism of the calcium switch of the muscle contraction also has been elucidated. Further, on the basis of these findings, the present invention provides for a screening system of active agents capable of exerting some influences on the muscle contraction, more particularly a screening system for active agents capable of exerting some influences on the binding of calcium ion to the complex described above or on the formation of the complex described above, using the complex consisting of troponin C and troponin $I_{1-47}$, the complex consisting of troponin C and the peptide containing troponin $I_{1-47}$ fragment and which is capable of binding to troponin C or the complex consisting of troponin C and troponinI.

## SEQUENCE LISTING

<1 1 0 > MATSUSHITA ELECTRIC INDUSTRIAL CO. LTD.

<1 2 0 > Troponin CI complex.

<1 3 0 > MT-9-001-F

<1 5 0 > JP PH9-368833

<1 5 1 > 1997-12-27

<1 6 0 > 4

<2 1 0 > 1

<2 1 1 > 2 3

<2 1 2 > DNA

<2 1 3 > Artificial Sequence

<4 0 0 > 1

ggcgatcgct gagttcaagg ccg                                                23

<2 1 0 > 2

<2 1 1 > 2 8

<2 1 2 > DNA

<2 1 3 > Artificial Sequence

<4 0 0 > 2

ccggatcccc agccttactg gcacgccc                                           28

<2 1 0 > 3

<2 1 1 > 5 0

<2 1 2 > DNA

<2 1 3 > Artificial Sequence

<4 0 0 > 3

atgaccgacc agcaggcgga agcgcgtagc tacctgagcg aagaaatgat                   50

<2 1 0 > 4

<2 1 1 > 4 8

```
<212>DNA

<213>Artificial Sequence

<400>4

catttcttcg ctcaggtagc tacgcgcttc cgcctgctgg tcggtcat          48
```

**Claims**

1. A complex consisting of troponin C (TnC) and the troponin $I_{1-47}$ (TnI$_{1-47}$) fragment peptide wherein said complex has atomic coordinates as set forth in Fig.7 to Fig.47.

2. A complex consisting of TnC and a peptide containing the Tn $I_{1-47}$ fragment and which is capable of binding to TnC wherein said complex has atomic coordinates as set forth in Fig.7 to Fig.47.

3. The complex according to claim 2 wherein said peptide is troponin I (TnI).

4. The complex according to any one of claims 1 to 3 wherein said peptide is obtained by deletion, addition, insertion or substitution of one or more amino acid residues in the TnI$_{1-47}$ fragment.

5. The crystals of the complex according to claim 1.

6. A screening system for an active agent using the complex according to any one of claims 1 to 4 wherein said active agent is capable of exerting some influence on the binding of calcium ion to said complex.

7. The screening system according to claim 6 wherein said active agent facilitates the binding of calcium ion to the TnC/TnI$_{1-47}$ complex.

8. The screening system according to claim 6 wherein said active agent inhibits the binding of calcium ion to the TnC/TnI$_{1-47}$ complex.

9. The screening system according to claim 6 wherein said active agent facilitates the binding of calcium ion to the complex consisting of TnC and a peptide containing the TnI$_{1-47}$ fragment and which is capable of binding to TnC.

10. The screening system according to claim 6 wherein said active agent inhibits the binding of calcium ion to the complex consisting of TnC and a peptide containing the TnI$_{1-47}$ fragment and which is capable of binding to TnC.

11. The screening system according to claim 6 wherein said active agent facilitates the binding of calcium ion to the complex consisting of TnC and TnI.

12. The screening system according to claim 6 wherein said active agent inhibits the binding of calcium ion to the complex consisting of TnC and TnI.

13. The screening system according to any one of claims 6 to 12 wherein a method measuring the binding of calcium ion to the complex is the indicator method using methylmurexide.

14. A screening system for an active agent using the complex according to any one of claims 1 to 4 wherein said active agent is capable of exerting some influence on the formation of said complex.

15. The screening system according to claim 14 wherein said active agent facilitates the formation of the TnC/TnI$_{1-47}$ complex.

16. The screening system according to claim 14 wherein said active agent inhibits the formation of the TnC/TnI$_{1-47}$

complex.

17. The screening system according to claim 14 wherein said active agent facilitates the formation of the complex consisting of TnC and a peptide containing the $TnI_{1-47}$ fragment and which is capable of binding to TnC.

18. The screening system according to claim 14 wherein said active agent inhibits the formation of the complex consisting of TnC and a peptide containing the $TnI_{1-47}$ fragment and which is capable of binding to TnC.

19. The screening system according to claim 14 wherein said active agent facilitates the formation of the TnC/TnI complex.

20. The screening system according to claim 14 wherein said active agent inhibits the formation of the TnC/TnI complex.

21. The screening system according to any one of claims 14 to 20 wherein a method of measurement of the strength of the binding between TnC and $TnI_{1-47}$, a peptide containing the $TnI_{1-47}$ fragment and which is capable of binding to TnC or TnI is one utilizing the change of fluorescence spectra due to a fluorescent pigment introduced into TnC.

## Fig. 1

Fig. 2

F i g. 3

F i g.  4

| | Sequence Identity (%) Residues 117-127 | | | |
| | Total | Total | Hydrophobic Residues | |
|---|---|---|---|---|
| Rabbit, fast skeletal muscle | - | - | - | 117 SADAMLKALLG 127 |
| Amphiphilic portion of TnI₁₋₄₇ | - | - | - | 17 LSKVMLQ IAA T 27 |
| Rabbit, slow skeletal muscle | 62 | 91 | 100 | 116 SADAMLRALLG 126 |
| Human, slow skeletal muscle | 61 | 91 | 100 | 118 SADAMLRALLG 128 |
| Rat, slow skeletal muscle | 59 | 91 | 100 | 118 SADAMLRALLG 128 |
| Chicken, cardiac muscle | 55 | 82 | 88 | 102 SADAMMAALLG 112 |
| Japanese quail, cardiac muscle | 53 | 82 | 88 | 141 SADAMMAALLG 151 |
| African clawed frog, cardiac muscle | 64 | 82 | 88 | 174 SADAMMMALLG 184 |
| Rabbit, cardiac muscle | 60 | 82 | 88 | 146 SADAMMQALLG 156 |
| Human, cardiac muscle | 59 | 82 | 88 | 149 SADAMMQALLG 159 |
| Mouse, cardiac muscle | 57 | 82 | 88 | 150 SADAMMQALLG 160 |
| Rat, cardiac muscle | 57 | 82 | 88 | 150 SADAMMQALLG 160 |
| Bovine, cardiac muscle | 60 | 82 | 88 | 151 SADAMMQALLG 161 |

EP 1 043 334 A1

Fig. 5

Fig. 6

F i g. 7

```
REMARK Written by O version 6.1.1
REMARK Tue Dec 23 15:14:17 1997
CRYST1    1.000     1.000     1.000  90.00   90.00   90.00
ORIGX1         1.000000  0.000000  0.000000        0.00000
ORIGX2         0.000000  1.000000  0.000000        0.00000
ORIGX3         0.000000  0.000000  1.000000        0.00000
SCALE1         1.000000 -0.000026 -0.000026        0.00000
SCALE2         0.000000  1.000000 -0.000026        0.00000
SCALE3         0.000000  0.000000  1.000000        0.00000
ATOM     1  CB  ASP   2       30.837  -2.922   7.216  1.00 69.46      6
ATOM     2  CG  ASP   2       31.985  -3.579   7.951  1.00 78.50      6
ATOM     3  OD1 ASP   2       32.541  -2.942   8.878  1.00 79.39      8
ATOM     4  OD2 ASP   2       32.322  -4.737   7.603  1.00 79.21      8
ATOM     5  C   ASP   2       28.783  -2.147   8.365  1.00 59.18      6
ATOM     6  O   ASP   2       28.070  -2.270   9.361  1.00 54.82      8
ATOM     7  N   ASP   2       28.663  -3.980   6.677  1.00 53.49      7
ATOM     8  CA  ASP   2       29.478  -3.353   7.761  1.00 61.82      6
ATOM     9  N   GLN   3       29.002  -0.982   7.755  1.00 61.18      7
ATOM    10  CA  GLN   3       28.381   0.263   8.200  1.00 58.72      6
ATOM    11  CB  GLN   3       29.062   1.467   7.544  1.00 60.55      6
ATOM    12  CG  GLN   3       29.721   1.167   6.205  1.00 69.54      6
ATOM    13  CD  GLN   3       28.713   0.978   5.074  1.00 75.82      6
ATOM    14  OE1 GLN   3       28.455   1.898   4.392  1.00 79.91      8
ATOM    15  NE2 GLN   3       28.143  -0.223   4.981  1.00 74.66      7
ATOM    16  C   GLN   3       26.908   0.237   7.800  1.00 59.62      6
ATOM    17  O   GLN   3       26.287   1.274   7.571  1.00 58.82      8
ATOM    18  N   GLN   4       26.360  -0.969   7.715  1.00 60.33      7
ATOM    19  CA  GLN   4       24.974  -1.156   7.334  1.00 62.73      6
ATOM    20  CB  GLN   4       24.796  -2.513   6.635  1.00 62.38      6
ATOM    21  CG  GLN   4       25.986  -2.956   5.771  1.00 72.10      6
ATOM    22  CD  GLN   4       25.744  -2.787   4.270  1.00 71.80      8
ATOM    23  OE1 GLN   4       26.086  -1.755   3.683  1.00 74.42      8
ATOM    24  NE2 GLN   4       25.157  -3.804   3.645  1.00 64.88      7
ATOM    25  C   GLN   4       24.064  -1.072   8.551  1.00 61.58      6
ATOM    26  O   GLN   4       23.191  -0.207   8.614  1.00 61.80      8
ATOM    27  N   ALA   5       24.288  -1.964   9.517  1.00 62.97      7
ATOM    28  CA  ALA   5       23.488  -2.028  10.747  1.00 58.31      6
ATOM    29  CB  ALA   5       24.202  -2.870  11.782  1.00 58.45      6
ATOM    30  C   ALA   5       23.182  -0.648  11.322  1.00 54.06      6
ATOM    31  O   ALA   5       22.177  -0.450  12.006  1.00 48.21      8
```

# Fig. 8

| | | | | | | | | |
|---|---|---|---|---|---|---|---|---|
| ATOM | 32 | N | GLU | 6 | 24.065 | 0.301 | 11.046 | 1.00 53.88 | 7 |
| ATOM | 33 | CA | GLU | 6 | 23.865 | 1.655 | 11.512 | 1.00 55.58 | 6 |
| ATOM | 34 | CB | GLU | 6 | 25.069 | 2.623 | 11.149 | 1.00 58.94 | 6 |
| ATOM | 35 | CG | GLU | 6 | 24.996 | 3.944 | 11.697 | 1.00 65.76 | 6 |
| ATOM | 36 | CD | GLU | 6 | 25.790 | 4.129 | 12.988 | 1.00 72.64 | 6 |
| ATOM | 37 | OE1 | GLU | 6 | 25.399 | 3.546 | 14.029 | 1.00 74.18 | 8 |
| ATOM | 38 | OE2 | GLU | 6 | 26.808 | 4.860 | 12.957 | 1.00 72.38 | 8 |
| ATOM | 39 | C | GLU | 6 | 22.624 | 2.148 | 10.790 | 1.00 57.77 | 6 |
| ATOM | 40 | O | GLU | 6 | 21.631 | 2.527 | 11.420 | 1.00 50.65 | 8 |
| ATOM | 41 | N | ALA | 7 | 22.691 | 2.116 | 9.458 | 1.00 58.68 | 7 |
| ATOM | 42 | CA | ALA | 7 | 21.587 | 2.541 | 8.600 | 1.00 59.35 | 6 |
| ATOM | 43 | CB | ALA | 7 | 21.915 | 2.234 | 7.151 | 1.00 55.15 | 6 |
| ATOM | 44 | C | ALA | 7 | 20.298 | 1.827 | 9.005 | 1.00 62.98 | 6 |
| ATOM | 45 | O | ALA | 7 | 19.209 | 2.401 | 8.943 | 1.00 63.84 | 8 |
| ATOM | 46 | N | ARG | 8 | 20.432 | 0.567 | 9.412 | 1.00 62.69 | 7 |
| ATOM | 47 | CA | ARG | 8 | 19.290 | -0.226 | 9.840 | 1.00 57.09 | 6 |
| ATOM | 48 | CB | ARG | 8 | 19.705 | -1.675 | 10.086 | 1.00 55.59 | 6 |
| ATOM | 49 | CG | ARG | 8 | 19.698 | -2.541 | 8.844 | 1.00 47.65 | 6 |
| ATOM | 50 | CD | ARG | 8 | 18.720 | -3.689 | 8.980 | 1.00 39.81 | 6 |
| ATOM | 51 | NE | ARG | 8 | 18.702 | -4.508 | 7.774 | 1.00 45.96 | 7 |
| ATOM | 52 | CZ | ARG | 8 | 17.751 | -5.389 | 7.480 | 1.00 44.00 | 6 |
| ATOM | 53 | NH1 | ARG | 8 | 16.732 | -5.570 | 8.304 | 1.00 40.59 | 7 |
| ATOM | 54 | NH2 | ARG | 8 | 17.816 | -6.087 | 6.352 | 1.00 44.78 | 7 |
| ATOM | 55 | C | ARG | 8 | 18.761 | 0.367 | 11.130 | 1.00 58.08 | 6 |
| ATOM | 56 | O | ARG | 8 | 17.554 | 0.584 | 11.283 | 1.00 60.55 | 8 |
| ATOM | 57 | N | SER | 9 | 19.676 | 0.639 | 12.055 | 1.00 57.22 | 7 |
| ATOM | 58 | CA | SER | 9 | 19.306 | 1.205 | 13.344 | 1.00 56.39 | 6 |
| ATOM | 59 | CB | SER | 9 | 20.556 | 1.402 | 14.204 | 1.00 58.61 | 6 |
| ATOM | 60 | OG | SER | 9 | 21.055 | 2.731 | 14.093 | 1.00 64.72 | 8 |
| ATOM | 61 | C | SER | 9 | 18.597 | 2.552 | 13.178 | 1.00 56.30 | 6 |
| ATOM | 62 | O | SER | 9 | 17.568 | 2.808 | 13.822 | 1.00 51.27 | 8 |
| ATOM | 63 | N | TYR | 10 | 19.167 | 3.399 | 12.315 | 1.00 54.43 | 7 |
| ATOM | 64 | CA | TYR | 10 | 18.651 | 4.738 | 12.041 | 1.00 50.57 | 6 |
| ATOM | 65 | CB | TYR | 10 | 19.585 | 5.444 | 11.053 | 1.00 51.45 | 6 |
| ATOM | 66 | CG | TYR | 10 | 19.325 | 6.930 | 10.835 | 1.00 62.91 | 6 |
| ATOM | 67 | CD1 | TYR | 10 | 20.150 | 7.901 | 11.416 | 1.00 65.19 | 6 |
| ATOM | 68 | CE1 | TYR | 10 | 19.951 | 9.260 | 11.157 | 1.00 65.65 | 6 |
| ATOM | 69 | CD2 | TYR | 10 | 18.294 | 7.363 | 9.996 | 1.00 64.00 | 6 |
| ATOM | 70 | CE2 | TYR | 10 | 18.091 | 8.712 | 9.735 | 1.00 61.68 | 6 |
| ATOM | 71 | CZ | TYR | 10 | 18.918 | 9.654 | 10.315 | 1.00 66.93 | 6 |

F i g. 9

| ATOM | 72 | OH | TYR | 10 | 18. 704 | 10. 989 | 10. 044 | 1. 00 | 70. 38 | 8 |
|---|---|---|---|---|---|---|---|---|---|---|
| ATOM | 73 | C | TYR | 10 | 17. 216 | 4. 730 | 11. 519 | 1. 00 | 50. 92 | 6 |
| ATOM | 74 | O | TYR | 10 | 16. 602 | 5. 736 | 11. 630 | 1. 00 | 45. 01 | 8 |
| ATOM | 75 | N | LEU | 11 | 16. 789 | 3. 599 | 10. 962 | 1. 00 | 47. 90 | 7 |
| ATOM | 76 | CA | LEU | 11 | 15. 429 | 3. 477 | 10. 441 | 1. 00 | 49. 44 | 6 |
| ATOM | 77 | CB | LEU | 11 | 15. 419 | 2. 782 | 9. 080 | 1. 00 | 49. 23 | 6 |
| ATOM | 78 | CG | LEU | 11 | 16. 330 | 3. 405 | 8. 026 | 1. 00 | 46. 86 | 6 |
| ATOM | 79 | CD1 | LEU | 11 | 16. 622 | 2. 363 | 6. 955 | 1. 00 | 51. 99 | 6 |
| ATOM | 80 | CD2 | LEU | 11 | 15. 677 | 4. 650 | 7. 436 | 1. 00 | 47. 83 | 6 |
| ATOM | 81 | C | LEU | 11 | 14. 572 | 2. 680 | 11. 403 | 1. 00 | 45. 64 | 6 |
| ATOM | 82 | O | LEU | 11 | 15. 057 | 1. 750 | 12. 039 | 1. 00 | 50. 42 | 8 |
| ATOM | 83 | N | SER | 12 | 13. 302 | 3. 061 | 11. 501 | 1. 00 | 42. 17 | 7 |
| ATOM | 84 | CA | SER | 12 | 12. 347 | 2. 411 | 12. 386 | 1. 00 | 42. 47 | 6 |
| ATOM | 85 | CB | SER | 12 | 11. 061 | 3. 241 | 12. 480 | 1. 00 | 43. 33 | 6 |
| ATOM | 86 | OG | SER | 12 | 10. 234 | 3. 083 | 11. 331 | 1. 00 | 42. 59 | 8 |
| ATOM | 87 | C | SER | 12 | 12. 024 | 1. 018 | 11. 881 | 1. 00 | 42. 70 | 6 |
| ATOM | 88 | O | SER | 12 | 12. 531 | 0. 604 | 10. 849 | 1. 00 | 45. 74 | 8 |
| ATOM | 89 | N | GLU | 13 | 11. 168 | 0. 305 | 12. 606 | 1. 00 | 43. 76 | 7 |
| ATOM | 90 | CA | GLU | 13 | 10. 795 | -1. 048 | 12. 230 | 1. 00 | 46. 48 | 6 |
| ATOM | 91 | CB | GLU | 13 | 10. 161 | -1. 760 | 13. 421 | 1. 00 | 50. 74 | 6 |
| ATOM | 92 | CG | GLU | 13 | 11. 150 | -2. 532 | 14. 267 | 1. 00 | 60. 34 | 6 |
| ATOM | 93 | CD | GLU | 13 | 10. 846 | -4. 019. | 14. 323 | 1. 00 | 67. 08 | 6 |
| ATOM | 94 | OE1 | GLU | 13 | 9. 651 | -4. 381 | 14. 362 | 1. 00 | 63. 92 | 8 |
| ATOM | 95 | OE2 | GLU | 13 | 11. 804 | -4. 828 | 14. 331 | 1. 00 | 74. 08 | 8 |
| ATOM | 96 | C | GLU | 13 | 9. 811 | -1. 045 | 11. 076 | 1. 00 | 48. 45 | 6 |
| ATOM | 97 | O | GLU | 13 | 9. 913 | -1. 848 | 10. 144 | 1. 00 | 50. 07 | 8 |
| ATOM | 98 | N | GLU | 14 | 8. 852 | -0. 133 | 11. 154 | 1. 00 | 47. 38 | 7 |
| ATOM | 99 | CA | GLU | 14 | 7. 812 | -0. 007 | 10. 147 | 1. 00 | 41. 86 | 6 |
| ATOM | 100 | CB | GLU | 14 | 6. 672 | 0. 850 | 10. 700 | 1. 00 | 43. 25 | 6 |
| ATOM | 101 | CG | GLU | 14 | 6. 964 | 1. 465 | 12. 077 | 1. 00 | 50. 19 | 6 |
| ATOM | 102 | CD | GLU | 14 | 7. 149 | 0. 417 | 13. 173 | 1. 00 | 42. 62 | 6 |
| ATOM | 103 | OE1 | GLU | 14 | 6. 235 | -0. 413 | 13. 344 | 1. 00 | 39. 86 | 8 |
| ATOM | 104 | OE2 | GLU | 14 | 8. 203 | 0. 420 | 13. 851 | 1. 00 | 33. 80 | 8 |
| ATOM | 105 | C | GLU | 14 | 8. 346 | 0. 582 | 8. 834 | 1. 00 | 41. 53 | 6 |
| ATOM | 106 | O | GLU | 14 | 7. 751 | 0. 383 | 7. 771 | 1. 00 | 32. 10 | 8 |
| ATOM | 107 | N | MET | 15 | 9. 475 | 1. 288 | 8. 915 | 1. 00 | 39. 12 | 7 |
| ATOM | 108 | CA | MET | 15 | 10. 097 | 1. 895 | 7. 738 | 1. 00 | 35. 21 | 6 |
| ATOM | 109 | CB | MET | 15 | 11. 117 | 2. 964 | 8. 151 | 1. 00 | 33. 47 | 6 |
| ATOM | 110 | CG | MET | 15 | 10. 723 | 4. 394 | 7. 755 | 1. 00 | 41. 44 | 6 |
| ATOM | 111 | SD | MET | 15 | 11. 902 | 5. 721 | 8. 260 | 1. 00 | 51. 80 | 16 |

# Fig. 10

| | | | | | | | | | | | |
|---|---|---|---|---|---|---|---|---|---|---|---|
| ATOM | 112 | CE | MET | 15 | 10.846 | 7.174 | 8.186 | 1.00 | 35.09 | 6 |
| ATOM | 113 | C | MET | 15 | 10.782 | 0.788 | 6.958 | 1.00 | 34.65 | 6 |
| ATOM | 114 | O | MET | 15 | 10.488 | 0.572 | 5.793 | 1.00 | 34.92 | 8 |
| ATOM | 115 | N | ILE | 16 | 11.699 | 0.083 | 7.605 | 1.00 | 35.28 | 7 |
| ATOM | 116 | CA | ILE | 16 | 12.377 | -1.026 | 6.960 | 1.00 | 37.83 | 6 |
| ATOM | 117 | CB | ILE | 16 | 13.256 | -1.776 | 7.939 | 1.00 | 37.57 | 6 |
| ATOM | 118 | CG2 | ILE | 16 | 13.825 | -3.024 | 7.270 | 1.00 | 36.88 | 6 |
| ATOM | 119 | CG1 | ILE | 16 | 14.347 | -0.844 | 8.456 | 1.00 | 40.83 | 6 |
| ATOM | 120 | CD1 | ILE | 16 | 15.100 | -1.400 | 9.652 | 1.00 | 40.77 | 6 |
| ATOM | 121 | C | ILE | 16 | 11.354 | -2.025 | 6.429 | 1.00 | 42.31 | 6 |
| ATOM | 122 | O | ILE | 16 | 11.623 | -2.749 | 5.464 | 1.00 | 52.33 | 8 |
| ATOM | 123 | N | ALA | 17 | 10.185 | -2.072 | 7.065 | 1.00 | 38.02 | 7 |
| ATOM | 124 | CA | ALA | 17 | 9.139 | -2.997 | 6.649 | 1.00 | 38.76 | 6 |
| ATOM | 125 | CB | ALA | 17 | 8.099 | -3.132 | 7.735 | 1.00 | 34.84 | 6 |
| ATOM | 126 | C | ALA | 17 | 8.488 | -2.514 | 5.369 | 1.00 | 38.13 | 6 |
| ATOM | 127 | O | ALA | 17 | 8.098 | -3.314 | 4.517 | 1.00 | 47.45 | 8 |
| ATOM | 128 | N | GLU | 18 | 8.374 | -1.199 | 5.229 | 1.00 | 35.99 | 7 |
| ATOM | 129 | CA | GLU | 18 | 7.762 | -0.622 | 4.044 | 1.00 | 34.78 | 6 |
| ATOM | 130 | CB | GLU | 18 | 7.236 | 0.787 | 4.344 | 1.00 | 35.94 | 6 |
| ATOM | 131 | CG | GLU | 18 | 6.873 | 1.600 | 3.096 | 1.00 | 41.13 | 6 |
| ATOM | 132 | CD | GLU | 18 | 6.767 | 3.106 | 3.362 | 1.00 | 42.01 | 6 |
| ATOM | 133 | OE1 | GLU | 18 | 7.233 | 3.565 | 4.429 | 1.00 | 32.29 | 8 |
| ATOM | 134 | OE2 | GLU | 18 | 6.210 | 3.831 | 2.499 | 1.00 | 46.49 | 8 |
| ATOM | 135. | C | GLU | 18 | 8.754 | -0.564 | 2.896 | 1.00 | 36.23 | 6 |
| ATOM | 136 | O | GLU | 18 | 8.389 | -0.824 | 1.749 | 1.00 | 39.81 | 8 |
| ATOM | 137 | N | PHE | 19 | 10.005 | -0.223 | 3.206 | 1.00 | 33.54 | 7 |
| ATOM | 138 | CA | PHE | 19 | 11.050 | -0.114 | 2.196 | 1.00 | 28.74 | 6 |
| ATOM | 139 | CB | PHE | 19 | 12.393 | 0.192 | 2.845 | 1.00 | 30.11 | 6 |
| ATOM | 140 | CG | PHE | 19 | 12.576 | 1.647 | 3.224 | 1.00 | 28.94 | 6 |
| ATOM | 141 | CD1 | PHE | 19 | 11.477 | 2.501 | 3.376 | 1.00 | 31.28 | 6 |
| ATOM | 142 | CD2 | PHE | 19 | 13.855 | 2.150 | 3.479 | 1.00 | 34.39 | 6 |
| ATOM | 143 | CE1 | PHE | 19 | 11.647 | 3.836 | 3.781 | 1.00 | 16.47 | 6 |
| ATOM | 144 | CE2 | PHE | 19 | 14.036 | 3.480 | 3.885 | 1.00 | 26.12 | 6 |
| ATOM | 145 | CZ | PHE | 19 | 12.930 | 4.322 | 4.036 | 1.00 | 26.72 | 6 |
| ATOM | 146 | C | PHE | 19 | 11.128 | -1.426 | 1.471 | 1.00 | 31.30 | 6 |
| ATOM | 147 | O | PHE | 19 | 11.102 | -1.471 | 0.251 | 1.00 | 29.16 | 8 |
| ATOM | 148 | N | LYS | 20 | 11.196 | -2.503 | 2.239 | 1.00 | 35.58 | 7 |
| ATOM | 149 | CA | LYS | 20 | 11.261 | -3.835 | 1.665 | 1.00 | 38.90 | 6 |
| ATOM | 150 | CB | LYS | 20 | 11.051 | -4.888 | 2.746 | 1.00 | 45.25 | 6 |
| ATOM | 151 | CG | LYS | 20 | 11.300 | -6.302 | 2.282 | 1.00 | 33.64 | 6 |

EP 1 043 334 A1

Fig. 11

| ATOM | 152 | CD | LYS | 20 | 12.270 | -6.975 | 3.213 | 1.00 | 32.87 | 6 |
| ATOM | 153 | CE | LYS | 20 | 13.436 | -7.584 | 2.446 | 1.00 | 41.96 | 6 |
| ATOM | 154 | NZ | LYS | 20 | 13.025 | -8.665 | 1.490 | 1.00 | 36.16 | 7 |
| ATOM | 155 | C | LYS | 20 | 10.160 | -3.966 | 0.633 | 1.00 | 39.93 | 6 |
| ATOM | 156 | O | LYS | 20 | 10.431 | -4.196 | -0.544 | 1.00 | 40.01 | 8 |
| ATOM | 157 | N | ALA | 21 | 8.920 | -3.813 | 1.099 | 1.00 | 38.64 | 7 |
| ATOM | 158 | CA | ALA | 21 | 7.723 | -3.905 | 0.264 | 1.00 | 41.19 | 6 |
| ATOM | 159 | CB | ALA | 21 | 6.562 | -3.162 | 0.936 | 1.00 | 37.08 | 6 |
| ATOM | 160 | C | ALA | 21 | 7.924 | -3.384 | -1.161 | 1.00 | 37.94 | 6 |
| ATOM | 161 | O | ALA | 21 | 7.655 | -4.101 | -2.127 | 1.00 | 42.78 | 8 |
| ATOM | 162 | N | ALA | 22 | 8.399 | -2.142 | -1.279 | 1.00 | 43.14 | 7 |
| ATOM | 163 | CA | ALA | 22 | 8.651 | -1.497 | -2.574 | 1.00 | 37.59 | 6 |
| ATOM | 164 | CB | ALA | 22 | 9.016 | -0.029 | -2.371 | 1.00 | 28.15 | 6 |
| ATOM | 165 | C | ALA | 22 | 9.789 | -2.213 | -3.293 | 1.00 | 41.82 | 6 |
| ATOM | 166 | O | ALA | 22 | 9.690 | -2.517 | -4.483 | 1.00 | 38.90 | 8 |
| ATOM | 167 | N | PHE | 23 | 10.871 | -2.460 | -2.553 | 1.00 | 43.18 | 7 |
| ATOM | 168 | CA | PHE | 23 | 12.051 | -3.150 | -3.069 | 1.00 | 44.02 | 6 |
| ATOM | 169 | CB | PHE | 23 | 13.041 | -3.402 | -1.933 | 1.00 | 44.09 | 6 |
| ATOM | 170 | CG | PHE | 23 | 14.444 | -3.686 | -2.393 | 1.00 | 41.16 | 6 |
| ATOM | 171 | CD1 | PHE | 23 | 14.821 | -4.970 | -2.787 | 1.00 | 45.18 | 6 |
| ATOM | 172 | CD2 | PHE | 23 | 15.397 | -2.674 | -2.413 | 1.00 | 39.38 | 6 |
| ATOM | 173 | CE1 | PHE | 23 | 16.138 | -5.243 | -3.194 | 1.00 | 41.35 | 6 |
| ATOM | 174 | CE2 | PHE | 23 | 16.710 | -2.930 | -2.814 | 1.00 | 45.34 | 6 |
| ATOM | 175 | CZ | PHE | 23 | 17.083 | -4.220 | -3.206 | 1.00 | 44.54 | 6 |
| ATOM | 176 | C | PHE | 23 | 11.614 | -4.479 | -3.665 | 1.00 | 44.22 | 6 |
| ATOM | 177 | O | PHE | 23 | 11.714 | -4.693 | -4.876 | 1.00 | 48.50 | 8 |
| ATOM | 178 | N | ASP | 24 | 11.136 | -5.368 | -2.800 | 1.00 | 44.16 | 7 |
| ATOM | 179 | CA | ASP | 24 | 10.659 | -6.674 | -3.220 | 1.00 | 44.12 | 6 |
| ATOM | 180 | CB | ASP | 24 | 9.846 | -7.311 | -2.099 | 1.00 | 29.91 | 6 |
| ATOM | 181 | CG | ASP | 24 | 10.708 | -8.056 | -1.120 | 1.00 | 44.27 | 6 |
| ATOM | 182 | OD1 | ASP | 24 | 11.924 | -7.769 | -1.087 | 1.00 | 43.79 | 8 |
| ATOM | 183 | OD2 | ASP | 24 | 10.176 | -8.925 | -0.389 | 1.00 | 53.01 | 8 |
| ATOM | 184 | C | ASP | 24 | 9.788 | -6.540 | -4.459 | 1.00 | 47.50 | 6 |
| ATOM | 185 | O | ASP | 24 | 9.830 | -7.381 | -5.356 | 1.00 | 48.23 | 8 |
| ATOM | 186 | N | MET | 25 | 8.993 | -5.475 | -4.486 | 1.00 | 51.96 | 7 |
| ATOM | 187 | CA | MET | 25 | 8.086 | -5.194 | -5.594 | 1.00 | 52.29 | 6 |
| ATOM | 188 | CB | MET | 25 | 7.444 | -3.816 | -5.405 | 1.00 | 54.25 | 6 |
| ATOM | 189 | CG | MET | 25 | 5.922 | -3.798 | -5.441 | 1.00 | 51.60 | 6 |
| ATOM | 190 | SD | MET | 25 | 5.282 | -2.203 | -6.044 | 1.00 | 50.72 | 16 |
| ATOM | 191 | CE | MET | 25 | 3.882 | -2.776 | -7.035 | 1.00 | 45.37 | 6 |

30

Fig. 1 2

| | | | | | | | | | | |
|---|---|---|---|---|---|---|---|---|---|---|
| ATOM | 192 | C | MET | 25 | 8.786 | -5.243 | -6.951 | 1.00 | 51.45 | 6 |
| ATOM | 193 | O | MET | 25 | 8.261 | -5.824 | -7.905 | 1.00 | 55.14 | 8 |
| ATOM | 194 | N | PHE | 26 | 9.971 | -4.648 | -7.035 | 1.00 | 47.40 | 7 |
| ATOM | 195 | CA | PHE | 26 | 10.715 | -4.624 | -8.288 | 1.00 | 45.85 | 6 |
| ATOM | 196 | CB | PHE | 26 | 11.301 | -3.236 | -8.501 | 1.00 | 34.86 | 6 |
| ATOM | 197 | CG | PHE | 26 | 10.267 | -2.170 | -8.449 | 1.00 | 38.32 | 6 |
| ATOM | 198 | CD1 | PHE | 26 | 9.648 | -1.858 | -7.246 | 1.00 | 35.43 | 6 |
| ATOM | 199 | CD2 | PHE | 26 | 9.843 | -1.538 | -9.604 | 1.00 | 33.77 | 6 |
| ATOM | 200 | CE1 | PHE | 26 | 8.612 | -0.949 | -7.193 | 1.00 | 44.50 | 6 |
| ATOM | 201 | CE2 | PHE | 26 | 8.803 | -0.620 | -9.569 | 1.00 | 46.36 | 6 |
| ATOM | 202 | CZ | PHE | 26 | 8.185 | -0.322 | -8.356 | 1.00 | 50.95 | 6 |
| ATOM | 203 | C | PHE | 26 | 11.790 | -5.681 | -8.334 | 1.00 | 49.41 | 6 |
| ATOM | 204 | O | PHE | 26 | 12.792 | -5.540 | -9.027 | 1.00 | 52.49 | 8 |
| ATOM | 205 | N | ASP | 27 | 11.560 | -6.752 | -7.590 | 1.00 | 53.44 | 7 |
| ATOM | 206 | CA | ASP | 27 | 12.490 | -7.867 | -7.535 | 1.00 | 57.82 | 6 |
| ATOM | 207 | CB | ASP | 27 | 12.890 | -8.153 | -6.086 | 1.00 | 58.00 | 6 |
| ATOM | 208 | CG | ASP | 27 | 13.866 | -9.308 | -5.969 | 1.00 | 62.59 | 6 |
| ATOM | 209 | OD1 | ASP | 27 | 14.262 | -9.853 | -7.017 | 1.00 | 57.79 | 8 |
| ATOM | 210 | OD2 | ASP | 27 | 14.235 | -9.669 | -4.830 | 1.00 | 62.54 | 8 |
| ATOM | 211 | C | ASP | 27 | 11.804 | -9.094 | -8.129 | 1.00 | 60.01 | 6 |
| ATOM | 212 | O | ASP | 27 | 11.133 | -9.845 | -7.413 | 1.00 | 58.12 | 8 |
| ATOM | 213 | N | ALA | 28 | 11.948 | -9.268 | -9.444 | 1.00 | 60.15 | 7 |
| ATOM | 214 | CA | ALA | 28 | 11.373 | -10.411 | -10.152 | 1.00 | 53.52 | 6 |
| ATOM | 215 | CB | ALA | 28 | 11.213 | -10.080 | -11.617 | 1.00 | 43.26 | 6 |
| ATOM | 216 | C | ALA | 28 | 12.342 | -11.584 | -9.969 | 1.00 | 53.52 | 6 |
| ATOM | 217 | O | ALA | 28 | 11.945 | -12.714 | -9.671 | 1.00 | 51.26 | 8 |
| ATOM | 218 | N | ASP | 29 | 13.626 | -11.288 | -10.138 | 1.00 | 54.12 | 7 |
| ATOM | 219 | CA | ASP | 29 | 14.681 | -12.274 | -9.980 | 1.00 | 53.75 | 6 |
| ATOM | 220 | CB | ASP | 29 | 16.023 | -11.657 | -9.970 | 1.00 | 54.38 | 6 |
| ATOM | 221 | CG | ASP | 29 | 15.979 | -10.261 | -9.173 | 1.00 | 60.32 | 6 |
| ATOM | 222 | OD1 | ASP | 29 | 15.429 | -9.251 | -9.672 | 1.00 | 46.03 | 8 |
| ATOM | 223 | OD2 | ASP | 29 | 16.492 | -10.257 | -8.033 | 1.00 | 69.75 | 8 |
| ATOM | 224 | C | ASP | 29 | 14.480 | -13.010 | -8.661 | 1.00 | 59.28 | 6 |
| ATOM | 225 | O | ASP | 29 | 14.939 | -14.140 | -8.494 | 1.00 | 60.59 | 8 |
| ATOM | 226 | N | GLY | 30 | 13.792 | -12.350 | -7.729 | 1.00 | 62.20 | 7 |
| ATOM | 227 | CA | GLY | 30 | 13.527 | -12.928 | -6.421 | 1.00 | 54.79 | 6 |
| ATOM | 228 | C | GLY | 30 | 14.808 | -13.186 | -5.653 | 1.00 | 51.53 | 6 |
| ATOM | 229 | O | GLY | 30 | 14.828 | -13.999 | -4.731 | 1.00 | 47.05 | 8 |
| ATOM | 230 | N | GLY | 31 | 15.879 | -12.491 | -6.034 | 1.00 | 51.92 | 7 |
| ATOM | 231 | CA | GLY | 31 | 17.159 | -12.666 | -5.368 | 1.00 | 48.07 | 6 |

F i g. 1 3

| ATOM | 232 | C | GLY | 31 | 17.501 | -11.504 | -4.454 | 1.00 | 44.68 | 6 |
|---|---|---|---|---|---|---|---|---|---|---|
| ATOM | 233 | O | GLY | 31 | 18.673 | -11.260 | -4.197 | 1.00 | 50.25 | 8 |
| ATOM | 234 | N | GLY | 32 | 16.486 | -10.792 | -3.961 | 1.00 | 43.74 | 7 |
| ATOM | 235 | CA | GLY | 32 | 16.713 | -9.654 | -3.085 | 1.00 | 39.81 | 6 |
| ATOM | 236 | C | GLY | 32 | 17.577 | -8.682 | -3.857 | 1.00 | 45.44 | 6 |
| ATOM | 237 | O | GLY | 32 | 18.488 | -8.063 | -3.301 | 1.00 | 45.90 | 8 |
| ATOM | 238 | N | ASP | 33 | 17.269 | -8.554 | -5.151 | 1.00 | 46.63 | 7 |
| ATOM | 239 | CA | ASP | 33 | 18.023 | -7.709 | -6.090 | 1.00 | 45.20 | 6 |
| ATOM | 240 | CB | ASP | 33 | 18.964 | -8.608 | -6.906 | 1.00 | 40.99 | 6 |
| ATOM | 241 | CG | ASP | 33 | 20.370 | -8.069 | -6.988 | 1.00 | 45.73 | 6 |
| ATOM | 242 | OD1 | ASP | 33 | 21.054 | -8.412 | -7.967 | 1.00 | 50.21 | 8 |
| ATOM | 243 | OD2 | ASP | 33 | 20.800 | -7.312 | -6.087 | 1.00 | 47.17 | 8 |
| ATOM | 244 | C | ASP | 33 | 17.134 | -6.924 | -7.070 | 1.00 | 38.47 | 6 |
| ATOM | 245 | O | ASP | 33 | 16.108 | -7.433 | -7.533 | 1.00 | 43.47 | 8 |
| ATOM | 246 | N | ILE | 34 | 17.528 | -5.692 | -7.387 | 1.00 | 35.22 | 7 |
| ATOM | 247 | CA | ILE | 34 | 16.776 | -4.881 | -8.345 | 1.00 | 32.26 | 6 |
| ATOM | 248 | CB | ILE | 34 | 15.840 | -3.843 | -7.673 | 1.00 | 22.92 | 6 |
| ATOM | 249 | CG2 | ILE | 34 | 15.111 | -4.467 | -6.503 | 1.00 | 28.40 | 6 |
| ATOM | 250 | CG1 | ILE | 34 | 16.634 | -2.611 | -7.263 | 1.00 | 25.64 | 6 |
| ATOM | 251 | CD1 | ILE | 34 | 15.744 | -1.475 | -6.809 | 1.00 | 36.10 | 6 |
| ATOM | 252 | C | ILE | 34 | 17.738 | -4.145 | -9.259 | 1.00 | 35.95 | 6 |
| ATOM | 253 | O | ILE | 34 | 18.883 | -3.892 | -8.893 | 1.00 | 37.12 | 8 |
| ATOM | 254 | N | SER | 35 | 17.254 | -3.804 | -10.452 | 1.00 | 39.77 | 7 |
| ATOM | 255 | CA | SER | 35 | 18.052 | -3.112 | -11.489 | 1.00 | 38.24 | 6 |
| ATOM | 256 | CB | SER | 35 | 17.375 | -3.299 | -12.852 | 1.00 | 35.25 | 6 |
| ATOM | 257 | OG | SER | 35 | 17.326 | -2.108 | -13.626 | 1.00 | 33.53 | 8 |
| ATOM | 258 | C | SER | 35 | 18.229 | -1.626 | -11.135 | 1.00 | 33.33 | 6 |
| ATOM | 259 | O | SER | 35 | 17.272 | -0.967 | -10.725 | 1.00 | 31.36 | 8 |
| ATOM | 260 | N | VAL | 36 | 19.447 | -1.104 | -11.304 | 1.00 | 31.19 | 7 |
| ATOM | 261 | CA | VAL | 36 | 19.716 | 0.312 | -11.021 | 1.00 | 34.04 | 6 |
| ATOM | 262 | CB | VAL | 36 | 21.163 | 0.730 | -11.348 | 1.00 | 34.39 | 6 |
| ATOM | 263 | CG1 | VAL | 36 | 22.079 | 0.333 | -10.217 | 1.00 | 34.30 | 6 |
| ATOM | 264 | CG2 | VAL | 36 | 21.599 | 0.125 | -12.682 | 1.00 | 33.48 | 6 |
| ATOM | 265 | C | VAL | 36 | 18.818 | 1.238 | -11.826 | 1.00 | 39.19 | 6 |
| ATOM | 266 | O | VAL | 36 | 18.707 | 2.431 | -11.518 | 1.00 | 47.04 | 8 |
| ATOM | 267 | N | LYS | 37 | 18.191 | 0.702 | -12.867 | 1.00 | 36.11 | 7 |
| ATOM | 268 | CA | LYS | 37 | 17.310 | 1.508 | -13.690 | 1.00 | 34.02 | 6 |
| ATOM | 269 | CB | LYS | 37 | 17.208 | 0.892 | -15.084 | 1.00 | 36.45 | 6 |
| ATOM | 270 | CG | LYS | 37 | 18.118 | 1.545 | -16.105 | 1.00 | 33.17 | 6 |
| ATOM | 271 | CD | LYS | 37 | 19.130 | 0.575 | -16.651 | 1.00 | 22.87 | 6 |

F i g. 1 4

| ATOM | 272 | CB | LYS | 37 | 18.779 | 0.240 | -18.085 | 1.00 | 42.16 | 6 |
|---|---|---|---|---|---|---|---|---|---|---|
| ATOM | 273 | NZ | LYS | 37 | 19.767 | 0.764 | -19.084 | 1.00 | 39.30 | 7 |
| ATOM | 274 | C | LYS | 37 | 15.937 | 1.588 | -13.032 | 1.00 | 31.10 | 6 |
| ATOM | 275 | O | LYS | 37 | 15.022 | 2.201 | -13.555 | 1.00 | 28.23 | 8 |
| ATOM | 276 | N | GLU | 38 | 15.805 | 0.966 | -11.867 | 1.00 | 34.42 | 7 |
| ATOM | 277 | CA | GLU | 38 | 14.546 | 0.958 | -11.134 | 1.00 | 33.70 | 6 |
| ATOM | 278 | CB | GLU | 38 | 14.128 | -0.474 | -10.850 | 1.00 | 32.83 | 6 |
| ATOM | 279 | CG | GLU | 38 | 12.774 | -0.827 | -11.431 | 1.00 | 54.84 | 6 |
| ATOM | 280 | CD | GLU | 38 | 12.586 | -0.318 | -12.859 | 1.00 | 58.92 | 6 |
| ATOM | 281 | OE1 | GLU | 38 | 13.447 | -0.631 | -13.710 | 1.00 | 65.27 | 8 |
| ATOM | 282 | OE2 | GLU | 38 | 11.680 | 0.386 | -13.128 | 1.00 | 59.78 | 8 |
| ATOM | 283 | C | GLU | 38 | 14.633 | 1.730 | -9.825 | 1.00 | 30.51 | 6 |
| ATOM | 284 | O | GLU | 38 | 13.623 | 2.012 | -9.194 | 1.00 | 29.92 | 8 |
| ATOM | 285 | N | LEU | 39 | 15.856 | 2.060 | -9.435 | 1.00 | 30.22 | 7 |
| ATOM | 286 | CA | LEU | 39 | 16.113 | 2.796 | -8.205 | 1.00 | 34.42 | 6 |
| ATOM | 287 | CB | LEU | 39 | 17.585 | 3.187 | -8.133 | 1.00 | 39.53 | 6 |
| ATOM | 288 | CG | LEU | 39 | 18.328 | 3.083 | -6.796 | 1.00 | 50.13 | 6 |
| ATOM | 289 | CD1 | LEU | 39 | 19.391 | 4.187 | -6.771 | 1.00 | 42.75 | 6 |
| ATOM | 290 | CD2 | LEU | 39 | 17.378 | 3.203 | -5.592 | 1.00 | 41.09 | 6 |
| ATOM | 291 | C | LEU | 39 | 15.263 | 4.051 | -8.170 | 1.00 | 30.03 | 6 |
| ATOM | 292 | O | LEU | 39 | 14.313 | 4.155 | -7.406 | 1.00 | 34.44 | 8 |
| ATOM | 293 | N | GLY | 40 | 15.621 | 5.017 | -8.995 | 1.00 | 27.94 | 7 |
| ATOM | 294 | CA | GLY | 40 | 14.853 | 6.234 | -9.035 | 1.00 | 21.54 | 6 |
| ATOM | 295 | C | GLY | 40 | 13.385 | 6.003 | -8.754 | 1.00 | 27.92 | 6 |
| ATOM | 296 | O | GLY | 40 | 12.852 | 6.606 | -7.834 | 1.00 | 28.56 | 8 |
| ATOM | 297 | N | THR | 41 | 12.720 | 5.120 | -9.497 | 1.00 | 34.46 | 7 |
| ATOM | 298 | CA | THR | 41 | 11.283 | 4.927 | -9.263 | 1.00 | 40.52 | 6 |
| ATOM | 299 | CB | THR | 41 | 10.569 | 4.121 | -10.459 | 1.00 | 34.79 | 6 |
| ATOM | 300 | OG1 | THR | 41 | 9.703 | 3.105 | -9.937 | 1.00 | 42.98 | 8 |
| ATOM | 301 | CG2 | THR | 41 | 11.578 | 3.496 | -11.402 | 1.00 | 35.28 | 6 |
| ATOM | 302 | C | THR | 41 | 10.940 | 4.339 | -7.864 | 1.00 | 45.63 | 6 |
| ATOM | 303 | O | THR | 41 | 9.972 | 4.782 | -7.214 | 1.00 | 36.63 | 8 |
| ATOM | 304 | N | VAL | 42 | 11.739 | 3.380 | -7.384 | 1.00 | 44.34 | 7 |
| ATOM | 305 | CA | VAL | 42 | 11.493 | 2.799 | -6.054 | 1.00 | 39.86 | 6 |
| ATOM | 306 | CB | VAL | 42 | 12.458 | 1.619 | -5.752 | 1.00 | 37.59 | 6 |
| ATOM | 307 | CG1 | VAL | 42 | 12.366 | 1.237 | -4.301 | 1.00 | 27.39 | 6 |
| ATOM | 308 | CG2 | VAL | 42 | 12.107 | 0.419 | -6.616 | 1.00 | 35.98 | 6 |
| ATOM | 309 | C | VAL | 42 | 11.672 | 3.882 | -4.986 | 1.00 | 30.80 | 6 |
| ATOM | 310 | O | VAL | 42 | 10.909 | 3.960 | -4.032 | 1.00 | 34.33 | 8 |
| ATOM | 311 | N | MET | 43 | 12.675 | 4.730 | -5.170 | 1.00 | 28.93 | 7 |

Fig. 15

| | | | | | | | | |
|------|-----|-----|-----|----|--------|--------|--------|------|----|
| ATOM | 312 | CA | MET | 43 | 12.944 | 5.804 | -4.232 | 1.00 27.60 | 6 |
| ATOM | 313 | CB | MET | 43 | 14.242 | 6.504 | -4.602 | 1.00 17.69 | 6 |
| ATOM | 314 | CG | MET | 43 | 15.512 | 5.688 | -4.368 | 1.00 12.73 | 6 |
| ATOM | 315 | SD | MET | 43 | 17.054 | 6.677 | -4.346 | 1.00 26.19 | 16 |
| ATOM | 316 | CE | MET | 43 | 16.687 | 7.908 | -3.070 | 1.00 25.29 | 6 |
| ATOM | 317 | C | MET | 43 | 11.809 | 6.815 | -4.227 | 1.00 33.14 | 6 |
| ATOM | 318 | O | MET | 43 | 11.599 | 7.519 | -3.239 | 1.00 38.63 | 8 |
| ATOM | 319 | N | ARG | 44 | 11.077 | 6.883 | -5.338 | 1.00 36.18 | 7 |
| ATOM | 320 | CA | ARG | 44 | 9.955 | 7.814 | -5.451 | 1.00 35.77 | 6 |
| ATOM | 321 | CB | ARG | 44 | 9.498 | 8.000 | -6.918 | 1.00 42.57 | 6 |
| ATOM | 322 | CG | ARG | 44 | 10.578 | 8.217 | -7.968 | 1.00 37.02 | 6 |
| ATOM | 323 | CD | ARG | 44 | 11.457 | 9.413 | -7.660 | 1.00 29.86 | 6 |
| ATOM | 324 | NE | ARG | 44 | 10.808 | 10.679 | -7.940 | 1.00 24.98 | 7 |
| ATOM | 325 | CZ | ARG | 44 | 11.406 | 11.722 | -8.505 | 1.00 22.65 | 6 |
| ATOM | 326 | NH1 | ARG | 44 | 12.678 | 11.652 | -8.857 | 1.00 22.62 | 7 |
| ATOM | 327 | NH2 | ARG | 44 | 10.741 | 12.857 | -8.669 | 1.00 27.38 | 7 |
| ATOM | 328 | C | ARG | 44 | 8.768 | 7.299 | -4.636 | 1.00 31.14 | 6 |
| ATOM | 329 | O | ARG | 44 | 8.031 | 8.086 | -4.065 | 1.00 23.85 | 8 |
| ATOM | 330 | N | MET | 45 | 8.579 | 5.978 | -4.622 | 1.00 35.76 | 7 |
| ATOM | 331 | CA | MET | 45 | 7.490 | 5.345 | -3.875 | 1.00 34.51 | 6 |
| ATOM | 332 | CB | MET | 45 | 7.495 | 3.833 | -4.104 | 1.00 38.44 | 6 |
| ATOM | 333 | CG | MET | 45 | 7.251 | 3.394 | -5.541 | 1.00 41.84 | 6 |
| ATOM | 334 | SD | MET | 45 | 7.161 | 1.586 | -5.708 | 1.00 48.55 | 16 |
| ATOM | 335 | CE | MET | 45 | 5.434 | 1.249 | -5.285 | 1.00 38.00 | 6 |
| ATOM | 336 | C | MET | 45 | 7.625 | 5.604 | -2.372 | 1.00 33.98 | 6 |
| ATOM | 337 | O | MET | 45 | 6.623 | 5.696 | -1.664 | 1.00 34.77 | 8 |
| ATOM | 338 | N | LEU | 46 | 8.864 | 5.725 | -1.895 | 1.00 31.27 | 7 |
| ATOM | 339 | CA | LEU | 46 | 9.121 | 5.946 | -0.474 | 1.00 31.95 | 6 |
| ATOM | 340 | CB | LEU | 46 | 10.143 | 4.921 | 0.052 | 1.00 29.27 | 6 |
| ATOM | 341 | CG | LEU | 46 | 9.895 | 3.442 | -0.306 | 1.00 24.57 | 6 |
| ATOM | 342 | CD1 | LEU | 46 | 11.158 | 2.649 | -0.046 | 1.00 29.80 | 6 |
| ATOM | 343 | CD2 | LEU | 46 | 8.744 | 2.863 | 0.493 | 1.00 18.79 | 6 |
| ATOM | 344 | C | LEU | 46 | 9.596 | 7.358 | -0.174 | 1.00 29.15 | 6 |
| ATOM | 345 | O | LEU | 46 | 10.595 | 7.566 | 0.508 | 1.00 34.66 | 8 |
| ATOM | 346 | N | GLY | 47 | 8.888 | 8.327 | -0.725 | 1.00 30.32 | 7 |
| ATOM | 347 | CA | GLY | 47 | 9.200 | 9.722 | -0.463 | 1.00 27.92 | 6 |
| ATOM | 348 | C | GLY | 47 | 10.396 | 10.452 | -1.040 | 1.00 26.71 | 6 |
| ATOM | 349 | O | GLY | 47 | 10.317 | 11.662 | -1.225 | 1.00 37.63 | 8 |
| ATOM | 350 | N | GLN | 48 | 11.496 | 9.770 | -1.319 | 1.00 29.05 | 7 |
| ATOM | 351 | CA | GLN | 48 | 12.662 | 10.465 | -1.850 | 1.00 28.95 | 6 |

F i g. 1 6

| | | | | | | | | | | |
|---|---|---|---|---|---|---|---|---|---|---|
| ATOM | 352 | CB | GLN | 48 | 13.883 | 9.563 | -1.771 | 1.00 | 28.05 | 6 |
| ATOM | 353 | CG | GLN | 48 | 14.086 | 8.927 | -0.400 | 1.00 | 43.28 | 6 |
| ATOM | 354 | CD | GLN | 48 | 13.705 | 9.839 | 0.767 | 1.00 | 37.05 | 6 |
| ATOM | 355 | OE1 | GLN | 48 | 12.700 | 9.610 | 1.436 | 1.00 | 43.87 | 8 |
| ATOM | 356 | NE2 | GLN | 48 | 14.513 | 10.862 | 1.019 | 1.00 | 35.67 | 7 |
| ATOM | 357 | C | GLN | 48 | 12.494 | 10.974 | -3.284 | 1.00 | 35.70 | 6 |
| ATOM | 358 | O | GLN | 48 | 11.880 | 10.306 | -4.123 | 1.00 | 38.79 | 8 |
| ATOM | 359 | N | THR | 49 | 13.045 | 12.167 | -3.538 | 1.00 | 31.83 | 7 |
| ATOM | 360 | CA | THR | 49 | 12.991 | 12.813 | -4.842 | 1.00 | 23.93 | 6 |
| ATOM | 361 | CB | THR | 49 | 11.960 | 13.921 | -4.867 | 1.00 | 17.55 | 6 |
| ATOM | 362 | OG1 | THR | 49 | 12.362 | 14.947 | -3.952 | 1.00 | 25.12 | 8 |
| ATOM | 363 | CG2 | THR | 49 | 10.587 | 13.384 | -4.480 | 1.00 | 22.92 | 6 |
| ATOM | 364 | C | THR | 49 | 14.338 | 13.412 | -5.255 | 1.00 | 18.89 | 6 |
| ATOM | 365 | O | THR | 49 | 14.497 | 14.621 | -5.393 | 1.00 | 19.01 | 8 |
| ATOM | 366 | N | PRO | 50 | 15.319 | 12.563 | -5.510 | 1.00 | 20.20 | 7 |
| ATOM | 367 | CD | PRO | 50 | 15.290 | 11.085 | -5.541 | 1.00 | 12.28 | 6 |
| ATOM | 368 | CA | PRO | 50 | 16.615 | 13.087 | -5.909 | 1.00 | 23.21 | 6 |
| ATOM | 369 | CB | PRO | 50 | 17.538 | 11.879 | -5.812 | 1.00 | 20.58 | 6 |
| ATOM | 370 | CG | PRO | 50 | 16.640 | 10.727 | -6.155 | 1.00 | 12.67 | 6 |
| ATOM | 371 | C | PRO | 50 | 16.472 | 13.520 | -7.356 | 1.00 | 28.87 | 6 |
| ATOM | 372 | O | PRO | 50 | 15.617 | 13.011 | -8.073 | 1.00 | 27.90 | 8 |
| ATOM | 373 | N | THR | 51 | 17.300 | 14.458 | -7.782 | 1.00 | 29.74 | 7 |
| ATOM | 374 | CA | THR | 51 | 17.286 | 14.890 | -9.161 | 1.00 | 26.00 | 6 |
| ATOM | 375 | CB | THR | 51 | 18.044 | 16.173 | -9.335 | 1.00 | 25.85 | 6 |
| ATOM | 376 | OG1 | THR | 51 | 19.446 | 15.902 | -9.199 | 1.00 | 30.81 | 8 |
| ATOM | 377 | CG2 | THR | 51 | 17.605 | 17.181 | -8.292 | 1.00 | 25.93 | 6 |
| ATOM | 378 | C | THR | 51 | 18.029 | 13.788 | -9.910 | 1.00 | 28.36 | 6 |
| ATOM | 379 | O | THR | 51 | 18.875 | 13.111 | -9.327 | 1.00 | 22.30 | 8 |
| ATOM | 380 | N | LYS | 52 | 17.698 | 13.614 | -11.192 | 1.00 | 28.52 | 7 |
| ATOM | 381 | CA | LYS | 52 | 18.303 | 12.589 | -12.036 | 1.00 | 23.94 | 6 |
| ATOM | 382 | CB | LYS | 52 | 17.868 | 12.815 | -13.493 | 1.00 | 36.36 | 6 |
| ATOM | 383 | CG | LYS | 52 | 18.410 | 11.827 | -14.505 | 1.00 | 24.39 | 6 |
| ATOM | 384 | CD | LYS | 52 | 18.175 | 10.392 | -14.058 | 1.00 | 37.93 | 6 |
| ATOM | 385 | CE | LYS | 52 | 19.044 | 9.411 | -14.845 | 1.00 | 31.48 | 6 |
| ATOM | 386 | NZ | LYS | 52 | 18.821 | 8.016 | -14.410 | 1.00 | 34.12 | 7 |
| ATOM | 387 | C | LYS | 52 | 19.826 | 12.612 | -11.897 | 1.00 | 28.22 | 6 |
| ATOM | 388 | O | LYS | 52 | 20.481 | 11.562 | -11.941 | 1.00 | 28.07 | 8 |
| ATOM | 389 | N | GLU | 53 | 20.389 | 13.806 | -11.707 | 1.00 | 31.02 | 7 |
| ATOM | 390 | CA | GLU | 53 | 21.832 | 13.929 | -11.543 | 1.00 | 34.03 | 6 |
| ATOM | 391 | CB | GLU | 53 | 22.274 | 15.375 | -11.649 | 1.00 | 38.12 | 6 |

Ｆｉｇ． １７

| ATOM | 392 | CG | GLU | 53 | 23.751 | 15.515 | -11.922 | 1.00 | 50.71 | 6 |
|------|-----|-----|-----|-----|--------|--------|---------|------|-------|---|
| ATOM | 393 | CD | GLU | 53 | 24.101 | 16.882 | -12.473 | 1.00 | 71.63 | 6 |
| ATOM | 394 | OE1 | GLU | 53 | 23.212 | 17.764 | -12.461 | 1.00 | 78.31 | 8 |
| ATOM | 395 | OE2 | GLU | 53 | 25.260 | 17.077 | -12.918 | 1.00 | 79.69 | 8 |
| ATOM | 396 | C | GLU | 53 | 22.267 | 13.383 | -10.196 | 1.00 | 38.70 | 6 |
| ATOM | 397 | O | GLU | 53 | 23.376 | 12.867 | -10.057 | 1.00 | 33.20 | 8 |
| ATOM | 398 | N | GLU | 54 | 21.392 | 13.511 | -9.201 | 1.00 | 40.99 | 7 |
| ATOM | 399 | CA | GLU | 54 | 21.678 | 13.000 | -7.863 | 1.00 | 36.27 | 6 |
| ATOM | 400 | CB | GLU | 54 | 20.719 | 13.618 | -6.854 | 1.00 | 38.64 | 6 |
| ATOM | 401 | CG | GLU | 54 | 21.190 | 14.928 | -6.300 | 1.00 | 37.78 | 6 |
| ATOM | 402 | CD | GLU | 54 | 20.091 | 15.647 | -5.543 | 1.00 | 44.27 | 6 |
| ATOM | 403 | OE1 | GLU | 54 | 20.081 | 16.896 | -5.595 | 1.00 | 51.37 | 8 |
| ATOM | 404 | OE2 | GLU | 54 | 19.244 | 14.968 | -4.907 | 1.00 | 41.33 | 8 |
| ATOM | 405 | C | GLU | 54 | 21.521 | 11.478 | -7.847 | 1.00 | 31.49 | 6 |
| ATOM | 406 | O | GLU | 54 | 22.279 | 10.774 | -7.186 | 1.00 | 32.81 | 8 |
| ATOM | 407 | N | LEU | 55 | 20.527 | 10.985 | -8.579 | 1.00 | 28.36 | 7 |
| ATOM | 408 | CA | LEU | 55 | 20.274 | 9.554 | -8.677 | 1.00 | 25.96 | 6 |
| ATOM | 409 | CB | LEU | 55 | 18.995 | 9.297 | -9.478 | 1.00 | 15.75 | 6 |
| ATOM | 410 | CG | LEU | 55 | 18.538 | 7.853 | -9.690 | 1.00 | 24.52 | 6 |
| ATOM | 411 | CD1 | LEU | 55 | 18.232 | 7.113 | -8.360 | 1.00 | 22.86 | 6 |
| ATOM | 412 | CD2 | LEU | 55 | 17.301 | 7.902 | -10.555 | 1.00 | 22.16 | 6 |
| ATOM | 413 | C | LEU | 55 | 21.461 | 8.912 | -9.377 | 1.00 | 26.75 | 6 |
| ATOM | 414 | O | LEU | 55 | 21.814 | 7.750 | -9.116 | 1.00 | 27.04 | 8 |
| ATOM | 415 | N | ASP | 56 | 22.094 | 9.671 | -10.260 | 1.00 | 21.76 | 7 |
| ATOM | 416 | CA | ASP | 56 | 23.233 | 9.124 | -10.955 | 1.00 | 33.41 | 6 |
| ATOM | 417 | CB | ASP | 56 | 23.575 | 9.984 | -12.173 | 1.00 | 38.91 | 6 |
| ATOM | 418 | CG | ASP | 56 | 22.716 | 9.643 | -13.394 | 1.00 | 34.55 | 6 |
| ATOM | 419 | OD1 | ASP | 56 | 22.248 | 8.475 | -13.523 | 1.00 | 27.30 | 8 |
| ATOM | 420 | OD2 | ASP | 56 | 22.524 | 10.561 | -14.220 | 1.00 | 32.64 | 8 |
| ATOM | 421 | C | ASP | 56 | 24.410 | 9.017 | -9.983 | 1.00 | 37.68 | 6 |
| ATOM | 422 | O | ASP | 56 | 25.136 | 8.009 | -9.984 | 1.00 | 32.88 | 8 |
| ATOM | 423 | N | ALA | 57 | 24.587 | 10.043 | -9.147 | 1.00 | 35.37 | 7 |
| ATOM | 424 | CA | ALA | 57 | 25.660 | 10.047 | -8.146 | 1.00 | 32.50 | 6 |
| ATOM | 425 | CB | ALA | 57 | 25.575 | 11.285 | -7.295 | 1.00 | 29.68 | 6 |
| ATOM | 426 | C | ALA | 57 | 25.523 | 8.805 | -7.269 | 1.00 | 30.65 | 6 |
| ATOM | 427 | O | ALA | 57 | 26.484 | 8.043 | -7.104 | 1.00 | 30.57 | 8 |
| ATOM | 428 | N | ILE | 58 | 24.314 | 8.612 | -6.729 | 1.00 | 30.66 | 7 |
| ATOM | 429 | CA | ILE | 58 | 23.964 | 7.466 | -5.870 | 1.00 | 29.84 | 6 |
| ATOM | 430 | CB | ILE | 58 | 22.447 | 7.445 | -5.569 | 1.00 | 27.46 | 6 |
| ATOM | 431 | CG2 | ILE | 58 | 22.058 | 6.139 | -4.930 | 1.00 | 28.94 | 6 |

F i g. 1 8

| | | | | | | | | | |
|---|---|---|---|---|---|---|---|---|---|
| ATOM | 432 | CG1 | ILE | 58 | 22.069 | 8.615 | -4.661 | 1.00 30.66 | 6 |
| ATOM | 433 | CD1 | ILE | 58 | 20.601 | 8.997 | -4.750 | 1.00 35.09 | 6 |
| ATOM | 434 | C | ILE | 58 | 24.334 | 6.136 | -6.525 | 1.00 27.86 | 6 |
| ATOM | 435 | O | ILE | 58 | 25.016 | 5.307 | -5.922 | 1.00 29.39 | 8 |
| ATOM | 436 | N | ILE | 59 | 23.854 | 5.934 | -7.751 | 1.00 32.15 | 7 |
| ATOM | 437 | CA | ILE | 59 | 24.141 | 4.725 | -8.526 | 1.00 27.42 | 6 |
| ATOM | 438 | CB | ILE | 59 | 23.590 | 4.875 | -9.960 | 1.00 35.35 | 6 |
| ATOM | 439 | CG2 | ILE | 59 | 23.945 | 3.651 | -10.814 | 1.00 32.20 | 6 |
| ATOM | 440 | CG1 | ILE | 59 | 22.082 | 5.156 | -9.893 | 1.00 36.30 | 6 |
| ATOM | 441 | CD1 | ILE | 59 | 21.207 | 3.937 | -9.741 | 1.00 45.19 | 6 |
| ATOM | 442 | C | ILE | 59 | 25.653 | 4.475 | -8.595 | 1.00 27.96 | 6 |
| ATOM | 443 | O | ILE | 59 | 26.102 | 3.333 | -8.547 | 1.00 25.83 | 8 |
| ATOM | 444 | N | GLU | 60 | 26.430 | 5.554 | -8.707 | 1.00 36.59 | 7 |
| ATOM | 445 | CA | GLU | 60 | 27.897 | 5.473 | -8.777 | 1.00 38.36 | 6 |
| ATOM | 446 | CB | GLU | 60 | 28.509 | 6.830 | -9.141 | 1.00 38.28 | 6 |
| ATOM | 447 | CG | GLU | 60 | 29.538 | 6.790 | -10.387 | 1.00 53.94 | 6 |
| ATOM | 448 | CD | GLU | 60 | 30.639 | 5.730 | -10.120 | 1.00 60.76 | 6 |
| ATOM | 449 | OE1 | GLU | 60 | 31.788 | 6.110 | -9.781 | 1.00 53.65 | 8 |
| ATOM | 450 | OE2 | GLU | 60 | 30.359 | 4.523 | -10.345 | 1.00 61.79 | 8 |
| ATOM | 451 | C | GLU | 60 | 28.492 | 5.030 | -7.454 | 1.00 36.12 | 6 |
| ATOM | 452 | O | GLU | 60 | 29.454 | 4.279 | -7.429 | 1.00 38.23 | 8 |
| ATOM | 453 | N | GLU | 61 | 27.921 | 5.521 | -6.361 | 1.00 42.95 | 7 |
| ATOM | 454 | CA | GLU | 61 | 28.372 | 5.179 | -5.011 | 1.00 46.78 | 6 |
| ATOM | 455 | CB | GLU | 61 | 27.683 | 6.093 | -3.975 | 1.00 51.76 | 6 |
| ATOM | 456 | CG | GLU | 61 | 27.982 | 5.763 | -2.515 | 1.00 45.04 | 6 |
| ATOM | 457 | CD | GLU | 61 | 29.403 | 6.138 | -2.118 | 1.00 49.81 | 6 |
| ATOM | 458 | OE1 | GLU | 61 | 29.857 | 7.241 | -2.510 | 1.00 42.86 | 8 |
| ATOM | 459 | OE2 | GLU | 61 | 30.061 | 5.328 | -1.425 | 1.00 45.49 | 8 |
| ATOM | 460 | C | GLU | 61 | 28.094 | 3.703 | -4.680 | 1.00 42.14 | 6 |
| ATOM | 461 | O | GLU | 61 | 29.028 | 2.915 | -4.561 | 1.00 48.78 | 8 |
| ATOM | 462 | N | VAL | 62 | 26.819 | 3.339 | -4.530 | 1.00 40.54 | 7 |
| ATOM | 463 | CA | VAL | 62 | 26.433 | 1.960 | -4.216 | 1.00 40.69 | 6 |
| ATOM | 464 | CB | VAL | 62 | 24.916 | 1.789 | -4.271 | 1.00 40.05 | 6 |
| ATOM | 465 | CG1 | VAL | 62 | 24.556 | 0.312 | -4.166 | 1.00 35.16 | 6 |
| ATOM | 466 | CG2 | VAL | 62 | 24.274 | 2.599 | -3.156 | 1.00 39.40 | 6 |
| ATOM | 467 | C | VAL | 62 | 27.057 | 0.932 | -5.160 | 1.00 44.20 | 6 |
| ATOM | 468 | O | VAL | 62 | 27.868 | 0.098 | -4.751 | 1.00 50.30 | 8 |
| ATOM | 469 | N | ASP | 63 | 26.646 | 0.978 | -6.420 | 1.00 50.86 | 7 |
| ATOM | 470 | CA | ASP | 63 | 27.185 | 0.081 | -7.439 | 1.00 53.36 | 6 |
| ATOM | 471 | CB | ASP | 63 | 26.142 | -0.144 | -8.533 | 1.00 38.11 | 6 |

Fig. 19

| ATOM | 472 | CG | ASP | 63 | 26.388 | -1.409 | -9.308 | 1.00 | 39.86 | 6 |
|------|-----|-----|-----|----|--------|--------|--------|------|-------|---|
| ATOM | 473 | OD1 | ASP | 63 | 26.991 | -2.346 | -8.741 | 1.00 | 35.15 | 8 |
| ATOM | 474 | OD2 | ASP | 63 | 25.976 | -1.462 | -10.486 | 1.00 | 42.36 | 8 |
| ATOM | 475 | C | ASP | 63 | 28.459 | 0.702 | -8.038 | 1.00 | 55.14 | 6 |
| ATOM | 476 | O | ASP | 63 | 28.402 | 1.415 | -9.036 | 1.00 | 57.91 | 8 |
| ATOM | 477 | N | GLU | 64 | 29.601 | 0.430 | -7.405 | 1.00 | 57.00 | 7 |
| ATOM | 478 | CA | GLU | 64 | 30.899 | 0.952 | -7.841 | 1.00 | 55.75 | 6 |
| ATOM | 479 | CB | GLU | 64 | 31.838 | 1.077 | -6.640 | 1.00 | 57.49 | 6 |
| ATOM | 480 | CG | GLU | 64 | 31.895 | 2.462 | -6.016 | 1.00 | 64.94 | 6 |
| ATOM | 481 | CD | GLU | 64 | 32.767 | 2.510 | -4.773 | 1.00 | 64.96 | 6 |
| ATOM | 482 | OE1 | GLU | 64 | 33.347 | 1.460 | -4.415 | 1.00 | 64.02 | 8 |
| ATOM | 483 | OE2 | GLU | 64 | 32.868 | 3.598 | -4.160 | 1.00 | 60.93 | 8 |
| ATOM | 484 | C | GLU | 64 | 31.543 | 0.032 | -8.874 | 1.00 | 59.46 | 6 |
| ATOM | 485 | O | GLU | 64 | 32.031 | 0.475 | -9.912 | 1.00 | 58.29 | 8 |
| ATOM | 486 | N | ASP | 65 | 31.546 | -1.260 | -8.553 | 1.00 | 64.41 | 7 |
| ATOM | 487 | CA | ASP | 65 | 32.102 | -2.304 | -9.417 | 1.00 | 68.96 | 6 |
| ATOM | 488 | CB | ASP | 65 | 32.076 | -3.660 | -8.713 | 1.00 | 74.83 | 6 |
| ATOM | 489 | CG | ASP | 65 | 30.669 | -4.225 | -8.658 | 1.00 | 83.52 | 6 |
| ATOM | 490 | OD1 | ASP | 65 | 30.408 | -5.330 | -9.091 | 1.00 | 82.65 | 8 |
| ATOM | 491 | OD2 | ASP | 65 | 29.808 | -3.576 | -7.903 | 1.00 | 85.23 | 8 |
| ATOM | 492 | C | ASP | 65 | 31.286 | -2.375 | -10.730 | 1.00 | 66.98 | 6 |
| ATOM | 493 | O | ASP | 65 | 31.773 | -2.846 | -11.747 | 1.00 | 74.78 | 8 |
| ATOM | 494 | N | GLY | 66 | 30.036 | -1.959 | -10.670 | 1.00 | 59.15 | 7 |
| ATOM | 495 | CA | GLY | 66 | 29.222 | -1.927 | -11.864 | 1.00 | 57.71 | 6 |
| ATOM | 496 | C | GLY | 66 | 28.453 | -3.111 | -12.405 | 1.00 | 58.73 | 6 |
| ATOM | 497 | O | GLY | 66 | 28.705 | -3.500 | -13.531 | 1.00 | 68.82 | 8 |
| ATOM | 498 | N | SER | 67 | 27.586 | -3.726 | -11.611 | 1.00 | 56.54 | 7 |
| ATOM | 499 | CA | SER | 67 | 26.786 | -4.817 | -12.134 | 1.00 | 48.12 | 6 |
| ATOM | 500 | CB | SER | 67 | 26.732 | -5.907 | -11.100 | 1.00 | 58.98 | 6 |
| ATOM | 501 | OG | SER | 67 | 26.275 | -5.387 | -9.854 | 1.00 | 53.58 | 8 |
| ATOM | 502 | C | SER | 67 | 25.380 | -4.472 | -12.658 | 1.00 | 42.21 | 6 |
| ATOM | 503 | O | SER | 67 | 24.753 | -5.279 | -13.224 | 1.00 | 38.01 | 8 |
| ATOM | 504 | N | GLY | 68 | 24.902 | -3.281 | -12.205 | 1.00 | 37.47 | 7 |
| ATOM | 505 | CA | GLY | 68 | 23.559 | -2.883 | -12.570 | 1.00 | 36.82 | 6 |
| ATOM | 506 | C | GLY | 68 | 22.454 | -3.195 | -11.551 | 1.00 | 42.19 | 6 |
| ATOM | 507 | O | GLY | 68 | 21.262 | -3.023 | -11.850 | 1.00 | 44.64 | 8 |
| ATOM | 508 | N | THR | 69 | 22.830 | -3.625 | -10.349 | 1.00 | 40.58 | 7 |
| ATOM | 509 | CA | THR | 69 | 21.868 | -3.954 | -9.321 | 1.00 | 38.11 | 6 |
| ATOM | 510 | CB | THR | 69 | 21.492 | -5.465 | -9.379 | 1.00 | 34.81 | 6 |
| ATOM | 511 | OG1 | THR | 69 | 22.676 | -6.278 | -9.310 | 1.00 | 37.46 | 8 |

Fig. 20

| ATOM | 512 | CG2 | THR | 69 | 20.751 | -5.768 | -10.666 | 1.00 | 33.06 | 6 |
|------|-----|-----|-----|-----|--------|--------|---------|------|-------|---|
| ATOM | 513 | C | THR | 69 | 22.336 | -3.606 | -7.899 | 1.00 | 36.91 | 6 |
| ATOM | 514 | O | THR | 69 | 23.516 | -3.313 | -7.656 | 1.00 | 33.26 | 8 |
| ATOM | 515 | N | ILE | 70 | 21.397 | -3.659 | -6.957 | 1.00 | 38.76 | 7 |
| ATOM | 516 | CA | ILE | 70 | 21.684 | -3.373 | -5.582 | 1.00 | 41.86 | 6 |
| ATOM | 517 | CB | ILE | 70 | 21.484 | -1.904 | -5.196 | 1.00 | 44.89 | 6 |
| ATOM | 518 | CG2 | ILE | 70 | 22.503 | -1.058 | -5.902 | 1.00 | 45.88 | 6 |
| ATOM | 519 | CG1 | ILE | 70 | 20.039 | -1.481 | -5.472 | 1.00 | 44.53 | 6 |
| ATOM | 520 | CD1 | ILE | 70 | 19.582 | -0.335 | -4.639 | 1.00 | 43.98 | 6 |
| ATOM | 521 | C | ILE | 70 | 20.787 | -4.186 | -4.700 | 1.00 | 42.10 | 6 |
| ATOM | 522 | O | ILE | 70 | 19.618 | -4.423 | -5.011 | 1.00 | 43.21 | 8 |
| ATOM | 523 | N | ASP | 71 | 21.344 | -4.592 | -3.572 | 1.00 | 42.69 | 7 |
| ATOM | 524 | CA | ASP | 71 | 20.635 | -5.378 | -2.616 | 1.00 | 45.76 | 6 |
| ATOM | 525 | CB | ASP | 71 | 21.573 | -6.257 | -1.778 | 1.00 | 52.40 | 6 |
| ATOM | 526 | CG | ASP | 71 | 22.663 | -5.451 | -1.092 | 1.00 | 59.39 | 6 |
| ATOM | 527 | OD1 | ASP | 71 | 22.371 | -4.672 | -0.154 | 1.00 | 58.54 | 8 |
| ATOM | 528 | OD2 | ASP | 71 | 23.848 | -5.623 | -1.465 | 1.00 | 63.98 | 8 |
| ATOM | 529 | C | ASP | 71 | 19.887 | -4.430 | -1.705 | 1.00 | 48.90 | 6 |
| ATOM | 530 | O | ASP | 71 | 19.957 | -3.199 | -1.811 | 1.00 | 47.41 | 8 |
| ATOM | 531 | N | PHE | 72 | 19.191 | -5.043 | -0.794 | 1.00 | 50.05 | 7 |
| ATOM | 532 | CA | PHE | 72 | 18.399 | -4.361 | 0.178 | 1.00 | 52.95 | 6 |
| ATOM | 533 | CB | PHE | 72 | 17.416 | -5.402 | 0.666 | 1.00 | 55.80 | 6 |
| ATOM | 534 | CG | PHE | 72 | 16.565 | -4.961 | 1.733 | 1.00 | 59.50 | 6 |
| ATOM | 535 | CD1 | PHE | 72 | 15.794 | -3.807 | 1.640 | 1.00 | 59.80 | 6 |
| ATOM | 536 | CD2 | PHE | 72 | 16.507 | -5.688 | 2.908 | 1.00 | 61.02 | 6 |
| ATOM | 537 | CE1 | PHE | 72 | 15.013 | -3.399 | 2.676 | 1.00 | 63.76 | 6 |
| ATOM | 538 | CE2 | PHE | 72 | 15.737 | -5.296 | 3.951 | 1.00 | 70.04 | 6 |
| ATOM | 539 | CZ | PHE | 72 | 14.986 | -4.141 | 3.843 | 1.00 | 64.42 | 6 |
| ATOM | 540 | C | PHE | 72 | 19.199 | -3.686 | 1.303 | 1.00 | 48.62 | 6 |
| ATOM | 541 | O | PHE | 72 | 18.809 | -2.631 | 1.811 | 1.00 | 49.77 | 8 |
| ATOM | 542 | N | GLU | 73 | 20.310 | -4.280 | 1.704 | 1.00 | 41.54 | 7 |
| ATOM | 543 | CA | GLU | 73 | 21.134 | -3.679 | 2.730 | 1.00 | 40.12 | 6 |
| ATOM | 544 | CB | GLU | 73 | 22.175 | -4.692 | 3.195 | 1.00 | 40.13 | 6 |
| ATOM | 545 | CG | GLU | 73 | 21.594 | -6.044 | 3.587 | 1.00 | 39.49 | 6 |
| ATOM | 546 | CD | GLU | 73 | 20.680 | -5.979 | 4.787 | 1.00 | 50.87 | 6 |
| ATOM | 547 | OE1 | GLU | 73 | 19.795 | -6.852 | 4.914 | 1.00 | 59.42 | 8 |
| ATOM | 548 | OE2 | GLU | 73 | 20.817 | -5.070 | 5.635 | 1.00 | 61.57 | 8 |
| ATOM | 549 | C | GLU | 73 | 21.815 | -2.451 | 2.135 | 1.00 | 41.20 | 6 |
| ATOM | 550 | O | GLU | 73 | 22.091 | -1.478 | 2.833 | 1.00 | 39.12 | 8 |
| ATOM | 551 | N | GLU | 74 | 22.064 | -2.523 | 0.832 | 1.00 | 41.82 | 7 |

F i g. 2 1

| | | | | | | | | | |
|---|---|---|---|---|---|---|---|---|---|
| ATOM | 552 | CA | GLU | 74 | 22.682 | -1.439 | 0.076 | 1.00 42.42 | 6 |
| ATOM | 553 | CB | GLU | 74 | 23.118 | -1.932 | -1.305 | 1.00 41.32 | 6 |
| ATOM | 554 | CG | GLU | 74 | 24.381 | -2.775 | -1.297 | 1.00 45.00 | 6 |
| ATOM | 555 | CD | GLU | 74 | 24.764 | -3.292 | -2.679 | 1.00 43.00 | 6 |
| ATOM | 556 | OE1 | GLU | 74 | 25.698 | -2.732 | -3.284 | 1.00 47.78 | 8 |
| ATOM | 557 | OE2 | GLU | 74 | 24.144 | -4.256 | -3.168 | 1.00 41.10 | 8 |
| ATOM | 558 | C | GLU | 74 | 21.614 | -0.361 | -0.084 | 1.00 38.49 | 6 |
| ATOM | 559 | O | GLU | 74 | 21.888 | 0.834 | -0.046 | 1.00 34.24 | 8 |
| ATOM | 560 | N | PHE | 75 | 20.394 | -0.803 | -0.373 | 1.00 35.90 | 7 |
| ATOM | 561 | CA | PHE | 75 | 19.278 | 0.114 | -0.521 | 1.00 38.17 | 6 |
| ATOM | 562 | CB | PHE | 75 | 17.979 | -0.656 | -0.746 | 1.00 32.48 | 6 |
| ATOM | 563 | CG | PHE | 75 | 16.813 | 0.227 | -1.078 | 1.00 38.08 | 6 |
| ATOM | 564 | CD1 | PHE | 75 | 16.984 | 1.358 | -1.872 | 1.00 36.50 | 6 |
| ATOM | 565 | CD2 | PHE | 75 | 15.548 | -0.064 | -0.608 | 1.00 34.17 | 6 |
| ATOM | 566 | CE1 | PHE | 75 | 15.910 | 2.184 | -2.195 | 1.00 34.07 | 6 |
| ATOM | 567 | CE2 | PHE | 75 | 14.469 | 0.759 | -0.928 | 1.00 41.84 | 6 |
| ATOM | 568 | CZ | PHE | 75 | 14.855 | 1.885 | -1.726 | 1.00 27.97 | 8 |
| ATOM | 569 | C | PHE | 75 | 19.162 | 0.912 | 0.772 | 1.00 44.11 | 6 |
| ATOM | 570 | O | PHE | 75 | 19.069 | 2.145 | 0.764 | 1.00 45.23 | 8 |
| ATOM | 571 | N | LEU | 76 | 19.173 | 0.182 | 1.888 | 1.00 47.86 | 7 |
| ATOM | 572 | CA | LEU | 76 | 19.059 | 0.792 | 3.205 | 1.00 41.82 | 6 |
| ATOM | 573 | CB | LEU | 76 | 19.216 | -0.263 | 4.296 | 1.00 43.62 | 6 |
| ATOM | 574 | CG | LEU | 76 | 18.141 | -1.340 | 4.343 | 1.00 44.89 | 6 |
| ATOM | 575 | CD1 | LEU | 76 | 18.301 | -2.146 | 5.629 | 1.00 32.33 | 6 |
| ATOM | 576 | CD2 | LEU | 76 | 16.769 | -0.690 | 4.252 | 1.00 37.21 | 6 |
| ATOM | 577 | C | LEU | 76 | 20.126 | 1.842 | 3.381 | 1.00 38.09 | 6 |
| ATOM | 578 | O | LEU | 76 | 19.847 | 3.034 | 3.347 | 1.00 34.29 | 8 |
| ATOM | 579 | N | VAL | 77 | 21.355 | 1.378 | 3.565 | 1.00 36.39 | 7 |
| ATOM | 580 | CA | VAL | 77 | 22.482 | 2.270 | 3.775 | 1.00 42.31 | 6 |
| ATOM | 581 | CB | VAL | 77 | 23.833 | 1.589 | 3.401 | 1.00 35.08 | 6 |
| ATOM | 582 | CG1 | VAL | 77 | 24.999 | 2.525 | 3.713 | 1.00 32.98 | 6 |
| ATOM | 583 | CG2 | VAL | 77 | 23.985 | 0.283 | 4.161 | 1.00 38.81 | 6 |
| ATOM | 584 | C | VAL | 77 | 22.357 | 3.559 | 2.977 | 1.00 48.52 | 6 |
| ATOM | 585 | O | VAL | 77 | 22.590 | 4.645 | 3.522 | 1.00 55.64 | 8 |
| ATOM | 586 | N | MET | 78 | 21.960 | 3.443 | 1.705 | 1.00 50.25 | 7 |
| ATOM | 587 | CA | MET | 78 | 21.867 | 4.615 | 0.828 | 1.00 48.49 | 6 |
| ATOM | 588 | CB | MET | 78 | 21.959 | 4.173 | -0.653 | 1.00 47.00 | 6 |
| ATOM | 589 | CG | MET | 78 | 20.647 | 3.834 | -1.339 | 1.00 52.21 | 6 |
| ATOM | 590 | SD | MET | 78 | 19.794 | 5.315 | -1.872 | 1.00 52.98 | 16 |
| ATOM | 591 | CE | MET | 78 | 21.169 | 6.545 | -1.794 | 1.00 31.34 | 6 |

Fig. 22

| ATOM | 592 | C | MET | 78 | 20. 630 | 5. 467 | 1. 089 | 1. 00 | 43. 26 | 6 |
| ATOM | 593 | O | MET | 78 | 20. 716 | 6. 686 | 1. 247 | 1. 00 | 44. 08 | 8 |
| ATOM | 594 | N | MET | 79 | 19. 482 | 4. 817 | 1. 142 | 1. 00 | 36. 36 | 7 |
| ATOM | 595 | CA | MET | 79 | 18. 237 | 5. 499 | 1. 406 | 1. 00 | 34. 89 | 6 |
| ATOM | 596 | CB | MET | 79 | 17. 181 | 4. 443 | 1. 683 | 1. 00 | 37. 69 | 6 |
| ATOM | 597 | CG | MET | 79 | 15. 787 | 4. 894 | 1. 442 | 1. 00 | 48. 08 | 6 |
| ATOM | 598 | SD | MET | 79 | 15. 536 | 5. 434 | -0. 223 | 1. 00 | 46. 80 | 16 |
| ATOM | 599 | CE | MET | 79 | 13. 748 | 5. 191 | -0. 395 | 1. 00 | 37. 17 | 6 |
| ATOM | 600 | C | MET | 79 | 18. 420 | 6. 428 | 2. 616 | 1. 00 | 32. 77 | 6 |
| ATOM | 601 | O | MET | 79 | 18. 026 | 7. 590 | 2. 606 | 1. 00 | 30. 48 | 8 |
| ATOM | 602 | N | VAL | 80 | 19. 052 | 5. 905 | 3. 655 | 1. 00 | 39. 32 | 7 |
| ATOM | 603 | CA | VAL | 80 | 19. 279 | 6. 662 | 4. 871 | 1. 00 | 40. 26 | 6 |
| ATOM | 604 | CB | VAL | 80 | 19. 904 | 5. 767 | 5. 948 | 1. 00 | 44. 58 | 6 |
| ATOM | 605 | CG1 | VAL | 80 | 20. 175 | 6. 584 | 7. 193 | 1. 00 | 48. 89 | 6 |
| ATOM | 606 | CG2 | VAL | 80 | 18. 969 | 4. 614 | 6. 270 | 1. 00 | 40. 14 | 6 |
| ATOM | 607 | C | VAL | 80 | 20. 171 | 7. 883 | 4. 658 | 1. 00 | 43. 03 | 6 |
| ATOM | 608 | O | VAL | 80 | 19. 898 | 8. 975 | 5. 170 | 1. 00 | 44. 78 | 8 |
| ATOM | 609 | N | ARG | 81 | 21. 253 | 7. 698 | 3. 916 | 1. 00 | 46. 66 | 7 |
| ATOM | 610 | CA | ARG | 81 | 22. 149 | 8. 808 | 3. 653 | 1. 00 | 41. 97 | 6 |
| ATOM | 611 | CB | ARG | 81 | 23. 352 | 8. 352 | 2. 832 | 1. 00 | 35. 06 | 6 |
| ATOM | 612 | CG | ARG | 81 | 23. 932 | 9. 449 | 1. 969 | 1. 00 | 33. 58 | 6 |
| ATOM | 513 | CD | ARG | 81 | 25. 424 | 9. 674 | 2. 232 | 1. 00 | 41. 44 | 6 |
| ATOM | 614 | NE | ARG | 81 | 26. 286 | 8. 624 | 1. 669 | 1. 00 | 46. 16 | 7 |
| ATOM | 615 | CZ | ARG | 81 | 27. 134 | 8. 789 | 0. 648 | 1. 00 | 42. 30 | 6 |
| ATOM | 616 | NH1 | ARG | 81 | 27. 265 | 9. 968 | 0. 046 | 1. 00 | 41. 09 | 7 |
| ATOM | 617 | NH2 | ARG | 81 | 27. 870 | 7. 771 | 0. 234 | 1. 00 | 44. 14 | 7 |
| ATOM | 618 | C | ARG | 81 | 21. 364 | 9. 856 | 2. 886 | 1. 00 | 40. 67 | 6 |
| ATOM | 619 | O | ARG | 81 | 21. 401 | 11. 034 | 3. 221 | 1. 00 | 43. 67 | 8 |
| ATOM | 620 | N | GLN | 82 | 20. 627 | 9. 426 | 1. 869 | 1. 00 | 41. 98 | 7 |
| ATOM | 621 | CA | GLN | 82 | 19. 853 | 10. 375 | 1. 077 | 1. 00 | 42. 80 | 6 |
| ATOM | 622 | CB | GLN | 82 | 19. 078 | 9. 666 | -0. 035 | 1. 00 | 35. 01 | 6 |
| ATOM | 623 | CG | GLN | 82 | 18. 999 | 10. 467 | -1. 315 | 1. 00 | 37. 78 | 6 |
| ATOM | 624 | CD | GLN | 82 | 17. 606 | 11. 016 | -1. 602 | 1. 00 | 40. 98 | 6 |
| ATOM | 625 | OE1 | GLN | 82 | 16. 620 | 10. 280 | -1. 598 | 1. 00 | 41. 20 | 8 |
| ATOM | 626 | NE2 | GLN | 82 | 17. 528 | 12. 319 | -1. 874 | 1. 00 | 43. 72 | 7 |
| ATOM | 627 | C | GLN | 82 | 18. 879 | 11. 119 | 1. 987 | 1. 00 | 44. 54 | 6 |
| ATOM | 628 | O | GLN | 82 | 18. 893 | 12. 352 | 2. 041 | 1. 00 | 41. 38 | 8 |
| ATOM | 629 | N | MET | 83 | 18. 049 | 10. 365 | 2. 705 | 1. 00 | 44. 04 | 7 |
| ATOM | 630 | CA | MET | 83 | 17. 073 | 10. 960 | 3. 607 | 1. 00 | 44. 42 | 6 |
| ATOM | 631 | CB | MET | 83 | 16. 551 | 9. 912 | 4. 585 | 1. 00 | 42. 27 | 6 |

Fig. 23

| | | | | | | | | | |
|---|---|---|---|---|---|---|---|---|---|
| ATOM | 632 | CG | MET | 83 | 15.431 | 9.054 | 4.050 | 1.00 35.34 | 6 |
| ATOM | 633 | SD | MET | 83 | 14.874 | 7.848 | 5.270 | 1.00 48.46 | 16 |
| ATOM | 634 | CE | MET | 83 | 13.028 | 7.864 | 4.917 | 1.00 43.45 | 6 |
| ATOM | 635 | C | MET | 83 | 17.766 | 12.075 | 4.374 | 1.00 52.86 | 6 |
| ATOM | 636 | O | MET | 83 | 17.272 | 13.206 | 4.434 | 1.00 56.54 | 8 |
| ATOM | 637 | N | LYS | 84 | 18.929 | 11.755 | 4.943 | 1.00 54.05 | 7 |
| ATOM | 638 | CA | LYS | 84 | 19.701 | 12.731 | 5.713 | 1.00 56.22 | 6 |
| ATOM | 639 | CB | LYS | 84 | 20.947 | 12.072 | 6.313 | 1.00 51.27 | 6 |
| ATOM | 640 | CG | LYS | 84 | 21.750 | 12.989 | 7.231 | 1.00 54.95 | 6 |
| ATOM | 641 | CD | LYS | 84 | 23.191 | 13.142 | 6.753 | 1.00 61.38 | 6 |
| ATOM | 642 | CE | LYS | 84 | 23.897 | 11.781 | 6.665 | 1.00 56.98 | 6 |
| ATOM | 643 | NZ | LYS | 84 | 24.847 | 11.642 | 5.509 | 1.00 53.67 | 7 |
| ATOM | 644 | C | LYS | 84 | 20.117 | 13.938 | 4.868 | 1.00 58.29 | 6 |
| ATOM | 645 | O | LYS | 84 | 19.809 | 15.073 | 5.217 | 1.00 60.80 | 8 |
| ATOM | 646 | N | GLU | 85 | 20.813 | 13.689 | 3.760 | 1.00 61.51 | 7 |
| ATOM | 647 | CA | GLU | 85 | 21.274 | 14.758 | 2.876 | 1.00 61.65 | 6 |
| ATOM | 648 | CB | GLU | 85 | 21.812 | 14.140 | 1.581 | 1.00 63.51 | 6 |
| ATOM | 649 | CG | GLU | 85 | 23.274 | 13.755 | 1.625 | 1.00 69.55 | 6 |
| ATOM | 650 | CD | GLU | 85 | 23.680 | 13.141 | 2.934 | 1.00 80.62 | 6 |
| ATOM | 651 | OE1 | GLU | 85 | 24.093 | 13.881 | 3.861 | 1.00 88.99 | 8 |
| ATOM | 652 | OE2 | GLU | 85 | 23.602 | 11.902 | 3.059 | 1.00 80.56 | 8 |
| ATOM | 653 | C | GLU | 85 | 20.149 | 15.738 | 2.553 | 1.00 64.72 | 6 |
| ATOM | 654 | O | GLU | 85 | 20.255 | 16.946 | 2.825 | 1.00 68.35 | 8 |
| ATOM | 655 | N | ASP | 86 | 19.078 | 15.193 | 1.989 | 1.00 67.20 | 7 |
| ATOM | 656 | CA | ASP | 86 | 17.862 | 15.912 | 1.590 | 1.00 64.22 | 6 |
| ATOM | 657 | CB | ASP | 86 | 16.844 | 14.912 | 1.043 | 1.00 61.86 | 6 |
| ATOM | 658 | CG | ASP | 86 | 16.371 | 15.243 | −0.337 | 1.00 59.57 | 6 |
| ATOM | 659 | OD1 | ASP | 86 | 16.600 | 16.364 | −0.835 | 1.00 62.67 | 8 |
| ATOM | 660 | OD2 | ASP | 86 | 15.743 | 14.351 | −0.944 | 1.00 62.68 | 8 |
| ATOM | 661 | C | ASP | 86 | 17.211 | 16.638 | 2.770 | 1.00 64.44 | 6 |
| ATOM | 662 | O | ASP | 86 | 16.367 | 17.508 | 2.566 | 1.00 67.90 | 8 |
| ATOM | 663 | N | ALA | 87 | 17.639 | 16.347 | 3.993 | 1.00 61.44 | 7 |
| ATOM | 664 | CA | ALA | 87 | 16.980 | 16.912 | 5.170 | 1.00 55.63 | 6 |
| ATOM | 665 | CB | ALA | 87 | 17.736 | 16.530 | 6.423 | 1.00 51.21 | 6 |
| ATOM | 666 | C | ALA | 87 | 16.813 | 18.426 | 5.071 | 1.00 53.44 | 6 |
| ATOM | 667 | O | ALA | 87 | 17.643 | 19.129 | 4.481 | 1.00 57.76 | 8 |
| ATOM | 668 | N | LYS | 88 | 15.720 | 18.926 | 5.629 | 1.00 51.78 | 7 |
| ATOM | 669 | CA | LYS | 88 | 15.423 | 20.351 | 5.593 | 1.00 52.31 | 6 |
| ATOM | 670 | CB | LYS | 88 | 14.568 | 20.663 | 4.364 | 1.00 56.09 | 6 |
| ATOM | 671 | CG | LYS | 88 | 13.482 | 19.612 | 4.116 | 1.00 57.35 | 6 |

# Fig. 24

| | | | | | | | | | |
|---|---|---|---|---|---|---|---|---|---|
| ATOM | 672 | CD | LYS | 88 | 12.548 | 20.006 | 2.998 | 1.00 60.65 | 6 |
| ATOM | 673 | CE | LYS | 88 | 11.567 | 21.058 | 3.467 | 1.00 60.14 | 6 |
| ATOM | 674 | NZ | LYS | 88 | 12.154 | 22.435 | 3.392 | 1.00 63.67 | 7 |
| ATOM | 675 | C | LYS | 88 | 14.676 | 20.784 | 6.865 | 1.00 56.60 | 6 |
| ATOM | 676 | O | LYS | 88 | 13.738 | 21.579 | 6.796 | 1.00 61.86 | 8 |
| ATOM | 677 | N | GLY | 89 | 15.095 | 20.282 | 8.019 | 1.00 53.71 | 7 |
| ATOM | 678 | CA | GLY | 89 | 14.429 | 20.628 | 9.264 | 1.00 46.76 | 6 |
| ATOM | 679 | C | GLY | 89 | 15.051 | 21.824 | 9.967 | 1.00 40.92 | 6 |
| ATOM | 680 | O | GLY | 89 | 15.778 | 22.617 | 9.362 | 1.00 37.92 | 8 |
| ATOM | 681 | N | LYS | 90 | 14.762 | 21.968 | 11.257 | 1.00 38.87 | 7 |
| ATOM | 682 | CA | LYS | 90 | 15.310 | 23.069 | 12.032 | 1.00 31.91 | 6 |
| ATOM | 683 | CB | LYS | 90 | 14.620 | 23.134 | 13.410 | 1.00 34.15 | 6 |
| ATOM | 684 | CG | LYS | 90 | 13.169 | 23.557 | 13.385 | 1.00 21.89 | 6 |
| ATOM | 685 | CD | LYS | 90 | 13.055 | 25.043 | 13.621 | 1.00 32.49 | 6 |
| ATOM | 686 | CE | LYS | 90 | 11.837 | 25.624 | 12.923 | 1.00 39.60 | 6 |
| ATOM | 687 | NZ | LYS | 90 | 12.217 | 26.593 | 11.844 | 1.00 48.90 | 7 |
| ATOM | 688 | C | LYS | 90 | 16.816 | 22.886 | 12.223 | 1.00 29.25 | 6 |
| ATOM | 689 | O | LYS | 90 | 17.350 | 21.774 | 12.102 | 1.00 27.37 | 8 |
| ATOM | 690 | N | SER | 91 | 17.502 | 23.980 | 12.524 | 1.00 29.72 | 7 |
| ATOM | 691 | CA | SER | 91 | 18.939 | 23.916 | 12.757 | 1.00 36.36 | 6 |
| ATOM | 692 | CB | SER | 91 | 19.557 | 25.307 | 12.602 | 1.00 41.04 | 6 |
| ATOM | 693 | OG | SER | 91 | 19.002 | 26.208 | 13.547 | 1.00 38.60 | 8 |
| ATOM | 694 | C | SER | 91 | 19.195 | 23.396 | 14.175 | 1.00 36.90 | 6 |
| ATOM | 695 | O | SER | 91 | 18.260 | 23.024 | 14.877 | 1.00 42.23 | 8 |
| ATOM | 696 | N | GLU | 92 | 20.464 | 23.368 | 14.580 | 1.00 43.18 | 7 |
| ATOM | 697 | CA | GLU | 92 | 20.869 | 22.914 | 15.918 | 1.00 44.99 | 6 |
| ATOM | 698 | CB | GLU | 92 | 22.393 | 22.835 | 15.994 | 1.00 48.43 | 6 |
| ATOM | 699 | CG | GLU | 92 | 22.934 | 21.442 | 16.186 | 1.00 60.41 | 6 |
| ATOM | 700 | CD | GLU | 92 | 22.549 | 20.517 | 15.046 | 1.00 70.68 | 6 |
| ATOM | 701 | OE1 | GLU | 92 | 23.468 | 19.900 | 14.453 | 1.00 67.29 | 8 |
| ATOM | 702 | OE2 | GLU | 92 | 21.330 | 20.413 | 14.747 | 1.00 70.09 | 8 |
| ATOM | 703 | C | GLU | 92 | 20.385 | 23.891 | 16.995 | 1.00 43.15 | 6 |
| ATOM | 704 | O | GLU | 92 | 19.820 | 23.510 | 18.032 | 1.00 29.00 | 8 |
| ATOM | 705 | N | GLU | 93 | 20.637 | 25.165 | 16.733 | 1.00 43.93 | 7 |
| ATOM | 706 | CA | GLU | 93 | 20.265 | 26.219 | 17.652 | 1.00 43.74 | 6 |
| ATOM | 707 | CB | GLU | 93 | 20.605 | 27.572 | 17.009 | 1.00 44.16 | 6 |
| ATOM | 708 | CG | GLU | 93 | 20.800 | 28.722 | 17.969 | 1.00 53.18 | 6 |
| ATOM | 709 | CD | GLU | 93 | 19.682 | 29.760 | 17.870 | 1.00 66.45 | 6 |
| ATOM | 710 | OE1 | GLU | 93 | 19.889 | 30.803 | 17.203 | 1.00 73.56 | 8 |
| ATOM | 711 | OE2 | GLU | 93 | 18.596 | 29.535 | 18.460 | 1.00 69.61 | 8 |

Fig. 25

| | | | | | | | | | |
|---|---|---|---|---|---|---|---|---|---|
| ATOM | 712 | C | GLU | 93 | 18.783 | 26.134 | 18.042 | 1.00 43.54 | 6 |
| ATOM | 713 | O | GLU | 93 | 18.460 | 25.937 | 19.210 | 1.00 38.24 | 8 |
| ATOM | 714 | N | GLU | 94 | 17.891 | 26.243 | 17.062 | 1.00 39.27 | 7 |
| ATOM | 715 | CA | GLU | 94 | 16.453 | 26.213 | 17.323 | 1.00 41.79 | 6 |
| ATOM | 716 | CB | GLU | 94 | 15.661 | 26.549 | 16.052 | 1.00 43.21 | 6 |
| ATOM | 717 | CG | GLU | 94 | 16.313 | 26.060 | 14.756 | 1.00 57.16 | 6 |
| ATOM | 718 | CD | GLU | 94 | 15.865 | 26.831 | 13.500 | 1.00 54.62 | 6 |
| ATOM | 719 | OE1 | GLU | 94 | 15.119 | 27.826 | 13.627 | 1.00 48.80 | 8 |
| ATOM | 720 | OE2 | GLU | 94 | 16.264 | 26.431 | 12.380 | 1.00 49.90 | 8 |
| ATOM | 721 | C | GLU | 94 | 15.961 | 24.889 | 17.881 | 1.00 40.86 | 6 |
| ATOM | 722 | O | GLU | 94 | 15.041 | 24.862 | 18.700 | 1.00 42.31 | 8 |
| ATOM | 723 | N | LEU | 95 | 16.567 | 23.790 | 17.449 | 1.00 39.52 | 7 |
| ATOM | 724 | CA | LEU | 95 | 16.149 | 22.485 | 17.937 | 1.00 38.91 | 6 |
| ATOM | 725 | CB | LEU | 95 | 16.713 | 21.362 | 17.052 | 1.00 30.53 | 6 |
| ATOM | 726 | CG | LEU | 95 | 15.929 | 20.998 | 15.773 | 1.00 34.12 | 6 |
| ATOM | 727 | CD1 | LEU | 95 | 16.394 | 19.649 | 15.272 | 1.00 24.73 | 6 |
| ATOM | 728 | CD2 | LEU | 95 | 14.422 | 20.951 | 16.027 | 1.00 20.89 | 6 |
| ATOM | 729 | C | LEU | 95 | 16.565 | 22.278 | 19.394 | 1.00 38.76 | 6 |
| ATOM | 730 | O | LEU | 95 | 15.892 | 21.565 | 20.136 | 1.00 40.22 | 8 |
| ATOM | 731 | N | ALA | 96 | 17.653 | 22.919 | 19.811 | 1.00 40.52 | 7 |
| ATOM | 732 | CA | ALA | 96 | 18.124 | 22.784 | 21.192 | 1.00 43.50 | 6 |
| ATOM | 733 | CB | ALA | 96 | 19.550 | 23.297 | 21.327 | 1.00 35.80 | 6 |
| ATOM | 734 | C | ALA | 96 | 17.208 | 23.550 | 22.143 | 1.00 43.87 | 6 |
| ATOM | 735 | O | ALA | 96 | 17.067 | 23.177 | 23.308 | 1.00 45.55 | 8 |
| ATOM | 736 | N | GLU | 97 | 16.587 | 24.615 | 21.636 | 1.00 42.21 | 7 |
| ATOM | 737 | CA | GLU | 97 | 15.679 | 25.431 | 22.433 | 1.00 44.60 | 6 |
| ATOM | 738 | CB | GLU | 97 | 15.409 | 26.772 | 21.738 | 1.00 43.91 | 6 |
| ATOM | 739 | CG | GLU | 97 | 16.532 | 27.794 | 21.896 | 1.00 53.30 | 6 |
| ATOM | 740 | CD | GLU | 97 | 16.307 | 28.768 | 23.051 | 1.00 62.31 | 6 |
| ATOM | 741 | OE1 | GLU | 97 | 16.146 | 28.309 | 24.202 | 1.00 63.27 | 8 |
| ATOM | 742 | OE2 | GLU | 97 | 16.298 | 29.999 | 22.808 | 1.00 67.47 | 8 |
| ATOM | 743 | C | GLU | 97 | 14.365 | 24.681 | 22.664 | 1.00 45.23 | 6 |
| ATOM | 744 | O | GLU | 97 | 13.682 | 24.883 | 23.666 | 1.00 45.95 | 8 |
| ATOM | 745 | N | CYS | 98 | 14.015 | 23.805 | 21.734 | 1.00 44.76 | 7 |
| ATOM | 746 | CA | CYS | 98 | 12.793 | 23.031 | 21.875 | 1.00 35.50 | 6 |
| ATOM | 747 | CB | CYS | 98 | 12.474 | 22.286 | 20.582 | 1.00 39.56 | 6 |
| ATOM | 748 | SG | CYS | 98 | 11.822 | 23.341 | 19.264 | 1.00 28.18 | 16 |
| ATOM | 749 | C | CYS | 98 | 13.095 | 22.029 | 22.955 | 1.00 33.88 | 6 |
| ATOM | 750 | O | CYS | 98 | 12.250 | 21.736 | 23.808 | 1.00 30.81 | 8 |
| ATOM | 751 | N | PHE | 99 | 14.326 | 21.520 | 22.897 | 1.00 30.93 | 7 |

# EP 1 043 334 A1

Fig. 26

| ATOM | 752 | CA | PHE | 99 | 14.808 | 20.529 | 23.833 | 1.00 | 30.85 | 6 |
| ATOM | 753 | CB | PHE | 99 | 16.258 | 20.161 | 23.506 | 1.00 | 21.03 | 6 |
| ATOM | 754 | CG | PHE | 99 | 16.757 | 18.944 | 24.263 | 1.00 | 23.95 | 6 |
| ATOM | 755 | CD1 | PHE | 99 | 16.326 | 17.675 | 23.922 | 1.00 | 17.08 | 6 |
| ATOM | 756 | CD2 | PHE | 99 | 17.634 | 19.076 | 25.333 | 1.00 | 16.35 | 6 |
| ATOM | 757 | CE1 | PHE | 99 | 16.754 | 16.560 | 24.634 | 1.00 | 27.25 | 6 |
| ATOM | 758 | CE2 | PHE | 99 | 18.071 | 17.967 | 26.050 | 1.00 | 12.19 | 6 |
| ATOM | 759 | CZ | PHE | 99 | 17.629 | 16.707 | 25.699 | 1.00 | 18.40 | 6 |
| ATOM | 760 | C | PHE | 99 | 14.687 | 21.016 | 25.282 | 1.00 | 34.14 | 6 |
| ATOM | 761 | O | PHE | 99 | 14.086 | 20.339 | 26.123 | 1.00 | 37.78 | 8 |
| ATOM | 762 | N | ARG | 100 | 15..250 | 22.186 | 25.574 | 1.00 | 34.80 | 7 |
| ATOM | 763 | CA | ARG | 100 | 15.178 | 22.736 | 26.922 | 1.00 | 32.27 | 6 |
| ATOM | 764 | CB | ARG | 100 | 16.002 | 24.019 | 27.062 | 1.00 | 28.45 | 6 |
| ATOM | 765 | CG | ARG | 100 | 16.045 | 24.887 | 25.834 | 1.00 | 41.60 | 6 |
| ATOM | 766 | CD | ARG | 100 | 15.921 | 26.339 | 26.198 | 1.00 | 39.98 | 6 |
| ATOM | 767 | NE | ARG | 100 | 14.677 | 26.607 | 26.900 | 1.00 | 47.71 | 7 |
| ATOM | 768 | CZ | ARG | 100 | 14.064 | 27.786 | 26.900 | 1.00 | 49.96 | 6 |
| ATOM | 769 | NH1 | ARG | 100 | 14.583 | 28.810 | 26.232 | 1.00 | 45.43 | 7 |
| ATOM | 770 | NH2 | ARG | 100 | 12.936 | 27.943 | 27.581 | 1.00 | 53.56 | 7 |
| ATOM | 771 | C | ARG | 100 | 13.743 | 23.027 | 27.294 | 1.00 | 35.09 | 6 |
| ATOM | 772 | O | ARG | 100 | 13.416 | 23.138 | 28.468 | 1.00 | 49.57 | 8 |
| ATOM | 773 | N | ILE | 101 | 12.869 | 23.163 | 26.312 | 1.00 | 28.83 | 7 |
| ATOM | 774 | CA | ILE | 101 | 11.486 | 23.413 | 26.653 | 1.00 | 30.53 | 6 |
| ATOM | 775 | CB | ILE | 101 | 10.697 | 23.884 | 25.445 | 1.00 | 28.38 | 6 |
| ATOM | 776 | CG2 | ILE | 101 | 9.216 | 23.719 | 25.695 | 1.00 | 21.65 | 6 |
| ATOM | 777 | CG1 | ILE | 101 | 10.995 | 25.354 | 25.192 | 1.00 | 24.23 | 6 |
| ATOM | 778 | CD1 | ILE | 101 | 10.306 | 26.270 | 26.154 | 1.00 | 25.40 | 6 |
| ATOM | 779 | C | ILE | 101 | 10.925 | 22.083 | 27.125 | 1.00 | 31.83 | 6 |
| ATOM | 780 | O | ILE | 101 | 10.105 | 22.013 | 28.051 | 1.00 | 31.18 | 8 |
| ATOM | 781 | N | PHE | 102 | 11.413 | 21.036 | 26.470 | 1.00 | 31.69 | 7 |
| ATOM | 782 | CA | PHE | 102 | 11.017 | 19.658 | 26.719 | 1.00 | 30.68 | 6 |
| ATOM | 783 | CB | PHE | 102 | 11.369 | 18.792 | 25.506 | 1.00 | 30.23 | 6 |
| ATOM | 784 | CG | PHE | 102 | 10.550 | 19.104 | 24.277 | 1.00 | 41.60 | 6 |
| ATOM | 785 | CD1 | PHE | 102 | 9.345 | 19.807 | 24.367 | 1.00 | 35.46 | 6 |
| ATOM | 786 | CD2 | PHE | 102 | 10.989 | 18.685 | 23.016 | 1.00 | 36.01 | 6 |
| ATOM | 787 | CE1 | PHE | 102 | 8.598 | 20.077 | 23.222 | 1.00 | 28.05 | 6 |
| ATOM | 788 | CE2 | PHE | 102 | 10.241 | 18.953 | 21.869 | 1.00 | 15.30 | 6 |
| ATOM | 789 | CZ | PHE | 102 | 9.066 | 19.648 | 21.971 | 1.00 | 18.81 | 6 |
| ATOM | 790 | C | PHE | 102 | 11.650 | 19.025 | 27.944 | 1.00 | 28.30 | 6 |
| ATOM | 791 | O | PHE | 102 | 11.078 | 18.107 | 28.507 | 1.00 | 39.00 | 8 |

45

Fig. 27

| ATOM | 792 | N | ASP | 103 | 12.837 | 19.469 | 28.336 | 1.00 | 25.39 | 7 |
|------|-----|-----|------|-----|--------|--------|--------|------|-------|---|
| ATOM | 793 | CA | ASP | 103 | 13.498 | 18.906 | 29.619 | 1.00 | 27.13 | 6 |
| ATOM | 794 | CB | ASP | 103 | 15.010 | 18.961 | 29.343 | 1.00 | 26.76 | 6 |
| ATOM | 795 | CG | ASP | 103 | 15.764 | 18.120 | 30.361 | 1.00 | 27.61 | 6 |
| ATOM | 796 | OD1 | ASP | 103 | 15.124 | 17.283 | 31.099 | 1.00 | 31.43 | 8 |
| ATOM | 797 | OD2 | ASP | 103 | 17.039 | 18.253 | 30.474 | 1.00 | 24.90 | 8 |
| ATOM | 798 | C | ASP | 103 | 13.106 | 19.764 | 30.738 | 1.00 | 33.34 | 6 |
| ATOM | 799 | O | ASP | 103 | 13.722 | 20.805 | 31.005 | 1.00 | 27.83 | 8 |
| ATOM | 800 | N | ARG | 104 | 12.078 | 19.315 | 31.458 | 1.00 | 35.38 | 7 |
| ATOM | 801 | CA | ARG | 104 | 11.546 | 20.065 | 32.624 | 1.00 | 36.62 | 6 |
| ATOM | 802 | CB | ARG | 104 | 10.215 | 19.465 | 33.161 | 1.00 | 40.72 | 6 |
| ATOM | 803 | CG | ARG | 104 | 9.053 | 19.358 | 32.134 | 1.00 | 49.58 | 6 |
| ATOM | 804 | CD | ARG | 104 | 8.129 | 20.595 | 32.050 | 1.00 | 44.22 | 6 |
| ATOM | 805 | NE | ARG | 104 | 7.677 | 20.695 | 30.706 | 1.00 | 50.25 | 7 |
| ATOM | 806 | CZ | ARG | 104 | 7.440 | 21.708 | 29.908 | 1.00 | 49.35 | 6 |
| ATOM | 807 | NH1 | ARG | 104 | 7.537 | 22.993 | 30.232 | 1.00 | 49.35 | 7 |
| ATOM | 808 | NH2 | ARG | 104 | 7.088 | 21.427 | 28.679 | 1.00 | 49.35 | 7 |
| ATOM | 809 | C | ARG | 104 | 12.551 | 20.090 | 33.808 | 1.00 | 36.80 | 6 |
| ATOM | 810 | O | ARG | 104 | 12.803 | 21.142 | 34.412 | 1.00 | 41.42 | 8 |
| ATOM | 811 | N | ASN | 105 | 13.120 | 18.929 | 34.134 | 1.00 | 34.07 | 7 |
| ATOM | 812 | CA | ASN | 105 | 14.058 | 18.801 | 35.285 | 1.00 | 36.24 | 6 |
| ATOM | 813 | CB | ASN | 105 | 14.065 | 17.375 | 35.822 | 1.00 | 35.10 | 6 |
| ATOM | 814 | CG | ASN | 105 | 14.181 | 16.327 | 34.725 | 1.00 | 30.75 | 6 |
| ATOM | 815 | OD1 | ASN | 105 | 14.634 | 16.638 | 33.625 | 1.00 | 42.71 | 8 |
| ATOM | 816 | ND2 | ASN | 105 | 13.792 | 15.092 | 34.963 | 1.00 | 39.02 | 7 |
| ATOM | 817 | C | ASN | 105 | 15.496 | 19.173 | 34.909 | 1.00 | 33.69 | 6 |
| ATOM | 818 | O | ASN | 105 | 16.362 | 19.275 | 35.775 | 1.00 | 41.69 | 8 |
| ATOM | 819 | N | ALA | 106 | 15.743 | 19.342 | 33.617 | 1.00 | 30.96 | 7 |
| ATOM | 820 | CA | ALA | 106 | 17.059 | 19.694 | 33.091 | 1.00 | 27.45 | 6 |
| ATOM | 821 | CB | ALA | 106 | 17.507 | 21.066 | 33.607 | 1.00 | 18.38 | 6 |
| ATOM | 822 | C | ALA | 106 | 18.129 | 18.668 | 33.385 | 1.00 | 23.27 | 6 |
| ATOM | 823 | O | ALA | 106 | 19.204 | 19.031 | 33.829 | 1.00 | 35.79 | 8 |
| ATOM | 824 | N | ASP | 107 | 17.851 | 17.392 | 33.131 | 1.00 | 27.00 | 7 |
| ATOM | 825 | CA | ASP | 107 | 18.847 | 16.338 | 33.369 | 1.00 | 31.66 | 6 |
| ATOM | 826 | CB | ASP | 107 | 18.167 | 15.073 | 33.913 | 1.00 | 29.25 | 6 |
| ATOM | 827 | CG | ASP | 107 | 16.845 | 14.784 | 33.241 | 1.00 | 26.66 | 6 |
| ATOM | 828 | OD1 | ASP | 107 | 16.187 | 13.810 | 33.657 | 1.00 | 23.76 | 8 |
| ATOM | 829 | OD2 | ASP | 107 | 16.461 | 15.519 | 32.296 | 1.00 | 30.73 | 8 |
| ATOM | 830 | C | ASP | 107 | 19.673 | 15.982 | 32.119 | 1.00 | 32.92 | 6 |
| ATOM | 831 | O | ASP | 107 | 20.612 | 15.181 | 32.194 | 1.00 | 36.32 | 8 |

Fig. 2 8

| | | | | | | | | | |
|---|---|---|---|---|---|---|---|---|---|
| ATOM | 832 | N | GLY | 108 | 19. 341 | 16. 603 | 30. 986 | 1. 00 35. 26 | 7 |
| ATOM | 833 | CA | GLY | 108 | 20. 043 | 16. 323 | 29. 742 | 1. 00 28. 31 | 6 |
| ATOM | 834 | C | GLY | 108 | 19. 313 | 15. 275 | 28. 913 | 1. 00 29. 83 | 6 |
| ATOM | 835 | O | GLY | 108 | 19. 841 | 14. 776 | 27. 912 | 1. 00 31. 14 | 8 |
| ATOM | 836 | N | TYR | 109 | 18. 101 | 14. 935 | 29. 353 | 1. 00 29. 74 | 7 |
| ATOM | 837 | CA | TYR | 109 | 17. 249 | 13. 958 | 28. 689 | 1. 00 25. 68 | 6 |
| ATOM | 838 | CB | TYR | 109 | 17. 254 | 12. 609 | 29. 419 | 1. 00 30. 19 | 6 |
| ATOM | 839 | CG | TYR | 109 | 18. 613 | 12. 018 | 29. 694 | 1. 00 42. 80 | 6 |
| ATOM | 840 | CD1 | TYR | 109 | 19. 267 | 12. 285 | 30. 898 | 1. 00 34. 37 | 6 |
| ATOM | 841 | CE1 | TYR | 109 | 20. 519 | 11. 741 | 31. 176 | 1. 00 35. 81 | 6 |
| ATOM | 842 | CD2 | TYR | 109 | 19. 251 | 11. 179 | 28. 753 | 1. 00 44. 13 | 6 |
| ATOM | 843 | CE2 | TYR | 109 | 20. 515 | 10. 623 | 29. 024 | 1. 00 41. 85 | 6 |
| ATOM | 844 | CZ | TYR | 109 | 21. 143 | 10. 918 | 30. 245 | 1. 00 43. 19 | 6 |
| ATOM | 845 | OH | TYR | 109 | 22. 389 | 10. 410 | 30: 545 | 1. 00 38. 26 | 8 |
| ATOM | 846 | C | TYR | 109 | 15. 807 | 14. 428 | 28. 685 | 1. 00 29. 62 | 6 |
| ATOM | 847 | O | TYR | 109 | 15. 453 | 15. 387 · | 29. 364 | 1. 00 32. 27 | 8 |
| ATOM | 848 | N | ILE | 110 | 14. 994 | 13. 718 | 27. 905 | 1. 00 35. 34 | 7 |
| ATOM | 849 | CA | ILE | 110 | 13. 548 | 13. 912 | 27. 790 | 1. 00 32. 15 | 6 |
| ATOM | 850 | CB | ILE | 110 | 13. 084 | 14. 340 | 26. 376 | 1. 00 24. 00 | 6 |
| ATOM | 851 | CG2 | ILE | 110 | 11. 578 | 14. 171 | 26. 264 | 1. 00 23. 85 | 6 |
| ATOM | 852 | CG1 | ILE | 110 | 13. 407 | 15. 821 | 26. 133 | 1. 00 30. 66 | 6 |
| ATOM | 853 | CD1 | ILE | 110 | 13. 260 | 16. 282 | 24. 679 | 1. 00 24. 05 | 6 |
| ATOM | 854 | C | ILE | 110 | 13. 096 | 12. 470 | 28. 044 | 1. 00 36. 35 | 6 |
| ATOM | 855 | O | ILE | 110 | 13. 524 | 11. 547 | 27. 337 | 1. 00 36. 93 | 8 |
| ATOM | 856 | N | ASP | 111 | 12. 263 | 12. 267 | 29. 064 | 1. 00 38. 27 | 7 |
| ATOM | 857 | CA | ASP | 111 | 11. 830 | 10. 914 | 29. 392 | 1. 00 32. 84 | 6 |
| ATOM | 858 | CB | ASP | 111 | 12. 149 | 10. 584 | 30. 868 | 1. 00 46. 53 | 6 |
| ATOM | 859 | CG | ASP | 111 | 11. 610 | 11. 621 | 31. 854 | 1. 00 40. 66 | 6 |
| ATOM | 860 | OD1 | ASP | 111 | 12. 245 | 11. 809 | 32. 919 | 1. 00 37. 57 | 8 |
| ATOM | 861 | OD2 | ASP | 111 | 10. 560 | 12. 237 | 31. 570 | 1. 00 48. 84 | 8 |
| ATOM | 862 | C | ASP | 111 | 10. 366 | 10. 669 | 29. 120 | 1. 00 27. 21 | 6 |
| ATOM | 863 | O | ASP | 111 | 9. 566 | 11. 595 | 29. 108 | 1. 00 27. 30 | 8 |
| ATOM | 864 | N | ALA | 112 | 10. 029 | 9. 407 | 28. 905 | 1. 00 24. 32 | 7 |
| ATOM | 865 | CA | ALA | 112 | 8. 661 | 9. 004 | 28. 636 | 1. 00 31. 47 | 6 |
| ATOM | 866 | CB | ALA | 112 | 8. 350 | 7. 656 | 29. 262 | 1. 00 37. 97 | 6 |
| ATOM | 867 | C | ALA | 112 | 7. 623 | 9. 969 | 29. 180 | 1. 00 32. 01 | 6 |
| ATOM | 868 | O | ALA | 112 | 6. 775 | 10. 463 | 28. 443 | 1. 00 41. 75 | 8 |
| ATOM | 869 | N | GLU | 113 | 7. 708 | 10. 227 | 30. 480 | 1. 00 35. 32 | 7 |
| ATOM | 870 | CA | GLU | 113 | 6. 764 | 11. 098 | 31. 174 | 1. 00 35. 11 | 6 |
| ATOM | 871 | CB | GLU | 113 | 7. 005 | 11. 050 | 32. 702 | 1. 00 40. 71 | 6 |

Fig. 29

| ATOM | 872 | CG | GLU | 113 | 8.338 | 10.387 | 33.159 | 1.00 | 47.73 | 6 |
|------|-----|-----|-----|-----|-------|--------|--------|------|-------|---|
| ATOM | 873 | CD | GLU | 113 | 8.323 | 8.854 | 33.155 | 1.00 | 43.50 | 6 |
| ATOM | 874 | OE1 | GLU | 113 | 7.239 | 8.255 | 33.361 | 1.00 | 49.05 | 8 |
| ATOM | 875 | OE2 | GLU | 113 | 9.407 | 8.250 | 32.948 | 1.00 | 43.76 | 8 |
| ATOM | 876 | C | GLU | 113 | 6.826 | 12.525 | 30.652 | 1.00 | 31.26 | 6 |
| ATOM | 877 | O | GLU | 113 | 5.791 | 13.151 | 30.410 | 1.00 | 34.05 | 8 |
| ATOM | 878 | N | GLU | 114 | 8.033 | 13.043 | 30.473 | 1.00 | 31.07 | 7 |
| ATOM | 879 | CA | GLU | 114 | 8.196 | 14.398 | 29.938 | 1.00 | 31.50 | 6 |
| ATOM | 880 | CB | GLU | 114 | 9.704 | 14.752 | 29.877 | 1.00 | 22.20 | 6 |
| ATOM | 881 | CG | GLU | 114 | 10.234 | 15.490 | 31.132 | 1.00 | 17.32 | 6 |
| ATOM | 882 | CD | GLU | 114 | 11.748 | 15.495 | 31.246 | 1.00 | 12.27 | 6 |
| ATOM | 883 | OE1 | GLU | 114 | 12.321 | 16.359 | 31.930 | 1.00 | 12.87 | 8 |
| ATOM | 884 | OE2 | GLU | 114 | 12.382 | 14.624 | 30.647 | 1.00 | 21.03 | 8 |
| ATOM | 885 | C | GLU | 114 | 7.541 | 14.451 | 28.525 | 1.00 | 36.78 | 6 |
| ATOM | 886 | O | GLU | 114 | 6.914 | 15.444 | 28.133 | 1.00 | 27.07 | 8 |
| ATOM | 887 | N | LEU | 115 | 7.677 | 13.350 | 27.790 | 1.00 | 38.62 | 7 |
| ATOM | 888 | CA | LEU | 115 | 7.130 | 13.221 | 26.444 | 1.00 | 44.08 | 6 |
| ATOM | 889 | CB | LEU | 115 | 7.455 | 11.839 | 25.876 | 1.00 | 43.17 | 6 |
| ATOM | 890 | CG | LEU | 115 | 7.941 | 11.861 | 24.426 | 1.00 | 41.90 | 6 |
| ATOM | 891 | CD1 | LEU | 115 | 6.974 | 12.636 | 23.556 | 1.00 | 36.53 | 6 |
| ATOM | 892 | CD2 | LEU | 115 | 9.301 | 12.516 | 24.374 | 1.00 | 52.36 | 6 |
| ATOM | 893 | C | LEU | 115 | 5.627 | 13.444 | 26.377 | 1.00 | 42.69 | 6 |
| ATOM | 894 | O | LEU | 115 | 5.148 | 14.249 | 25.583 | 1.00 | 47.38 | 8 |
| ATOM | 895 | N | ALA | 116 | 4.882 | 12.703 | 27.188 | 1.00 | 39.94 | 7 |
| ATOM | 896 | CA | ALA | 116 | 3.439 | 12.856 | 27.213 | 1.00 | 35.83 | 6 |
| ATOM | 897 | CB | ALA | 116 | 2.827 | 11.767 | 28.071 | 1.00 | 36.64 | 6 |
| ATOM | 898 | C | ALA | 116 | 3.082 | 14.229 | 27.771 | 1.00 | 37.45 | 6 |
| ATOM | 899 | O | ALA | 116 | 2.005 | 14.756 | 27.518 | 1.00 | 41.21 | 8 |
| ATOM | 900 | N | GLU | 117 | 3.996 | 14.809 | 28.534 | 1.00 | 39.33 | 7 |
| ATOM | 901 | CA | GLU | 117 | 3.751 | 16.117 | 29.116 | 1.00 | 40.70 | 6 |
| ATOM | 902 | CB | GLU | 117 | 4.892 | 16.513 | 30.050 | 1.00 | 47.89 | 6 |
| ATOM | 903 | CG | GLU | 117 | 4.654 | 16.152 | 31.509 | 1.00 | 52.68 | 6 |
| ATOM | 904 | CD | GLU | 117 | 5.249 | 17.169 | 32.486 | 1.00 | 67.75 | 6 |
| ATOM | 905 | OE1 | GLU | 117 | 5.660 | 18.270 | 32.042 | 1.00 | 64.92 | 8 |
| ATOM | 906 | OE2 | GLU | 117 | 5.302 | 16.859 | 33.702 | 1.00 | 65.21 | 8 |
| ATOM | 907 | C | GLU | 117 | 3.667 | 17.124 | 27.998 | 1.00 | 41.92 | 6 |
| ATOM | 908 | O | GLU | 117 | 3.075 | 18.191 | 28.158 | 1.00 | 43.50 | 8 |
| ATOM | 909 | N | ILE | 118 | 4.281 | 16.777 | 26.867 | 1.00 | 43.12 | 7 |
| ATOM | 910 | CA | ILE | 118 | 4.308 | 17.630 | 25.677 | 1.00 | 36.76 | 6 |
| ATOM | 911 | CB | ILE | 118 | 5.289 | 17.099 | 24.627 | 1.00 | 32.28 | 6 |

## Fig. 30

| | | | | | | | | | | |
|---|---|---|---|---|---|---|---|---|---|---|
| ATOM | 912 | CG2 | ILE | 118 | 5.261 | 17.994 | 23.404 | 1.00 | 35.84 | 6 |
| ATOM | 913 | CG1 | ILE | 118 | 6.697 | 17.035 | 25.212 | 1.00 | 48.89 | 6 |
| ATOM | 914 | CD1 | ILE | 118 | 7.589 | 15.987 | 24.552 | 1.00 | 45.85 | 6 |
| ATOM | 915 | C | ILE | 118 | 2.966 | 17.767 | 24.966 | 1.00 | 34.34 | 6 |
| ATOM | 916 | O | ILE | 118 | 2.419 | 18.857 | 24.821 | 1.00 | 37.43 | 8 |
| ATOM | 917 | N | PHE | 119 | 2.427 | 16.652 | 24.512 | 1.00 | 32.52 | 7 |
| ATOM | 918 | CA | PHE | 119 | 1.181 | 16.706 | 23.777 | 1.00 | 34.29 | 6 |
| ATOM | 919 | CB | PHE | 119 | 0.976 | 15.362 | 23.092 | 1.00 | 30.99 | 6 |
| ATOM | 920 | CG | PHE | 119 | 2.174 | 14.928 | 22.314 | 1.00 | 29.47 | 6 |
| ATOM | 921 | CD1 | PHE | 119 | 3.190 | 14.206 | 22.928 | 1.00 | 29.19 | 6 |
| ATOM | 922 | CD2 | PHE | 119 | 2.328 | 15.308 | 20.979 | 1.00 | 27.38 | 6 |
| ATOM | 923 | CE1 | PHE | 119 | 4.355 | 13.859 | 22.226 | 1.00 | 30.84 | 6 |
| ATOM | 924 | CE2 | PHE | 119 | 3.480 | 14.970 | 20.267 | 1.00 | 28.50 | 6 |
| ATOM | 925 | CZ | PHE | 119 | 4.502 | 14.242 | 20.896 | 1.00 | 34.35 | 6 |
| ATOM | 926 | C | PHE | 119 | -0.031 | 17.145 | 24.583 | 1.00 | 29.46 | 6 |
| ATOM | 927 | O | PHE | 119 | -1.142 | 17.252 | 24.063 | 1.00 | 37.79 | 8 |
| ATOM | 928 | N | ARG | 120 | 0.186 | 17.440 | 25.850 | 1.00 | 22.67 | 7 |
| ATOM | 929 | CA | ARG | 120 | -0.915 | 17.882 | 26.670 | 1.00 | 30.18 | 6 |
| ATOM | 930 | CB | ARG | 120 | -0.508 | 17.903 | 28.141 | 1.00 | 41.22 | 6 |
| ATOM | 931 | CG | ARG | 120 | -0.127 | 16.536 | 28.696 | 1.00 | 46.40 | 6 |
| ATOM | 932 | CD | ARG | 120 | -0.774 | 16.327 | 30.038 | 1.00 | 46.05 | 6 |
| ATOM | 933 | NE | ARG | 120 | -0.359 | 15.072 | 30.642 | 1.00 | 48.64 | 7 |
| ATOM | 934 | CZ | ARG | 120 | 0.531 | 14.984 | 31.623 | 1.00 | 47.62 | 6 |
| ATOM | 935 | NH1 | ARG | 120 | 1.103 | 16.083 | 32.109 | 1.00 | 41.59 | 7 |
| ATOM | 936 | NH2 | ARG | 120 | 0.830 | 13.796 | 32.133 | 1.00 | 50.44 | 7 |
| ATOM | 937 | C | ARG | 120 | -1.286 | 19.279 | 26.222 | 1.00 | 32.45 | 6 |
| ATOM | 938 | O | ARG | 120 | -2.463 | 19.632 | 26.193 | 1.00 | 41.75 | 8 |
| ATOM | 939 | N | ALA | 121 | -0.274 | 20.067 | 25.864 | 1.00 | 32.93 | 7 |
| ATOM | 940 | CA | ALA | 121 | -0.475 | 21.438 | 25.404 | 1.00 | 25.56 | 6 |
| ATOM | 941 | CB | ALA | 121 | 0.833 | 22.131 | 25.273 | 1.00 | 16.55 | 6 |
| ATOM | 942 | C | ALA | 121 | -1.154 | 21.439 | 24.058 | 1.00 | 28.02 | 6 |
| ATOM | 943 | O | ALA | 121 | -1.604 | 22.473 | 23.591 | 1.00 | 35.43 | 8 |
| ATOM | 944 | N | SER | 122 | -1.199 | 20.276 | 23.425 | 1.00 | 34.74 | 7 |
| ATOM | 945 | CA | SER | 122 | -1.814 | 20.145 | 22.107 | 1.00 | 38.77 | 6 |
| ATOM | 946 | CB | SER | 122 | -1.010 | 19.171 | 21.250 | 1.00 | 32.89 | 6 |
| ATOM | 947 | OG | SER | 122 | -1.504 | 17.850 | 21.395 | 1.00 | 33.91 | 8 |
| ATOM | 948 | C | SER | 122 | -3.211 | 19.599 | 22.273 | 1.00 | 41.00 | 6 |
| ATOM | 949 | O | SER | 122 | -3.488 | 18.909 | 23.247 | 1.00 | 48.83 | 8 |
| ATOM | 950 | N | GLY | 123 | -4.087 | 19.889 | 21.319 | 1.00 | 43.52 | 7 |
| ATOM | 951 | CA | GLY | 123 | -5.452 | 19.387 | 21.409 | 1.00 | 36.41 | 6 |

F i g.  3 1

| ATOM | 952 | C | GLY | 123 | -5.601 | 17.972 | 20.877 | 1.00 | 31.67 | 6 |
|------|-----|------|-----|-----|--------|--------|--------|------|-------|---|
| ATOM | 953 | O | GLY | 123 | -6.694 | 17.602 | 20.579 | 1.00 | 27.84 | 8 |
| ATOM | 954 | N | GLU | 124 | -4.496 | 17.262 | 20.785 | 1.00 | 33.94 | 7 |
| ATOM | 955 | CA | GLU | 124 | -4.554 | 15.921 | 20.252 | 1.00 | 41.03 | 6 |
| ATOM | 956 | CB | GLU | 124 | -3.415 | 15.758 | 19.265 | 1.00 | 47.34 | 6 |
| ATOM | 957 | CG | GLU | 124 | -3.320 | 16.949 | 18.312 | 1.00 | 53.46 | 6 |
| ATOM | 958 | CD | GLU | 124 | -2.344 | 16.724 | 17.191 | 1.00 | 42.81 | 6 |
| ATOM | 959 | OE1 | GLU | 124 | -1.849 | 17.729 | 16.646 | 1.00 | 52.58 | 8 |
| ATOM | 960 | OE2 | GLU | 124 | -2.071 | 15.552 | 16.868 | 1.00 | 39.44 | 8 |
| ATOM | 961 | C | GLU | 124 | -4.536 | 14.799 | 21.279 | 1.00 | 43.39 | 6 |
| ATOM | 962 | O | GLU | 124 | -4.212 | 15.006 | 22.452 | 1.00 | 51.18 | 8 |
| ATOM | 963 | N | HIS | 125 | -4.904 | 13.607 | 20.831 | 1.00 | 36.13 | 7 |
| ATOM | 964 | CA | HIS | 125 | -4.919 | 12.467 | 21.705 | 1.00 | 38.90 | 6 |
| ATOM | 965 | CB | HIS | 125 | -6.311 | 11.855 | 21.705 | 1.00 | 44.47 | 6 |
| ATOM | 966 | CG | HIS | 125 | -6.421 | 10.608 | 22.510 | 1.00 | 44.87 | 6 |
| ATOM | 967 | CD2 | HIS | 125 | -5.558 | 10.032 | 23.380 | 1.00 | 42.40 | 6 |
| ATOM | 968 | ND1 | HIS | 125 | -7.486 | 9.740 | 22.395 | 1.00 | 45.94 | 7 |
| ATOM | 969 | CE1 | HIS | 125 | -7.271 | 8.676 | 23.150 | 1.00 | 48.01 | 6 |
| ATOM | 970 | NE2 | HIS | 125 | -6.106 | 8.830 | 23.758 | 1.00 | 53.73 | 7 |
| ATOM | 971 | C | HIS | 125 | -3.856 | 11.461 | 21.268 | 1.00 | 41.68 | 6 |
| ATOM | 972 | O | HIS | 125 | -4.007 | 10.746 | 20.278 | 1.00 | 48.87 | 8 |
| ATOM | 973 | N | VAL | 126 | -2.777 | 11.427 | 22.042 | 1.00 | 42.69 | 7 |
| ATOM | 974 | CA | VAL | 126 | -1.623 | 10.576 | 21.799 | 1.00 | 39.70 | 6 |
| ATOM | 975 | CB | VAL | 126 | -0.310 | 11.300 | 22.195 | 1.00 | 32.39 | 6 |
| ATOM | 976 | CG1 | VAL | 126 | 0.882 | 10.642 | 21.540 | 1.00 | 41.79 | 6 |
| ATOM | 977 | CG2 | VAL | 126 | -0.382 | 12.761 | 21.804 | 1.00 | 40.69 | 6 |
| ATOM | 978 | C | VAL | 126 | -1.718 | 9.325 | 22.636 | 1.00 | 39.86 | 6 |
| ATOM | 979 | O | VAL | 126 | -1.601 | 9.376 | 23.852 | 1.00 | 50.50 | 8 |
| ATOM | 980 | N | THR | 127 | -1.907 | 8.191 | 21.990 | 1.00 | 46.62 | 7 |
| ATOM | 981 | CA | THR | 127 | -2.008 | 6.944 | 22.724 | 1.00 | 47.05 | 6 |
| ATOM | 982 | CB | THR | 127 | -2.371 | 5.790 | 21.788 | 1.00 | 45.26 | 6 |
| ATOM | 983 | OG1 | THR | 127 | -2.785 | 4.655 | 22.562 | 1.00 | 57.23 | 8 |
| ATOM | 984 | CG2 | THR | 127 | -1.179 | 5.420 | 20.933 | 1.00 | 36.79 | 6 |
| ATOM | 985 | C | THR | 127 | -0.701 | 6.609 | 23.447 | 1.00 | 49.74 | 6 |
| ATOM | 986 | O | THR | 127 | 0.377 | 7.091 | 23.091 | 1.00 | 45.79 | 8 |
| ATOM | 987 | N | ASP | 128 | -0.819 | 5.770 | 24.470 | 1.00 | 55.48 | 7 |
| ATOM | 988 | CA | ASP | 128 | 0.323 | 6.337 | 25.271 | 1.00 | 55.11 | 6 |
| ATOM | 989 | CB | ASP | 128 | -0.137 | 4.298 | 26.303 | 1.00 | 63.72 | 6 |
| ATOM | 990 | CG | ASP | 128 | 0.608 | 4.412 | 27.620 | 1.00 | 67.85 | 6 |
| ATOM | 991 | OD1 | ASP | 128 | 0.750 | 3.373 | 28.299 | 1.00 | 69.59 | 8 |

Fig. 32

| | | | | | | | | | |
|------|------|-----|-----|-----|--------|--------|--------|------|--------|---|
| ATOM | 992 | OD2 | ASP | 128 | 1.049 | 5.530 | 27.969 | 1.00 | 69.90 | 8 |
| ATOM | 993 | C | ASP | 128 | 1.391 | 4.738 | 24.366 | 1.00 | 52.75 | 6 |
| ATOM | 994 | O | ASP | 128 | 2.588 | 4.987 | 24.545 | 1.00 | 45.27 | 8 |
| ATOM | 995 | N | GLU | 129 | 0.945 | 3.938 | 23.400 | 1.00 | 50.43 | 7 |
| ATOM | 996 | CA | GLU | 129 | 1.858 | 3.327 | 22.454 | 1.00 | 45.79 | 6 |
| ATOM | 997 | CB | GLU | 129 | 1.135 | 2.328 | 21.563 | 1.00 | 42.08 | 6 |
| ATOM | 998 | CG | GLU | 129 | 1.713 | 0.943 | 21.698 | 1.00 | 59.08 | 6 |
| ATOM | 999 | CD | GLU | 129 | 2.422 | 0.751 | 23.038 | 1.00 | 73.25 | 6 |
| ATOM | 1000 | OE1 | GLU | 129 | 3.645 | 1.019 | 23.116 | 1.00 | 74.10 | 8 |
| ATOM | 1001 | OE2 | GLU | 129 | 1.753 | 0.339 | 24.013 | 1.00 | 75.33 | 8 |
| ATOM | 1002 | C | GLU | 129 | 2.507 | 4.389 | 21.595 | 1.00 | 38.60 | 6 |
| ATOM | 1003 | O | GLU | 129 | 3.696 | 4.301 | 21.299 | 1.00 | 34.39 | 8 |
| ATOM | 1004 | N | GLU | 130 | 1.732 | 5.402 | 21.215 | 1.00 | 30.48 | 7 |
| ATOM | 1005 | CA | GLU | 130 | 2.279 | 6.459 | 20.384 | 1.00 | 32.18 | 6 |
| ATOM | 1006 | CB | GLU | 130 | 1.253 | 7.567 | 20.171 | 1.00 | 31.33 | 6 |
| ATOM | 1007 | CG | GLU | 130 | 0.571 | 7.497 | 18.815 | 1.00 | 33.79 | 6 |
| ATOM | 1008 | CD | GLU | 130 | -0.747 | 8.232 | 18.777 | 1.00 | 34.32 | 6 |
| ATOM | 1009 | OE1 | GLU | 130 | -0.737 | 9.480 | 18.722 | 1.00 | 40.45 | 8 |
| ATOM | 1010 | OE2 | GLU | 130 | -1.795 | 7.564 | 18.795 | 1.00 | 44.91 | 8 |
| ATOM | 1011 | C | GLU | 130 | 3.492 | 6.991 | 21.104 | 1.00 | 36.03 | 6 |
| ATOM | 1012 | O | GLU | 130 | 4.612 | 6.936 | 20.591 | 1.00 | 35.03 | 8 |
| ATOM | 1013 | N | ILE | 131 | 3.273 | 7.490 | 22.312 | 1.00 | 43.38 | 7 |
| ATOM | 1014 | CA | ILE | 131 | 4.362 | 8.009 | 23.136 | 1.00 | 46.70 | 6 |
| ATOM | 1015 | CB | ILE | 131 | 3.864 | 8.273 | 24.596 | 1.00 | 45.39 | 6 |
| ATOM | 1016 | CG2 | ILE | 131 | 5.030 | 8.672 | 25.506 | 1.00 | 42.91 | 6 |
| ATOM | 1017 | CG1 | ILE | 131 | 2.767 | 9.350 | 24.585 | 1.00 | 44.20 | 6 |
| ATOM | 1018 | CD1 | ILE | 131 | 3.148 | 10.646 | 23.881 | 1.00 | 28.00 | 6 |
| ATOM | 1019 | C | ILE | 131 | 5.536 | 7.006 | 23.161 | 1.00 | 51.61 | 6 |
| ATOM | 1020 | O | ILE | 131 | 6.704 | 7.377 | 22.984 | 1.00 | 46.11 | 8 |
| ATOM | 1021 | N | GLU | 132 | 5.207 | 5.731 | 23.354 | 1.00 | 52.91 | 7 |
| ATOM | 1022 | CA | GLU | 132 | 6.213 | 4.681 | 23.418 | 1.00 | 55.39 | 6 |
| ATOM | 1023 | CB | GLU | 132 | 5.555 | 3.375 | 23.876 | 1.00 | 63.20 | 6 |
| ATOM | 1024 | CG | GLU | 132 | 6.047 | 2.887 | 25.236 | 1.00 | 80.79 | 6 |
| ATOM | 1025 | CD | GLU | 132 | 4.962 | 2.888 | 26.304 | 1.00 | 86.29 | 6 |
| ATOM | 1026 | OE1 | GLU | 132 | 4.100 | 1.981 | 26.279 | 1.00 | 85.22 | 8 |
| ATOM | 1027 | OE2 | GLU | 132 | 4.977 | 3.794 | 27.170 | 1.00 | 89.12 | 8 |
| ATOM | 1028 | C | GLU | 132 | 6.982 | 4.448 | 22.114 | 1.00 | 54.60 | 6 |
| ATOM | 1029 | O | GLU | 132 | 8.160 | 4.082 | 22.139 | 1.00 | 50.93 | 8 |
| ATOM | 1030 | N | SER | 133 | 6.328 | 4.680 | 20.977 | 1.00 | 53.38 | 7 |
| ATOM | 1031 | CA | SER | 133 | 6.961 | 4.473 | 19.666 | 1.00 | 45.38 | 6 |

Fig. 33

| | | | | | | | | | |
|---|---|---|---|---|---|---|---|---|---|
| ATOM | 1032 | CB | SER | 133 | 5.882 | 4.254 | 18.604 | 1.00 49.08 | 6 |
| ATOM | 1033 | OG | SER | 133 | 5.010 | 3.202 | 19.000 | 1.00 47.57 | 8 |
| ATOM | 1034 | C | SER | 133 | 7.854 | 5.644 | 19.281 | 1.00 38.61 | 6 |
| ATOM | 1035 | O | SER | 133 | 8.889 | 5.482 | 18.628 | 1.00 32.84 | 8 |
| ATOM | 1036 | N | LEU | 134 | 7.436 | 6.823 | 19.720 | 1.00 34.05 | 7 |
| ATOM | 1037 | CA | LEU | 134 | 8.173 | 8.048 | 19.475 | 1.00 33.13 | 6 |
| ATOM | 1038 | CB | LEU | 134 | 7.380 | 9.234 | 20.042 | 1.00 27.84 | 6 |
| ATOM | 1039 | CG | LEU | 134 | 6.532 | 10.017 | 19.046 | 1.00 25.84 | 6 |
| ATOM | 1040 | CD1 | LEU | 134 | 5.426 | 10.798 | 19.745 | 1.00 25.61 | 6 |
| ATOM | 1041 | CD2 | LEU | 134 | 7.452 | 10.948 | 18.294 | 1.00 30.42 | 6 |
| ATOM | 1042 | C | LEU | 134 | 9.503 | 7.918 | 20.210 | 1.00 35.39 | 6 |
| ATOM | 1043 | O | LEU | 134 | 10.565 | 8.270 | 19.684 | 1.00 33.82 | 8 |
| ATOM | 1044 | N | MET | 135 | 9.424 | 7.422 | 21.446 | 1.00 40.88 | 7 |
| ATOM | 1045 | CA | MET | 135 | 10.608 | 7.242 | 22.263 | 1.00 39.49 | 6 |
| ATOM | 1046 | CB | MET | 135 | 10.215 | 6.979 | 23.721 | 1.00 47.45 | 6 |
| ATOM | 1047 | CG | MET | 135 | 10.417 | 8.174 | 24.665 | 1.00 45.35 | 6 |
| ATOM | 1048 | SD | MET | 135 | 12.094 | 8.298 | 25.344 | 1.00 47.97 | 16 |
| ATOM | 1049 | CE | MET | 135 | 12.140 | 9.999 | 25.749 | 1.00 50.83 | 6 |
| ATOM | 1050 | C | MET | 135 | 11.404 | 6.067 | 21.717 | 1.00 49.43 | 6 |
| ATOM | 1051 | O | MET | 135 | 12.636 | 6.092 | 21.717 | 1.00 48.39 | 8 |
| ATOM | 1052 | N | LYS | 136 | 10.695 | 5.044 | 21.242 | 1.00 50.80 | 7 |
| ATOM | 1053 | CA | LYS | 136 | 11.348 | 3.854 | 20.699 | 1.00 56.89 | 6 |
| ATOM | 1054 | CB | LYS | 136 | 10.301 | 2.828 | 20.251 | 1.00 60.49 | 6 |
| ATOM | 1055 | CG | LYS | 136 | 10.845 | 1.392 | 20.125 | 1.00 70.61 | 6 |
| ATOM | 1056 | CD | LYS | 136 | 11.708 | 0.960 | 21.335 | 1.00 74.32 | 6 |
| ATOM | 1057 | CE | LYS | 136 | 11.880 | -0.570 | 21.411 | 1.00 67.03 | 6 |
| ATOM | 1058 | NZ | LYS | 136 | 13.141 | -1.005 | 22.104 | 1.00 62.78 | 7 |
| ATOM | 1059 | C | LYS | 136 | 12.288 | 4.168 | 19.538 | 1.00 60.12 | 6 |
| ATOM | 1060 | O | LYS | 136 | 13.465 | 3.790 | 19.558 | 1.00 60.70 | 8 |
| ATOM | 1061 | N | ASP | 137 | 11.773 | 4.843 | 18.517 | 1.00 61.87 | 7 |
| ATOM | 1062 | CA | ASP | 137 | 12.612 | 5.188 | 17.378 | 1.00 59.99 | 6 |
| ATOM | 1063 | CB | ASP | 137 | 11.737 | 5.649 | 16.181 | 1.00 66.88 | 6 |
| ATOM | 1064 | CG | ASP | 137 | 11.463 | 7.156 | 16.153 | 1.00 70.13 | 6 |
| ATOM | 1065 | OD1 | ASP | 137 | 11.819 | 7.882 | 17.107 | 1.00 71.42 | 8 |
| ATOM | 1066 | OD2 | ASP | 137 | 10.871 | 7.616 | 15.148 | 1.00 62.67 | 8 |
| ATOM | 1067 | C | ASP | 137 | 13.650 | 6.228 | 17.784 | 1.00 55.37 | 6 |
| ATOM | 1068 | O | ASP | 137 | 14.788 | 6.203 | 17.311 | 1.00 46.31 | 8 |
| ATOM | 1069 | N | GLY | 138 | 13.256 | 7.121 | 18.689 | 1.00 54.82 | 7 |
| ATOM | 1070 | CA | GLY | 138 | 14.162 | 8.150 | 19.162 | 1.00 58.10 | 6 |
| ATOM | 1071 | C | GLY | 138 | 15.279 | 7.609 | 20.043 | 1.00 62.56 | 6 |

Fig. 34

| | | | | | | | | | | |
|------|------|-----|-----|-----|---------|--------|---------|------|-------|---|
| ATOM | 1072 | O | GLY | 138 | 16. 395 | 8. 145 | 20. 033 | 1. 00 | 61. 01 | 8 |
| ATOM | 1073 | N | ASP | 139 | 14. 975 | 6. 549 | 20. 798 | 1. 00 | 64. 21 | 7 |
| ATOM | 1074 | CA | ASP | 139 | 15. 926 | 5. 895 | 21. 703 | 1. 00 | 61. 79 | 6 |
| ATOM | 1075 | CB | ASP | 139 | 15. 161 | 5. 041 | 22. 719 | 1. 00 | 59. 14 | 6 |
| ATOM | 1076 | CG | ASP | 139 | 15. 937 | 4. 821 | 24. 008 | 1. 00 | 60. 04 | 6 |
| ATOM | 1077 | OD1 | ASP | 139 | 16. 872 | 5. 609 | 24. 302 | 1. 00 | 37. 09 | 8 |
| ATOM | 1078 | OD2 | ASP | 139 | 15. 598 | 3. 860 | 24. 725 | 1. 00 | 61. 29 | 8 |
| ATOM | 1079 | C | ASP | 139 | ·16. 895 | 5. 009 | 20. 912 | 1. 00 | 59. 85 | 6 |
| ATOM | 1080 | O | ASP | 139 | 16. 491 | 4. 007 | 20. 324 | 1. 00 | 61. 78 | 8 |
| ATOM | 1081 | N | LYS | 140 | 18. 175 | 5. 366 | 20. 909 | 1. 00 | 62. 34 | 7 |
| ATOM | 1082 | CA | LYS | 140 | 19. 152 | 4. 598 | 20. 149 | 1. 00 | 64. 93 | 6 |
| ATOM | 1083 | CB | LYS | 140 | 19. 839 | 5. 517 | 19. 134 | 1. 00 | 66. 91 | 6 |
| ATOM | 1084 | CG | LYS | 140 | 18. 867 | 6. 152 | 18. 117 | 1. 00 | 71. 34 | 6 |
| ATOM | 1085 | CD | LYS | 140 | 17. 786 | 6. 159 | 17. 624 | 1. 00 | 66. 95 | 6 |
| ATOM | 1086 | CB | LYS | 140 | 17. 470 | 5. 310 | 16. 130 | 1. 00 | 58. 85 | 6 |
| ATOM | 1087 | NZ | LYS | 140 | 18. 547 | 6. 008 | 15. 350 | 1. 00 | 57. 98 | 7 |
| ATOM | 1088 | C | LYS | 140 | 20. 188 | 3. 848 | 20. 983 | 1. 00 | 64. 59 | 6 |
| ATOM | 1089 | O | LYS | 140 | 20. 784 | 2. 880 | 20. 509 | 1. 00 | 64. 37 | 8 |
| ATOM | 1090 | N | ASN | 141 | 20. 420 | 4. 311 | 22. 207 | 1. 00 | 64. 62 | 7 |
| ATOM | 1091 | CA | ASN | 141 | 21. 348 | 3. 629 | 23. 113 | 1. 00 | 63. 50 | 6 |
| ATOM | 1092 | CB | ASN | 141 | 22. 150 | 4. 619 | 23. 930 | 1. 00 | 61. 22 | 6 |
| ATOM | 1093 | CG | ASN | 141 | 21. 503 | 5. 969 | 23. 990 | 1. 00 | 66. 93 | 6 |
| ATOM | 1094 | OD1 | ASN | 141 | 20. 846 | 6. 316 | 24. 973 | 1. 00 | 62. 97 | 8 |
| ATOM | 1095 | ND2 | ASN | 141 | 21. 689 | 6. 751 | 22. 927 | 1. 00 | 68. 02 | 7 |
| ATOM | 1096 | C | ASN | 141 | 20. 458 | 2. 850 | 24. 050 | 1. 00 | 65. 55 | 6 |
| ATOM | 1097 | O | ASN | 141 | 20. 928 | 2. 073 | 24. 883 | 1. 00 | 69. 96 | 8 |
| ATOM | ·1098 | N | ASN | 142 | 19. 159 | 3. 091 | 23. 914 | 1. 00 | 66. 07 | 7 |
| ATOM | 1099 | CA | ASN | 142 | 18. 150 | 2. 422 | 24. 712 | 1. 00 | 65. 69 | 6 |
| ATOM | 1100 | CB | ASN | 142 | 18. 302 | 0. 907 | 24. 548 | 1. 00 | 66. 29 | 6 |
| ATOM | 1101 | CG | ASN | 142 | 18. 549 | 0. 495 | 23. 105 | 1. 00 | 66. 09 | 6 |
| ATOM | 1102 | OD1 | ASN | 142 | 19. 682 | 0. 223 | 22. 715 | 1. 00 | 72. 21 | 8 |
| ATOM | 1103 | ND2 | ASN | 142 | 17. 486 | 0. 443 | 22. 307 | 1. 00 | 64. 43 | 7 |
| ATOM | 1104 | C | ASN | 142 | 18. 148 | 2. 789 | 26. 200 | 1. 00 | 65. 88 | 6 |
| ATOM | 1105 | O | ASN | 142 | 17. 517 | 2. 094 | 27. 002 | 1. 00 | 66. 50 | 8 |
| ATOM | 1106 | N | ASP | 143 | 18. 825 | 3. 876 | 26. 574 | 1. 00 | 62. 15 | 7 |
| ATOM | 1107 | CA | ASP | 143 | 18. 861 | 4. 266 | 27. 978 | 1. 00 | 55. 43 | 6 |
| ATOM | 1108 | CB | ASP | 143 | 19. 781 | 5. 494 | 28. 207 | 1. 00 | 58. 08 | 6 |
| ATOM | 1109 | CG | ASP | 143 | 19. 334 | 6. 744 | 27. 450 | 1. 00 | 61. 06 | 6 |
| ATOM | 1110 | OD1 | ASP | 143 | 18. 141 | 6. 840 | 27. 105 | 1. 00 | 60. 73 | 8 |
| ATOM | 1111 | OD2 | ASP | 143 | 20. 183 | 7. 639 | 27. 205 | 1. 00 | 58. 12 | 8 |

Fig. 35

| ATOM | 1112 | C | ASP | 143 | 17.463 | 4.510 | 28.536 | 1.00 | 52.74 | 6 |
|---|---|---|---|---|---|---|---|---|---|---|
| ATOM | 1113 | O | ASP | 143 | 17.315 | 4.836 | 29.706 | 1.00 | 64.17 | 8 |
| ATOM | 1114 | N | GLY | 144 | 16.437 | 4.326 | 27.709 | 1.00 | 50.01 | 7 |
| ATOM | 1115 | CA | GLY | 144 | 15.069 | 4.516 | 28.170 | 1.00 | 45.91 | 6 |
| ATOM | 1116 | C | GLY | 144 | 14.552 | 5.926 | 27.971 | 1.00 | 48.66 | 6 |
| ATOM | 1117 | O | GLY | 144 | 13.358 | 6.180 | 28.115 | 1.00 | 48.06 | 8 |
| ATOM | 1118 | N | ARG | 145 | 15.460 | 6.844 | 27.654 | 1.00 | 51.25 | 7 |
| ATOM | 1119 | CA | ARG | 145 | 15.111 | 8.245 | 27.413 | 1.00 | 54.54 | 6 |
| ATOM | 1120 | CB | ARG | 145 | 15.524 | 9.125 | 28.602 | 1.00 | 55.73 | 6 |
| ATOM | 1121 | CG | ARG | 145 | 16.009 | 8.360 | 29.812 | 1.00 | 54.52 | 6 |
| ATOM | 1122 | CD | ARG | 145 | 17.028 | 9.157 | 30.601 | 1.00 | 51.69 | 6 |
| ATOM | 1123 | NE | ARG | 145 | 18.390 | 8.649 | 30.425 | 1.00 | 58.38 | 7 |
| ATOM | 1124 | CZ | ARG | 145 | 19.076 | 7.991 | 31.361 | 1.00 | 53.53 | 6 |
| ATOM | 1125 | NH1 | ARG | 145 | 18.524 | 7.751 | 32.546 | 1.00 | 53.19 | 7 |
| ATOM | 1126 | NH2 | ARG | 145 | 20.321 | 7.588 | 31.120 | 1.00 | 44.02 | 7 |
| ATOM | 1127 | C | ARG | 145 | 15.798 | 8.753 | 26.138 | 1.00 | 50.78 | 6 |
| ATOM | 1128 | O | ARG | 145 | 16.268 | 7.966 | 25.313 | 1.00 | 57.19 | 8 |
| ATOM | 1129 | N | ILE | 146 | 15.881 | 10.070 | 25.990 | 1.00 | 41.48 | 7 |
| ATOM | 1130 | CA | ILE | 146 | 16.478 | 10.653 | 24.800 | 1.00 | 32.86 | 6 |
| ATOM | 1131 | CB | ILE | 146 | 15.377 | 11.035 | 23.785 | 1.00 | 37.98 | 6 |
| ATOM | 1132 | CG2 | ILE | 146 | 15.797 | 12.247 | 22.985 | 1.00 | 32.21 | 6 |
| ATOM | 1133 | CG1 | ILE | 146 | 15.047 | 9.841 | 22.884 | 1.00 | 40.57 | 6 |
| ATOM | 1134 | CD1 | ILE | 146 | 13.556 | 9.684 | 22.594 | 1.00 | 42.16 | 6 |
| ATOM | 1135 | C | ILE | 146 | 17.259 | 11.906 | 25.109 | 1.00 | 24.26 | 6 |
| ATOM | 1136 | O | ILE | 146 | 16.737 | 12.822 | 25.709 | 1.00 | 28.03 | 8 |
| ATOM | 1137 | N | ASP | 147 | 18.511 | 11.952 | 24.695 | 1.00 | 23.53 | 7 |
| ATOM | 1138 | CA | ASP | 147 | 19.291 | 13.156 | 24.921 | 1.00 | 29.67 | 6 |
| ATOM | 1139 | CB | ASP | 147 | 20.730 | 12.785 | 25.217 | 1.00 | 32.68 | 6 |
| ATOM | 1140 | CG | ASP | 147 | 21.352 | 11.984 | 24.105 | 1.00 | 35.59 | 6 |
| ATOM | 1141 | OD1 | ASP | 147 | 21.716 | 12.576 | 23.067 | 1.00 | 32.41 | 8 |
| ATOM | 1142 | OD2 | ASP | 147 | 21.473 | 10.760 | 24.271 | 1.00 | 38.02 | 8 |
| ATOM | 1143 | C | ASP | 147 | 19.233 | 14.079 | 23.680 | 1.00 | 29.91 | 6 |
| ATOM | 1144 | O | ASP | 147 | 18.589 | 13.776 | 22.674 | 1.00 | 31.66 | 8 |
| ATOM | 1145 | N | PHE | 148 | 19.916 | 15.210 | 23.758 | 1.00 | 34.17 | 7 |
| ATOM | 1146 | CA | PHE | 148 | 19.919 | 16.157 | 22.664 | 1.00 | 35.20 | 6 |
| ATOM | 1147 | CB | PHE | 148 | 20.862 | 17.315 | 22.982 | 1.00 | 28.83 | 6 |
| ATOM | 1148 | CG | PHE | 148 | 20.869 | 18.410 | 21.937 | 1.00 | 40.32 | 6 |
| ATOM | 1149 | CD1 | PHE | 148 | 19.675 | 18.869 | 21.379 | 1.00 | 36.62 | 6 |
| ATOM | 1150 | CD2 | PHE | 148 | 22.066 | 19.008 | 21.539 | 1.00 | 41.49 | 6 |
| ATOM | 1151 | CE1 | PHE | 148 | 19.668 | 19.901 | 20.457 | 1.00 | 39.42 | 6 |

Fig. 36

| ATOM | 1152 | CE2 | PHE | 148 | 22.071 | 20.045 | 20.611 | 1.00 | 38.79 | 6 |
|------|------|-----|-----|-----|--------|--------|--------|------|-------|---|
| ATOM | 1153 | CZ | PHE | 148 | 20.869 | 20.493 | 20.071 | 1.00 | 47.84 | 6 |
| ATOM | 1154 | C | PHE | 148 | 20.331 | 15.506 | 21.349 | 1.00 | 41.16 | 6 |
| ATOM | 1155 | O | PHE | 148 | 19.541 | 15.455 | 20.410 | 1.00 | 40.69 | 8 |
| ATOM | 1156 | N | ASP | 149 | 21.565 | 15.012 | 21.278 | 1.00 | 42.55 | 7 |
| ATOM | 1157 | CA | ASP | 149 | 22.064 | 14.391 | 20.054 | 1.00 | 34.76 | 6 |
| ATOM | 1158 | CB | ASP | 149 | 23.415 | 13.737 | 20.290 | 1.00 | 39.12 | 6 |
| ATOM | 1159 | CG | ASP | 149 | 24.565 | 14.755 | 20.298 | 1.00 | 45.34 | 6 |
| ATOM | 1160 | OD1 | ASP | 149 | 24.357 | 15.927 | 20.708 | 1.00 | 39.03 | 8 |
| ATOM | 1161 | OD2 | ASP | 149 | 25.685 | 14.376 | 19.890 | 1.00 | 53.44 | 8 |
| ATOM | 1162 | C | ASP | 149 | 21.059 | 13.395 | 19.488 | 1.00 | 31.51 | 6 |
| ATOM | 1163 | O | ASP | 149 | 20.904 | 13.313 | 18.277 | 1.00 | 35.90 | 8 |
| ATOM | 1164 | N | GLU | 150 | 20.363 | 12.670 | 20.359 | 1.00 | 34.86 | 7 |
| ATOM | 1165 | CA | GLU | 150 | 19.355 | 11.696 | 19.922 | 1.00 | 32.11 | 6 |
| ATOM | 1166 | CB | GLU | 150 | 18.922 | 10.753 | 21.062 | 1.00 | 36.22 | 6 |
| ATOM | 1167 | CG | GLU | 150 | 20.008 | 10.191 | 21.979 | 1.00 | 43.30 | 6 |
| ATOM | 1168 | CD | GLU | 150 | 19.465 | 9.135 | 22.957 | 1.00 | 48.35 | 6 |
| ATOM | 1169 | OE1 | GLU | 150 | 19.054 | 9.490 | 24.085 | 1.00 | 60.05 | 8 |
| ATOM | 1170 | OE2 | GLU | 150 | 19.451 | 7.937 | 22.601 | 1.00 | 50.75 | 8 |
| ATOM | 1171 | C | GLU | 150 | 18.104 | 12.426 | 19.460 | 1.00 | 28.50 | 6 |
| ATOM | 1172 | O | GLU | 150 | 17.343 | 11.911 | 18.657 | 1.00 | 26.68 | 8 |
| ATOM | 1173 | N | PHE | 151 | 17.876 | 13.608 | 20.023 | 1.00 | 33.81 | 7 |
| ATOM | 1174 | CA | PHE | 151 | 16.704 | 14.428 | 19.704 | 1.00 | 32.52 | 6 |
| ATOM | 1175. | CB | PHE | 151 | 16.554 | 15.535 | 20.762 | 1.00 | 25.07 | 6 |
| ATOM | 1176 | CG | PHE | 151 | 15.374 | 16.445 | 20.548 | 1.00 | 12.80 | 6 |
| ATOM | 1177 | CD1 | PHE | 151 | 14.084 | 15.958 | 20.607 | 1.00 | 10.11 | 6 |
| ATOM | 1178 | CD2 | PHE | 151 | 15.564 | 17.800 | 20.299 | 1.00 | 15.15 | 6 |
| ATOM | 1179 | CE1 | PHE | 151 | 12.972 | 16.814 | 20.418 | 1.00 | 14.15 | 6 |
| ATOM | 1180 | CE2 | PHE | 151 | 14.464 | 18.665 | 20.107 | 1.00 | 20.17 | 6 |
| ATOM | 1181 | CZ | PHE | 151 | 13.165 | 18.165 | 20.169 | 1.00 | 7.24 | 6 |
| ATOM | 1182 | C | PHE | 151 | 16.886 | 15.034 | 18.317 | 1.00 | 37.21 | 6 |
| ATOM | 1183 | O | PHE | 151 | 15.910 | 15.273 | 17.599 | 1.00 | 44.35 | 8 |
| ATOM | 1184 | N | LEU | 152 | 18.148 | 15.269 | 17.954 | 1.00 | 33.93 | 7 |
| ATOM | 1185 | CA | LEU | 152 | 18.506 | 15.827 | 16.655 | 1.00 | 26.49 | 6 |
| ATOM | 1186 | CB | LEU | 152 | 20.016 | 16.033 | 16.583 | 1.00 | 28.62 | 6 |
| ATOM | 1187 | CG | LEU | 152 | 20.630 | 17.424 | 16.806 | 1.00 | 32.70 | 6 |
| ATOM | 1188 | CD1 | LEU | 152 | 19.636 | 18.382 | 17.447 | 1.00 | 30.65 | 6 |
| ATOM | 1189 | CD2 | LEU | 152 | 21.853 | 17.278 | 17.678 | 1.00 | 20.60 | 6 |
| ATOM | 1190 | C | LEU | 152 | 18.067 | 14.842 | 15.584 | 1.00 | 23.24 | 6 |
| ATOM | 1191 | O | LEU | 152 | 17.229 | 15.159 | 14.760 | 1.00 | 25.98 | 8 |

# Fig. 37

| | | | | | | | | | | |
|---|---|---|---|---|---|---|---|---|---|---|
| ATOM | 1192 | N | LYS | 153 | 18.614 | 13.634 | 15.630 | 1.00 | 20.71 | 7 |
| ATOM | 1193 | CA | LYS | 153 | 18.285 | 12.598 | 14.667 | 1.00 | 23.02 | 6 |
| ATOM | 1194 | CB | LYS | 153 | 18.846 | 11.254 | 15.113 | 1.00 | 16.86 | 6 |
| ATOM | 1195 | CG | LYS | 153 | 20.346 | 11.197 | 15.289 | 1.00 | 31.60 | 6 |
| ATOM | 1196 | CD | LYS | 153 | 21.072 | 12.202 | 14.435 | 1.00 | 34.78 | 6 |
| ATOM | 1197 | CE | LYS | 153 | 22.196 | 12.849 | 15.231 | 1.00 | 47.65 | 6 |
| ATOM | 1198 | NZ | LYS. | 153 | 22.543 | 14.225 | 14.738 | 1.00 | 54.43 | 7 |
| ATOM | 1199 | C | LYS | 153 | 16.791 | 12.437 | 14.484 | 1.00 | 28.22 | 6 |
| ATOM | 1200 | O | LYS | 153 | 16.295 | 12.350 | 13.363 | 1.00 | 38.48 | 8 |
| ATOM | 1201 | N | MET | 154 | 16.080 | 12.379 | 15.599 | 1.00 | 33.34 | 7 |
| ATOM | 1202 | CA | MET | 154 | 14.639 | 12.195 | 15.698 | 1.00 | 33.99 | 6 |
| ATOM | 1203 | CB | MET | 154 | 14.159 | 11.997 | 17.039 | 1.00 | 35.20 | 6 |
| ATOM | 1204 | CG | MET | 154 | 12.725 | 12.397 | 17.317 | 1.00 | 29.29 | 6 |
| ATOM | 1205 | SD | MET | 154 | 12.388 | 12.191 | 19.059 | 1.00 | 35.15 | 16 |
| ATOM | 1206 | CE | MET | 154 | 10.654 | 11.761 | 19.027 | 1.00 | 21.48 | 6 |
| ATOM | 1207 | C | MET | 154 | 13.935 | 13.380 | 14.959 | 1.00 | 40.87 | 6 |
| ATOM | 1208 | O | MET | 154 | 12.847 | 13.247 | 14.382 | 1.00 | 45.49 | 8 |
| ATOM | 1209 | N | MET | 155 | 14.572 | 14.540 | 15.025 | 1.00 | 38.46 | 7 |
| ATOM | 1210 | CA | MET | 155 | 13.959 | 15.731 | 14.471 | 1.00 | 39.98 | 6 |
| ATOM | 1211 | CB | MET | 155 | 14.230 | 16.915 | 15.397 | 1.00 | 36.95 | 6 |
| ATOM | 1212 | CG | MET | 155 | 13.320 | 16.976 | 16.624 | 1.00 | 32.13 | 6 |
| ATOM | 1213 | SD | MET | 155 | 12.099 | 15.637 | 16.830 | 1.00 | 43.77 | 16 |
| ATOM | 1214 | CE | MET | 155 | 10.585 | 16.559 | 16.876 | 1.00 | 18.07 | 6 |
| ATOM | 1215 | C | MET | 155 | 14.444 | 16.034 | 13.061 | 1.00 | 44.19 | 6 |
| ATOM | 1216 | O | MET | 155 | 13.932 | 16.944 | 12.407 | 1.00 | 52.32 | 8 |
| ATOM | 1217 | N | GLU | 156 | 15.411 | 15.246 | 12.592 | 1.00 | 44.67 | 7 |
| ATOM | 1218 | CA | GLU | 156 | 16.004 | 15.410 | 11.268 | 1.00 | 37.41 | 6 |
| ATOM | 1219 | CB | GLU | 156 | 16.770 | 14.142 | 10.884 | 1.00 | 39.84 | 6 |
| ATOM | 1220 | CG | GLU | 156 | 17.766 | 14.334 | 9.750 | 1.00 | 45.59 | 6 |
| ATOM | 1221 | CD | GLU | 156 | 19.201 | 14.097 | 10.174 | 1.00 | 55.33 | 6 |
| ATOM | 1222 | OE1 | GLU | 156 | 19.861 | 13.233 | 9.563 | 1.00 | 57.33 | 8 |
| ATOM | 1223 | OE2 | GLU | 156 | 19.673 | 14.772 | 11.116 | 1.00 | 60.96 | 8 |
| ATOM | 1224 | C | GLU | 156 | 14.977 | 15.738 | 10.187 | 1.00 | 38.40 | 6 |
| ATOM | 1225 | O | GLU | 156 | 14.077 | 14.940 | 9.894 | 1.00 | 38.41 | 8 |
| ATOM | 1226 | N | GLY | 157 | 15.111 | 16.935 | 9.619 | 1.00 | 38.17 | 7 |
| ATOM | 1227 | CA | GLY | 157 | 14.220 | 17.373 | 8.565 | 1.00 | 30.65 | 6 |
| ATOM | 1228 | C | GLY | 157 | 12.748 | 17.194 | 8.861 | 1.00 | 38.51 | 6 |
| ATOM | 1229 | O | GLY | 157 | 12.040 | 16.502 | 8.134 | 1.00 | 42.59 | 8 |
| ATOM | 1230 | N | VAL | 158 | 12.285 | 17.796 | 9.948 | 1.00 | 37.11 | 7 |
| ATOM | 1231 | CA | VAL | 158 | 10.882 | 17.713 | 10.303 | 1.00 | 32.38 | 6 |

Fig. 38

| | | | | | | | | | |
|---|---|---|---|---|---|---|---|---|---|
| ATOM | 1232 | CB | VAL | 158 | 10. 696 | 17. 143 | 11. 711 | 1. 00 29. 12 | 6 |
| ATOM | 1233 | CG1 | VAL | 158 | 9. 233 | 17. 288 | 12. 131 | 1. 00 32. 08 | 6 |
| ATOM | 1234 | CG2 | VAL | 158 | 11. 120 | 15. 667 | 11. 735 | 1. 00 19. 07 | 6 |
| ATOM | 1235 | C | VAL | 158 | 10. 292 | 19. 110 | 10. 244 | 1. 00 29. 44 | 6 |
| ATOM | 1236 | O | VAL | 158 | 10. 933 | 20. 070 | 10. 651 | 1. 00 28. 19 | 8 |
| ATOM | 1237 | N | GLN | 159 | 9. 070 | 19. 219 | 9. 741 | 1. 00 32. 00 | 7 |
| ATOM | 1238 | CA | GLN | 159 | 8. 411 | 20. 518 | 9. 612 | 1. 00 40. 74 | 6 |
| ATOM | 1239 | CB | GLN | 159 | 7. 956 | 20. 726 | 8. 159 | 1. 00 46. 40 | 6 |
| ATOM | 1240 | CG | GLN | 159 | 8. 473 | 19. 681 | 7. 179 | 1. 00 49. 06 | 6 |
| ATOM | 1241 | CD | GLN | 159 | 9. 657 | 20. 187 | 6. 390 | 1. 00 48. 99 | 6 |
| ATOM | 1242 | OE1 | GLN | 159 | 9. 492 | 20. 889 | 5. 395 | 1. 00 50. 28 | 8 |
| ATOM | 1243 | NE2 | GLN | 159 | 10. 862 | 19. 844 | 6. 832 | 1. 00 49. 65 | 7 |
| ATOM | 1244 | C | GLN | 159 | 7. 202 | 20. 670 | 10. 536 | 1. 00 40. 23 | 6 |
| ATOM | 1245 | O | GLN | 159 | 7. 114 | 21. 691 | 11. 266 | 1. 00 35. 24 | 8 |
| ATOM | 1246 | OT | GLN | 159 | 6. 347 | 19. 758 | 10. 488 | 1. 00 41. 23 | 8 |
| ATOM | 1247 | CB | GLU | 203 | −0. 399 | −6. 525 | 6. 898 | 1. 00 57. 28 | 6 |
| ATOM | 1248 | CG | GLU | 203 | 0. 907 | −7. 164 | 6. 349 | 1. 00 65. 50 | 6 |
| ATOM | 1249 | CD | GLU | 203 | 0. 688 | −8. 308 | 7. 332 | 1. 00 74. 39 | 6 |
| ATOM | 1250 | OE1 | GLU | 203 | −0. 196 | −9. 156 | 7. 076 | 1. 00 70. 42 | 8 |
| ATOM | 1251 | OE2 | GLU | 203 | 1. 399 | −8. 360 | 8. 361 | 1. 00 81. 67 | 8 |
| ATOM | 1252 | C | GLU | 203 | 0. 507 | −4. 327 | 6. 593 | 1. 00 54. 69 | 6 |
| ATOM | 1253 | O | GLU | 203 | 1. 252 | −4. 250 | 7. 562 | 1. 00 61. 02 | 8 |
| ATOM | 1254 | N | GLU | 203 | −1. 944 | −4. 582 | 6. 038 | 1. 00 50. 42 | 7 |
| ATOM | 1255 | CA | GLU | 203 | −0. 726 | −5. 224 | 6. 629 | 1. 00 54. 11 | 6 |
| ATOM | 1256 | N | GLU | 204 | 0. 736 | −3. 680 | 5. 461 | 1. 00 52. 77 | 7 |
| ATOM | 1257 | CA | GLU | 204 | 1. 870 | −2. 787 | 5. 312 | 1. 00 49. 83 | 6 |
| ATOM | 1258 | CB | GLU | 204 | 2. 664 | −3. 141 | 4. 053 | 1. 00 54. 91 | 6 |
| ATOM | 1259 | CG | GLU | 204 | 1. 890 | −2. 944 | 2. 747 | 1. 00 57. 77 | 6 |
| ATOM | 1260 | CD | GLU | 204 | 2. 039 | −4. 122 | 1. 803 | 1. 00 59. 83 | 6 |
| ATOM | 1261 | OE1 | GLU | 204 | 3. 123 | −4. 267 | 1. 192 | 1. 00 60. 55 | 8 |
| ATOM | 1262 | OE2 | GLU | 204 | 1. 074 | −4. 908 | 1. 677 | 1. 00 61. 77 | 8 |
| ATOM | 1263 | C | GLU | 204 | 1. 266 | −1. 404 | 5. 181 | 1. 00 48. 33 | 6 |
| ATOM | 1264 | O | GLU | 204 | 1. 960 | −0. 401 | 5. 128 | 1. 00 49. 87 | 8 |
| ATOM | 1265 | N | LYS | 205 | −0. 054 | −1. 369 | 5. 123 | 1. 00 49. 14 | 7 |
| ATOM | 1266 | CA | LYS | 205 | −0. 792 | −0. 121 | 5. 007 | 1. 00 53. 04 | 6 |
| ATOM | 1267 | CB | LYS | 205 | −2. 249 | −0. 412 | 4. 652 | 1. 00 59. 43 | 6 |
| ATOM | 1268 | CG | LYS | 205 | −2. 907 | 0. 698 | 3. 743 | 1. 00 60. 08 | 6 |
| ATOM | 1269 | CD | LYS | 205 | −4. 247 | 0. 067 | 3. 252 | 1. 00 61. 13 | 6 |
| ATOM | 1270 | CE | LYS | 205 | −4. 532 | 0. 526 | 1. 834 | 1. 00 60. 92 | 6 |
| ATOM | 1271 | NZ | LYS | 205 | −5. 325 | 1. 789 | 1. 808 | 1. 00 62. 92 | 7 |

## F i g. 3 9

| | | | | | | | | | | |
|---|---|---|---|---|---|---|---|---|---|---|
| ATOM | 1272 | C | LYS | 205 | -0.747 | 0.531 | 6.369 | 1.00 | 47.58 | 6 |
| ATOM | 1273 | O | LYS | 205 | -0.631 | 1.756 | 6.489 | 1.00 | 46.38 | 8 |
| ATOM | 1274 | N | ARG | 206 | -0.849 | -0.317 | 7.392 | 1.00 | 48.04 | 7 |
| ATOM | 1275 | CA | ARG | 206 | -0.834 | 0.120 | 8.784 | 1.00 | 51.17 | 6 |
| ATOM | 1276 | CB | ARG | 206 | -1.017 | -1.099 | 9.716 | 1.00 | 51.50 | 6 |
| ATOM | 1277 | CG | ARG | 206 | 0.021 | -1.277 | 10.828 | 1.00 | 56.07 | 6 |
| ATOM | 1278 | CD | ARG | 206 | -0.425 | -0.660 | 12.156 | 1.00 | 64.30 | 6 |
| ATOM | 1279 | NE | ARG | 206 | -1.866 | -0.413 | 12.225 | 1.00 | 66.92 | 7 |
| ATOM | 1280 | CZ | ARG | 206 | -2.429 | 0.794 | 12.200 | 1.00 | 65.00 | 6 |
| ATOM | 1281 | NH1 | ARG | 206 | -1.683 | 1.892 | 12.114 | 1.00 | 60.08 | 7 |
| ATOM | 1282 | NH2 | ARG | 206 | -3.750 | 0.901 | 12.269 | 1.00 | 63.76 | 7 |
| ATOM | 1283 | C | ARG | 206 | 0.459 | 0.866 | 9.088 | 1.00 | 49.59 | 6 |
| ATOM | 1284 | O | ARG | 206 | 0.429 | 2.043 | 9.436 | 1.00 | 51.33 | 8 |
| ATOM | 1285 | N | ASN | 207 | 1.590 | 0.189 | 8.918 | 1.00 | 49.76 | 7 |
| ATOM | 1286 | CA | ASN | 207 | 2.890 | 0.783 | 9.183 | 1.00 | 52.25 | 6 |
| ATOM | 1287 | CB | ASN | 207 | 4.011 | -0.171 | 8.756 | 1.00 | 53.10 | 6 |
| ATOM | 1288 | CG | ASN | 207 | 3.767 | -1.616 | 9.202 | 1.00 | 62.47 | 6 |
| ATOM | 1289 | OD1 | ASN | 207 | 4.433 | -2.542 | 8.736 | 1.00 | 67.63 | 8 |
| ATOM | 1290 | ND2 | ASN | 207 | 2.812 | -1.809 | 10.102 | 1.00 | 64.69 | 7 |
| ATOM | 1291 | C | ASN | 207 | 3.027 | 2.105 | 8.444 | 1.00 | 54.65 | 6 |
| ATOM | 1292 | O | ASN | 207 | 3.628 | 3.044 | 8.964 | 1.00 | 54.25 | 8 |
| ATOM | 1293 | N | ARG | 208 | 2.464 | 2.176 | 7.234 | 1.00 | 52.53 | 7 |
| ATOM | 1294 | CA | ARG | 208 | 2.523 | 3.393 | 6.421 | 1.00 | 46.95 | 6 |
| ATOM | 1295 | CB | ARG | 208 | 1.739 | 3.218 | 5.115 | 1.00 | 55.63 | 6 |
| ATOM | 1296 | CG | ARG | 208 | 2.284 | 2.153 | 4.161 | 1.00 | 55.66 | 6 |
| ATOM | 1297 | CD | ARG | 208 | 3.409 | 2.685 | 3.287 | 1.00 | 55.66 | 6 |
| ATOM | 1298 | NE | ARG | 208 | 3.030 | 3.892 | 2.554 | 1.00 | 56.25 | 7 |
| ATOM | 1299 | CZ | ARG | 208 | 3.222 | 5.131 | 3.003 | 1.00 | 57.91 | 6 |
| ATOM | 1300 | NH1 | ARG | 208 | 3.790 | 5.337 | 4.191 | 1.00 | 50.09 | 7 |
| ATOM | 1301 | NH2 | ARG | 208 | 2.848 | 6.167 | 2.261 | 1.00 | 54.42 | 7 |
| ATOM | 1302 | C | ARG | 208 | 1.877 | 4.488 | 7.239 | 1.00 | 45.84 | 6 |
| ATOM | 1303 | O | ARG | 208 | 2.363 | 5.626 | 7.296 | 1.00 | 40.63 | 8 |
| ATOM | 1304 | N | ALA | 209 | 0.763 | 4.119 | 7.868 | 1.00 | 45.23 | 7 |
| ATOM | 1305 | CA | ALA | 209 | 0.012 | 5.031 | 8.718 | 1.00 | 45.13 | 6 |
| ATOM | 1306 | CB | ALA | 209 | -1.341 | 4.414 | 9.059 | 1.00 | 40.54 | 6 |
| ATOM | 1307 | C | ALA | 209 | 0.800 | 5.330 | 9.996 | 1.00 | 38.26 | 6 |
| ATOM | 1308 | O | ALA | 209 | 0.915 | 6.476 | 10.421 | 1.00 | 41.12 | 8 |
| ATOM | 1309 | N | ILE | 210 | 1.355 | 4.281 | 10.585 | 1.00 | 34.98 | 7 |
| ATOM | 1310 | CA | ILE | 210 | 2.118 | 4.395 | -11.806 | 1.00 | 33.14 | 6 |
| ATOM | 1311 | CB | ILE | 210 | 2.756 | 3.053 | 12.150 | 1.00 | 32.09 | 6 |

Fig. 40

| | | | | | | | | | | |
|---|---|---|---|---|---|---|---|---|---|---|
| ATOM | 1312 | CG2 | ILE | 210 | 3.952 | 3.266 | 13.071 | 1.00 | 31.31 | 6 |
| ATOM | 1313 | CG1 | ILE | 210 | 1.695 | 2.154 | 12.787 | 1.00 | 30.51 | 6 |
| ATOM | 1314 | CD1 | ILE | 210 | 2.230 | 0.852 | 13.349 | 1.00 | 35.61 | 6 |
| ATOM | 1315 | C | ILE | 210 | 3.210 | 5.447 | 11.706 | 1.00 | 37.14 | 6 |
| ATOM | 1316 | O | ILE | 210 | 3.335 | 6.303 | 12.588 | 1.00 | 33.24 | 8 |
| ATOM | 1317 | N | THR | 211 | 3.995 | 5.365 | 10.629 | 1.00 | 38.59 | 7 |
| ATOM | 1318 | CA | THR | 211 | 5.105 | 6.289 | 10.367 | 1.00 | 31.06 | 6 |
| ATOM | 1319 | CB | THR | 211 | 5.894 | 5.878 | 9.123 | 1.00 | 27.67 | 6 |
| ATOM | 1320 | OG1 | THR | 211 | 6.021 | 4.455 | 9.084 | 1.00 | 38.67 | 8 |
| ATOM | 1321 | CG2 | THR | 211 | 7.259 | 6.473 | 9.156 | 1.00 | 23.23 | 6 |
| ATOM | 1322 | C | THR | 211 | 4.588 | 7.686 | 10.121 | 1.00 | 31.36 | 6 |
| ATOM | 1323 | O | THR | 211 | 5.238 | 8.673 | 10.475 | 1.00 | 28.39 | 8 |
| ATOM | 1324 | N | ALA | 212 | 3.415 | 7.759 | 9.502 | 1.00 | 32.18 | 7 |
| ATOM | 1325 | CA | ALA | 212 | 2.792 | 9.031 | 9.185 | 1.00 | 34.63 | 6 |
| ATOM | 1326 | CB | ALA | 212 | 1.524 | 8.799 | 8.357 | 1.00 | 35.13 | 6 |
| ATOM | 1327 | C | ALA | 212 | 2.451 | 9.744 | 10.479 | 1.00 | 34.72 | 6 |
| ATOM | 1328 | O | ALA | 212 | 2.951 | 10.836 | 10.755 | 1.00 | 39.02 | 8 |
| ATOM | 1329 | N | ARG | 213 | 1.592 | 9.106 | 11.268 | 1.00 | 35.17 | 7 |
| ATOM | 1330 | CA | ARG | 213 | 1.151 | 9.635 | 12.549 | 1.00 | 30.01 | 6 |
| ATOM | 1331 | CB | ARG | 213 | 0.413 | 8.544 | 13.326 | 1.00 | 25.29 | 6 |
| ATOM | 1332 | CG | ARG | 213 | -0.025 | 8.935 | 14.728 | 1.00 | 22.65 | 6 |
| ATOM | 1333 | CD | ARG | 213 | -1.262 | 9.823 | 14.754 | 1.00 | 30.54 | 6 |
| ATOM | 1334 | NE | ARG | 213 | -1.283 | 10.656 | 15.963 | 1.00 | 42.64 | 7 |
| ATOM | 1335 | CZ | ARG | 213 | -2.368 | 11.193 | 16.518 | 1.00 | 42.35 | 6 |
| ATOM | 1336 | NH1 | ARG | 213 | -3.565 | 10.998 | 15.985 | 1.00 | 55.55 | 7 |
| ATOM | 1337 | NH2 | ARG | 213 | -2.254 | 11.920 | 17.619 | 1.00 | 46.89 | 7 |
| ATOM | 1338 | C | ARG | 213 | 2.363 | 10.112 | 13.329 | 1.00 | 30.88 | 6 |
| ATOM | 1339 | O | ARG | 213 | 2.378 | 11.220 | 13.856 | 1.00 | 34.32 | 8 |
| ATOM | 1340 | N | ARG | 214 | 3.397 | 9.283 | 13.372 | 1.00 | 29.60 | 7 |
| ATOM | 1341 | CA | ARG | 214 | 4.598 | 9.637 | 14.103 | 1.00 | 32.67 | 6 |
| ATOM | 1342 | CB | ARG | 214 | 5.606 | 8.496 | 14.047 | 1.00 | 42.34 | 6 |
| ATOM | 1343 | CG | ARG | 214 | 5.818 | 7.816 | 15.386 | 1.00 | 45.90 | 6 |
| ATOM | 1344 | CD | ARG | 214 | 6.942 | 6.812 | 15.309 | 1.00 | 47.22 | 6 |
| ATOM | 1345 | NE | ARG | 214 | 6.444 | 5.501 | 14.904 | 1.00 | 51.97 | 7 |
| ATOM | 1346 | CZ | ARG | 214 | 7.205 | 4.416 | 14.837 | 1.00 | 49.08 | 6 |
| ATOM | 1347 | NH1 | ARG | 214 | 8.495 | 4.501 | 15.140 | 1.00 | 46.02 | 7 |
| ATOM | 1348 | NH2 | ARG | 214 | 6.684 | 3.255 | 14.457 | 1.00 | 49.01 | 7 |
| ATOM | 1349 | C | ARG | 214 | 5.242 | 10.906 | 13.587 | 1.00 | 36.53 | 6 |
| ATOM | 1350 | O | ARG | 214 | 5.590 | 11.794 | 14.367 | 1.00 | 40.80 | 8 |
| ATOM | 1351 | N | GLN | 215 | 5.416 | 10.979 | 12.270 | 1.00 | 40.13 | 7 |

## Fig. 41

| ATOM | 1352 | CA | GLN | 215 | 6.012 | 12.150 | 11.624 | 1.00 | 29.42 | 6 |
|------|------|----|----|-----|-------|--------|--------|------|-------|---|
| ATOM | 1353 | CB | GLN | 215 | 6.066 | 11.924 | 10.111 | 1.00 | 27.01 | 6 |
| ATOM | 1354 | CG | GLN | 215 | 6.841 | 10.668 | 9.719 | 1.00 | 38.59 | 6 |
| ATOM | 1355 | CD | GLN | 215 | 6.843 | 10.377 | 8.207 | 1.00 | 46.65 | 6 |
| ATOM | 1356 | OB1 | GLN | 215 | 5.807 | 10.480 | 7.518 | 1.00 | 32.59 | 8 |
| ATOM | 1357 | NB2 | GLN | 215 | 8.015 | 10.003 | 7.691 | 1.00 | 41.16 | 7 |
| ATOM | 1358 | C | GLN | 215 | 5.178 | 13.385 | 11.954 | 1.00 | 20.91 | 6 |
| ATOM | 1359 | O | GLN | 215 | 5.704 | 14.472 | 12.148 | 1.00 | 25.96 | 8 |
| ATOM | 1360 | N | HIS | 216 | 3.868 | 13.203 | 12.025 | 1.00 | 17.13 | 7 |
| ATOM | 1361 | CA | HIS | 216 | 2.958 | 14.297 | 12.344 | 1.00 | 18.40 | 6 |
| ATOM | 1362 | CB | HIS | 216 | 1.513 | 13.827 | 12.185 | 1.00 | 20.60 | 6 |
| ATOM | 1363 | CG | HIS | 216 | 0.511 | 14.772 | 12.778 | 1.00 | 22.30 | 6 |
| ATOM | 1364 | CD2 | HIS | 216 | 0.325 | 16.092 | 12.597 | 1.00 | 25.28 | 6 |
| ATOM | 1365 | ND1 | HIS | 216 | -0.434 | 14.366 | 13.696 | 1.00 | 30.69 | 7 |
| ATOM | 1366 | CB1 | HIS | 216 | -1.157 | 15.414 | 14.057 | 1.00 | 18.87 | 6 |
| ATOM | 1367 | NB2 | HIS | 216 | -0.722 | 16.475 | 13.406 | 1.00 | 21.64 | 7 |
| ATOM | 1368 | C | HIS | 216 | 3.155 | 14.800 | 13.779 | 1.00 | 17.96 | 6 |
| ATOM | 1369 | O | HIS | 216 | 3.243 | 15.996 | 14.012 | 1.00 | 21.18 | 8 |
| ATOM | 1370 | N | LEU | 217 | 3.204 | 13.873 | 14.736 | 1.00 | 23.11 | 7 |
| ATOM | 1371 | CA | LEU | 217 | 3.380 | 14.232 | 16.151 | 1.00 | 20.87 | 6 |
| ATOM | 1372 | CB | LEU | 217 | 3.375 | 12.965 | 17.024 | 1.00 | 13.39 | 6 |
| ATOM | 1373 | CG | LEU | 217 | 1.985 | 12.327 | 17.147 | 1.00 | 14.88 | 6 |
| ATOM | 1374 | CD1 | LEU | 217 | 1.988 | 11.059 | 17.981 | 1.00 | 20.62 | 6 |
| ATOM | 1375 | CD2 | LEU | 217 | 1.072 | 13.334 | 17.771 | 1.00 | 19.89 | 6 |
| ATOM | 1376 | C | LEU | 217 | 4.665 | 15.031 | 16.368 | 1.00 | 23.91 | 6 |
| ATOM | 1377 | O | LEU | 217 | 4.699 | 15.949 | 17.193 | 1.00 | 26.60 | 8 |
| ATOM | 1378 | N | LYS | 218 | 5.706 | 14.705 | 15.610 | 1.00 | 22.85 | 7 |
| ATOM | 1379 | CA | LYS | 218 | 6.981 | 15.412 | 15.728 | 1.00 | 20.98 | 6 |
| ATOM | 1380 | CB | LYS | 218 | 8.071 | 14.713 | 14.906 | 1.00 | 19.60 | 6 |
| ATOM | 1381 | CG | LYS | 218 | 8.059 | 13.208 | 15.012 | 1.00 | 22.09 | 6 |
| ATOM | 1382 | CD | LYS | 218 | 9.449 | 12.669 | 15.216 | 1.00 | 26.85 | 6 |
| ATOM | 1383 | CB | LYS | 218 | 10.033 | 12.188 | 13.907 | 1.00 | 32.49 | 6 |
| ATOM | 1384 | NZ | LYS | 218 | 11.102 | 11.154 | 14.084 | 1.00 | 31.72 | 7 |
| ATOM | 1385 | C | LYS | 218 | 6.819 | 16.841 | 15.236 | 1.00 | 23.23 | 6 |
| ATOM | 1386 | O | LYS | 218 | 7.498 | 17.766 | 15.716 | 1.00 | 20.39 | 8 |
| ATOM | 1387 | N | SER | 219 | 5.937 | 17.012 | 14.256 | 1.00 | 22.19 | 7 |
| ATOM | 1388 | CA | SER | 219 | 5.665 | 18.337 | 13.694 | 1.00 | 25.90 | 6 |
| ATOM | 1389 | CB | SER | 219 | 4.784 | 18.207 | 12.436 | 1.00 | 33.01 | 6 |
| ATOM | 1390 | OG | SER | 219 | 4.363 | 19.469 | 11.921 | 1.00 | 28.44 | 8 |
| ATOM | 1391 | C | SER | 219 | 4.942 | 19.152 | 14.766 | 1.00 | 24.18 | 6 |

# Fig. 4 2

| ATOM | 1392 | O | SER | 219 | 5.166 | 20.359 | 14.920 | 1.00 | 21.82 | 8 |
|------|------|------|-----|-----|-------|--------|--------|------|-------|----|
| ATOM | 1393 | N | YAL | 220 | 4.059 | 18.470 | 15.492 | 1.00 | 29.43 | 7 |
| ATOM | 1394 | CA | YAL | 220 | 3.296 | 19.070 | 16.577 | 1.00 | 25.98 | 6 |
| ATOM | 1395 | CB | YAL | 220 | 2.298 | 18.060 | 17.153 | 1.00 | 23.17 | 6 |
| ATOM | 1396 | CG1 | YAL | 220 | 1.276 | 18.768 | 18.035 | 1.00 | 22.66 | 6 |
| ATOM | 1397 | CG2 | YAL | 220 | 1.608 | ·17.353 | 16.029 | 1.00 | 23.11 | 6 |
| ATOM | 1398 | C | YAL | 220 | 4.283 | 19.504 | 17.660 | 1.00 | 25.42 | 6 |
| ATOM | 1399 | O | YAL | 220 | 4.265 | 20.653 | 18.111 | 1.00 | 35.92 | 8 |
| ATOM | 1400 | N | MET | 221 | 5.149 | 18.587 | 18.069 | 1.00 | 23.16 | 7 |
| ATOM | 1401 | CA | MET | 221 | 6.150 | 18.910 | 19.066 | 1.00 | 18.59 | 6 |
| ATOM | 1402 | CB | MET | 221 | 7.217 | 17.825 | 19.096 | 1.00 | 18.20 | 6 |
| ATOM | 1403 | CG | MET | 221 | 6.655 | 16.521 | 19.603 | 1.00 | 18.47 | 6 |
| ATOM | 1404 | SD | MET | 221 | 7.785 | 15.179 | 19.497 | 1.00 | 45.61 | 16 |
| ATOM | 1405 | CE | MET | 221 | 9.160 | 15.832 | 20.408 | 1.00 | 34.21 | 6 |
| ATOM | 1406 | C | MET | 221 | 6.768 | 20.258 | 18.759 | 1.00 | 25.26 | 6 |
| ATOM | 1407 | O | MET | 221 | 6.701 | 21.163 | 19.582 | 1.00 | 31.91 | 8 |
| ATOM | 1408 | N | LEU | 222 | 7.343 | 20.411 | 17.567 | 1.00 | 31.51 | 7 |
| ATOM | 1409 | CA | LEU | 222 | 7.961 | 21.686 | 17.165 | 1.00 | 29.67 | 6 |
| ATOM | 1410 | CB | LEU | 222 | 8.381 | 21.649 | 15.682 | 1.00 | 29.32 | 6 |
| ATOM | 1411 | CG | LEU | 222 | 9.579 | 20.823 | 15.184 | 1.00 | 35.52 | 6 |
| ATOM | 1412 | CD1 | LEU | 222 | 9.383 | 20.510 | 13.704 | 1.00 | 33.41 | 6 |
| ATOM | 1413 | CD2 | LEU | 222 | 10.890 | 21.583 | 15.381 | 1.00 | 42.40 | 6 |
| ATOM | 1414 | C | LEU | 222 | 7.037 | 22.895 | 17.381 | 1.00 | 23.36 | 6 |
| ATOM | 1415 | O | LEU | 222 | 7.488 | 23.965 | 17.783 | 1.00 | 26.75 | 8 |
| ATOM | 1416 | N | GLN | 223 | 5.749 | 22.739 | 17.092 | 1.00 | 27.99 | 7 |
| ATOM | 1417 | CA | GLN | 223 | 4.816 | 23.850 | 17.268 | 1.00 | 28.30 | 6 |
| ATOM | 1418 | CB | GLN | 223 | 3.439 | 23.522 | 16.679 | 1.00 | 34.21 | 6 |
| ATOM | 1419 | CG | GLN | 223 | 2.664 | 24.752 | 16.205 | 1.00 | 36.21 | 6 |
| ATOM | 1420 | CD | GLN | 223 | 3.574 | 25.795 | 15.557 | 1.00 | 51.52 | 6 |
| ATOM | 1421 | OE1 | GLN | 223 | 3.399 | 27.003 | 15.747 | 1.00 | 49.07 | 8 |
| ATOM | 1422 | NE2 | GLN | 223 | 4.556 | 25.328 | 14.795 | 1.00 | 48.06 | 7 |
| ATOM | 1423 | C | GLN | 223 | 4.668 | 24.186 | 18.738 | 1.00 | 24.98 | 6 |
| ATOM | 1424 | O | GLN | 223 | 4.874 | 25.326 | 19.137 | 1.00 | 22.91 | 8 |
| ATOM | 1425 | N | ILE | 224 | 4.331 | 23.177 | 19.535 | 1.00 | 28.90 | 7 |
| ATOM | 1426 | CA | ILE | 224 | 4.147 | 23.338 | 20.987 | 1.00 | 28.51 | 6 |
| ATOM | 1427 | CB | ILE | 224 | 4.034 | 21.974 | 21.684 | 1.00 | 25.37 | 6 |
| ATOM | 1428 | CG2 | ILE | 224 | 4.640 | 22.039 | 23.041 | 1.00 | 24.48 | 6 |
| ATOM | 1429 | CG1 | ILE | 224 | 2.562 | 21.565 | 21.803 | 1.00 | 34.15 | 6 |
| ATOM | 1430 | CD1 | ILE | 224 | 2.346 | 20.039 | 21.867 | 1.00 | 38.15 | 6 |
| ATOM | 1431 | C | ILE | 224 | 5.343 | 24.075 | 21.546 | 1.00 | 25.89 | 6 |

Fig. 4 3

| | | | | | | | | | |
|---|---|---|---|---|---|---|---|---|---|
| ATOM | 1432 | O | ILE | 224 | 5.197 | 25.049 | 22.279 | 1.00 33.02 | 8 |
| ATOM | 1433 | N | ALA | 225 | 6.530 | 23.602 | 21.183 | 1.00 26.88 | 7 |
| ATOM | 1434 | CA | ALA | 225 | 7.771 | 24.209 | 21.613 | 1.00 25.96 | 6 |
| ATOM | 1435 | CB | ALA | 225 | 8.953 | 23.475 | 20.991 | 1.00 34.45 | 6 |
| ATOM | 1436 | C | ALA | 225 | 7.767 | 25.666 | 21.169 | 1.00 32.14 | 6 |
| ATOM | 1437 | O | ALA | 225 | 7.810 | 26.672 | 22.002 | 1.00 31.11 | 8 |
| ATOM | 1438 | N | ALA | 226 | 7.702 | 25.885 | 19.854 | 1.00 34.26 | 7 |
| ATOM | 1439 | CA | ALA | 226 | 7.686 | 27.235 | 19.302 | 1.00 33.46 | 6 |
| ATOM | 1440 | CB | ALA | 226 | 7.286 | 27.192 | 17.849 | 1.00 31.02 | 6 |
| ATOM | 1441 | C | ALA | 226 | 6.708 | 28.103 | 20.088 | 1.00 36.02 | 6 |
| ATOM | 1442 | O | ALA | 226 | 7.098 | 29.080 | 20.716 | 1.00 36.16 | 8 |
| ATOM | 1443 | N | THR | 227 | 5.432 | 27.744 | 20.057 | 1.00 34.30 | 7 |
| ATOM | 1444 | CA | THR | 227 | 4.401 | 28.486 | 20.781 | 1.00 36.18 | 6 |
| ATOM | 1445 | CB | THR | 227 | 3.094 | 27.645 | 20.878 | 1.00 40.48 | 6 |
| ATOM | 1446 | OG1 | THR | 227 | 2.578 | 27.387 | 19.562 | 1.00 47.64 | 8 |
| ATOM | 1447 | CG2 | THR | 227 | 2.041 | 28.381 | 21.682 | 1.00 42.32 | 6 |
| ATOM | 1448 | C | THR | 227 | 4.860 | 28.878 | 22.195 | 1.00 37.11 | 6 |
| ATOM | 1449 | O | THR | 227 | 4.826 | 30.054 | 22.577 | 1.00 41.04 | 8 |
| ATOM | 1450 | N | GLU | 228 | 5.287 | 27.890 | 22.974 | 1.00 39.03 | 7 |
| ATOM | 1451 | CA | GLU | 228 | 5.738 | 28.139 | 24.334 | 1.00 38.62 | 6 |
| ATOM | 1452 | CB | GLU | 228 | 6.122 | 26.819 | 25.012 | 1.00 47.75 | 6 |
| ATOM | 1453 | CG | GLU | 228 | 6.376 | 26.982 | 26.515 | 1.00 65.12 | 6 |
| ATOM | 1454 | CD | GLU | 228 | 6.155 | 25.710 | 27.327 | 1.00 67.70 | 6 |
| ATOM | 1455 | OE1 | GLU | 228 | 5.335 | 24.862 | 26.902 | 1.00 61.72 | 8 |
| ATOM | 1456 | OE2 | GLU | 228 | 6.802 | 25.569 | 28.396 | 1.00 68.52 | 8 |
| ATOM | 1457 | C | GLU | 228 | 6.908 | 29.116 | 24.408 | 1.00 37.00 | 6 |
| ATOM | 1458 | O | GLU | 228 | 7.007 | 29.908 | 25.346 | 1.00 35.35 | 8 |
| ATOM | 1459 | N | LEU | 229 | 7.784 | 29.068 | 23.408 | 1.00 36.97 | 7 |
| ATOM | 1460 | CA | LEU | 229 | 8.956 | 29.942 | 23.361 | 1.00 35.01 | 6 |
| ATOM | 1461 | CB | LEU | 229 | 9.948 | 29.431 | 22.330 | 1.00 29.07 | 6 |
| ATOM | 1462 | CG | LEU | 229 | 10.806 | 28.331 | 22.949 | 1.00 33.51 | 6 |
| ATOM | 1463 | CD1 | LEU | 229 | 11.357 | 27.372 | 21.880 | 1.00 27.60 | 6 |
| ATOM | 1464 | CD2 | LEU | 229 | 11.918 | 29.005 | 23.729 | 1.00 34.72 | 6 |
| ATOM | 1465 | C | LEU | 229 | 8.658 | 31.402 | 23.084 | 1.00 32.92 | 6 |
| ATOM | 1466 | O | LEU | 229 | 9.493 | 32.266 | 23.341 | 1.00 40.13 | 8 |
| ATOM | 1467 | N | GLU | 230 | 7.470 | 31.671 | 22.559 | 1.00 35.30 | 7 |
| ATOM | 1468 | CA | GLU | 230 | 7.053 | 33.029 | 22.261 | 1.00 37.27 | 6 |
| ATOM | 1469 | CB | GLU | 230 | 5.996 | 33.059 | 21.156 | 1.00 42.55 | 6 |
| ATOM | 1470 | CG | GLU | 230 | 6.103 | 31.946 | 20.146 | 1.00 67.54 | 6 |
| ATOM | 1471 | CD | GLU | 230 | 7.466 | 31.908 | 19.468 | 1.00 80.62 | 6 |

# Fig. 4 4

| | | | | | | | | | | |
|---|---|---|---|---|---|---|---|---|---|---|
| ATOM | 1472 | OB1 | GLU | 230 | 8.176 | 30.883 | 19.611 | 1.00 | 89.46 | 8 |
| ATOM | 1473 | OB2 | GLU | 230 | 7.830 | 32.901 | 18.793 | 1.00 | 85.85 | 8 |
| ATOM | 1474 | C | GLU | 230 | 6.433 | 33.601 | 23.510 | 1.00 | 39.67 | 6 |
| ATOM | 1475 | O | GLU | 230 | 6.621 | 34.781 | 23.802 | 1.00 | 45.94 | 8 |
| ATOM | 1476 | N | LYS | 231 | 5.701 | 32.759 | 24.243 | 1.00 | 39.66 | 7 |
| ATOM | 1477 | CA | LYS | 231 | 5.014 | 33.190 | 25.458 | 1.00 | 41.62 | 6 |
| ATOM | 1478 | CB | LYS | 231 | 4.111 | 32.087 | 26.006 | 1.00 | 44.02 | 6 |
| ATOM | 1479 | CG | LYS | 231 | 3.271 | 31.361 | 24.961 | 1.00 | 46.41 | 6 |
| ATOM | 1480 | CD | LYS | 231 | 1.800 | 31.276 | 25.370 | 1.00 | 42.15 | 6 |
| ATOM | 1481 | CE | LYS | 231 | 1.630 | 31.129 | 26.881 | 1.00 | 44.22 | 6 |
| ATOM | 1482 | NZ | LYS | 231 | 0.726 | 29.997 | 27.222 | 1.00 | 49.59 | 7 |
| ATOM | 1483 | C | LYS | 231 | 5.991 | 33.584 | 26.533 | 1.00 | 46.69 | 6 |
| ATOM | 1484 | O | LYS | 231 | 5.615 | 34.231 | 27.508 | 1.00 | 45.18 | 8 |
| ATOM | 1485 | N | GLU | 232 | 7.241 | 33.171 | 26.352 | 1.00 | 51.44 | 7 |
| ATOM | 1486 | CA | GLU | 232 | 8.306 | 33.486 | 27.294 | 1.00 | 54.71 | 6 |
| ATOM | 1487 | CB | GLU | 232 | 9.565 | 32.715 | 26.917 | 1.00 | 50.72 | 6 |
| ATOM | 1488 | CG | GLU | 232 | 9.527 | 31.259 | 27.309 | 1.00 | 45.53 | 6 |
| ATOM | 1489 | CD | GLU | 232 | 10.912 | 30.656 | 27.348 | 1.00 | 51.72 | 6 |
| ATOM | 1490 | OB1 | GLU | 232 | 11.746 | 31.005 | 26.481 | 1.00 | 56.07 | 8 |
| ATOM | 1491 | OB2 | GLU | 232 | 11.170 | 29.833 | 28.248 | 1.00 | 58.06 | 8 |
| ATOM | 1492 | C | GLU | 232 | 8.608 | 34.990 | 27.355 | 1.00 | 59.56 | 6 |
| ATOM | 1493 | O | GLU | 232 | 9.416 | 35.440 | 28.178 | 1.00 | 65.03 | 8 |
| ATOM | 1494 | N | GLU | 233 | 7.962 | 35.763 | 26.482 | 1.00 | 63.43 | 7 |
| ATOM | 1495 | CA | GLU | 233 | 8.138 | 37.214 | 26.469 | 1.00 | 63.31 | 6 |
| ATOM | 1496 | CB | GLU | 233 | 7.817 | 37.808 | 25.101 | 1.00 | 58.16 | 6 |
| ATOM | 1497 | CG | GLU | 233 | 9.016 | 37.897 | 24.213 | 1.00 | 58.57 | 6 |
| ATOM | 1498 | CD | GLU | 233 | 9.766 | 36.593 | 24.189 | 1.00 | 58.61 | 6 |
| ATOM | 1499 | OB1 | GLU | 233 | 9.162 | 35.572 | 23.782 | 1.00 | 47.56 | 8 |
| ATOM | 1500 | OB2 | GLU | 233 | 10.949 | 36.597 | 24.589 | 1.00 | 61.39 | 8 |
| ATOM | 1501 | C | GLU | 233 | 7.182 | 37.803 | 27.480 | 1.00 | 63.82 | 6 |
| ATOM | 1502 | O | GLU | 233 | 6.049 | 38.136 | 27.072 | 1.00 | 67.36 | 8 |
| ATOM | 1503 | OT | GLU | 233 | 7.582 | 37.913 | 28.659 | 1.00 | 72.06 | 8 |
| ATOM | 1504 | CAL | CA2 | 160 | 14.262 | 15.320 | 31.722 | 1.00 | 2.00 | 6 |
| ATOM | 1505 | CAL | CA2 | 161 | 18.805 | 7.035 | 24.656 | 1.00 | 2.00 | 6 |
| ATOM | 1506 | OH2 | H2O | 402 | 21.434 | 20.339 | 32.504 | 1.00 | 47.68 | 8 |
| ATOM | 1507 | OH2 | H2O | 403 | 15.300 | 0.250 | 14.694 | 1.00 | 33.60 | 8 |
| ATOM | 1508 | OH2 | H2O | 404 | 20.436 | -4.063 | -14.818 | 1.00 | 40.45 | 8 |
| ATOM | 1509 | OH2 | H2O | 405 | 1.159 | 17.556 | 10.010 | 1.00 | 31.59 | 8 |
| ATOM | 1510 | OH2 | H2O | 406 | 12.037 | -8.321 | 13.469 | 1.00 | 31.85 | 8 |
| ATOM | 1511 | OH2 | H2O | 407 | 8.509 | 4.869 | 11.562 | 1.00 | 51.85 | 8 |

# Fig. 45

| | | | | | | | | | | |
|---|---|---|---|---|---|---|---|---|---|---|
| ATOM | 1512 | OH2 | H2O | 408 | 9.986 | 14.015 | 34.602 | 1.00 | 51.06 | 8 |
| ATOM | 1513 | OH2 | H2O | 409 | 16.720 | 19.002 | 11.704 | 1.00 | 39.52 | 8 |
| ATOM | 1514 | OH2 | H2O | 410 | 16.356 | 15.524 | 38.130 | 1.00 | 41.59 | 8 |
| ATOM | 1515 | OH2 | H2O | 411 | 13.439 | 14.895 | 38.525 | 1.00 | 40.80 | 8 |
| ATOM | 1516 | OH2 | H2O | 412 | 16.154 | -3.224 | -16.212 | 1.00 | 46.69 | 8 |
| ATOM | 1517 | OH2 | H2O | 413 | 13.854 | 4.292 | -12.349 | 1.00 | 43.55 | 8 |
| ATOM | 1518 | OH2 | H2O | 414 | 8.118 | 11.480 | -6.686 | 1.00 | 35.88 | 8 |
| ATOM | 1519 | OH2 | H2O | 415 | 22.285 | 13.391 | 27.604 | 1.00 | 60.33 | 8 |
| ATOM | 1520 | OH2 | H2O | 416. | -3.869 | 17.803 | 14.240 | 1.00 | 45.89 | 8 |
| ATOM | 1521 | OH2 | H2O | 417 | 6.922 | 2.652 | 7.041 | 1.00 | 45.49 | 8 |
| ATOM | 1522 | OH2 | H2O | 418 | 20.868 | -1.978 | -20.340 | 1.00 | 43.38 | 8 |
| ATOM | 1523 | OH2 | H2O | 419 | 9.199 | 12.143 | 10.557 | 1.00 | 47.26 | 8 |
| ATOM | 1524 | OH2 | H2O | 420 | 8.611 | 11.117 | -3.958 | 1.00 | 38.53 | 8 |
| ATOM | 1525 | OH2 | H2O | 421 | 2.837 | 25.586 | 23.040 | 1.00 | 54.91 | 8 |
| ATOM | 1526 | OH2 | H2O | 422 | 14.175 | 4.586 | 14.315 | 1.00 | 56.14 | 8 |
| ATOM | 1527 | OH2 | H2O | 423 | 13.085 | 19.959 | 11.940 | 1.00 | 41.54 | 8 |
| ATOM | 1528 | OH2 | H2O | 424 | -0.488 | 14.120 | 26.863 | 1.00 | 40.91 | 8 |
| ATOM | 1529 | OH2 | H2O | 425 | 21.451 | 15.804 | 26.318 | 1.00 | 37.08 | 8 |
| ATOM | 1530 | OH2 | H2O | 426 | 7.884 | 27.379 | 14.850 | 1.00 | 33.39 | 8 |
| ATOM | 1531 | OH2 | H2O | 427 | 8.880 | 14.219 | 7.669 | 1.00 | 46.74 | 8 |
| ATOM | 1532 | OH2 | H2O | 428 | -3.541 | 2.433 | 9.798 | 1.00 | 56.39 | 8 |
| ATOM | 1533 | OH2 | H2O | 429 | 4.079 | 5.937 | 16.544 | 1.00 | 62.77 | 8 |
| ATOM | 1534 | OH2 | H2O | 430 | 25.753 | 17.631 | 17.948 | 1.00 | 61.57 | 8 |
| ATOM | 1535 | OH2 | H2O | 431 | 15.786 | 14.241 | 6.279 | 1.00 | 51.22 | 8 |
| ATOM | 1536 | OH2 | H2O | 432 | 15.762 | 1.923 | 19.885 | 1.00 | 46.67 | 8 |
| ATOM | 1537 | OH2 | H2O | 433 | 17.718 | -8.139 | -0.591 | 1.00 | 48.47 | 8 |
| ATOM | 1538 | OH2 | H2O | 434 | 29.681 | -3.374 | 4.485 | 1.00 | 53.32 | 8 |
| ATOM | 1539 | OH2 | H2O | 435 | 17.229 | 18.740 | 9.243 | 1.00 | 44.47 | 8 |
| ATOM | 1540 | OH2 | H2O | 436 | 19.065 | 16.497 | -12.413 | 1.00 | 44.17 | 8 |
| ATOM | 1541 | OH2 | H2O | 437 | 6.533 | -2.928 | 5.126 | 1.00 | 48.68 | 8 |
| ATOM | 1542 | OH2 | H2O | 438 | 23.754 | 12.602 | -4.422 | 1.00 | 41.59 | 8 |
| ATOM | 1543 | OH2 | H2O | 439 | 8.909 | 0.483 | -16.287 | 1.00 | 42.36 | 8 |
| ATOM | 1544 | OH2 | H2O | 440 | 21.786 | -9.965 | -10.304 | 1.00 | 56.63 | 8 |
| ATOM | 1545 | OH2 | H2O | 441 | 28.886 | 8.764 | -6.641 | 1.00 | 44.22 | 8 |
| ATOM | 1546 | OH2 | H2O | 442 | 25.607 | 12.366 | -11.360 | 1.00 | 37.30 | 8 |
| ATOM | 1547 | OH2 | H2O | 443 | 22.291 | 22.968 | 12.360 | 1.00 | 35.81 | 8 |
| ATOM | 1548 | OH2 | H2O | 444 | 13.882 | 30.988 | 14.211 | 1.00 | 47.00 | 8 |
| ATOM | 1549 | OH2 | H2O | 445 | 5.574 | 21.933 | 31.964 | 1.00 | 36.98 | 8 |
| ATOM | 1550 | OH2 | H2O | 446 | 12.312 | 0.716 | 15.997 | 1.00 | 45.92 | 8 |
| ATOM | 1551 | OH2 | H2O | 447 | 6.404 | 4.429 | 29.230 | 1.00 | 50.39 | 8 |

Fig. 46

| | | | | | | | | | | |
|---|---|---|---|---|---|---|---|---|---|---|
| ATOM | 1552 | OH2 | H2O | 448 | 31.554 | 9.583 | -1.702 | 1.00 | 66.74 | 8 |
| ATOM | 1553 | OH2 | H2O | 449 | -4.186 | 13.559 | 16.931 | 1.00 | 54.39 | 8 |
| ATOM | 1554 | OH2 | H2O | 450 | 7.176 | 20.335 | 3.070 | 1.00 | 45.94 | 8 |
| ATOM | 1555 | OH2 | H2O | 451 | -3.812 | 16.721 | 24.738 | 1.00 | 45.94 | 8 |
| ATOM | 1556 | OH2 | H2O | 452 | 8.260 | 16.834 | 8.234 | 1.00 | 50.21 | 8 |
| ATOM | 1557 | OH2 | H2O | 453 | 25.702 | -5.761 | -7.120 | 1.00 | 46.53 | 8 |
| ATOM | 1558 | OH2 | H2O | 454 | 14.878 | 0.083 | 17.948 | 1.00 | 59.01 | 8 |
| ATOM | 1559 | OH2 | H2O | 455 | 29.572 | 0.922 | -2.443 | 1.00 | 53.04 | 8 |
| ATOM | 1560 | OH2 | H2O | 456 | 13.931 | -9.603 | -2.018 | 1.00 | 46.88 | 8 |
| ATOM | 1561 | OH2 | H2O | 457 | 6.881 | 1.203 | -14.036 | 1.00 | 51.00 | 8 |
| ATOM | 1562 | OH2 | H2O | 458 | 9.709 | 25.205 | 16.269 | 1.00 | 38.77 | 8 |
| ATOM | 1563 | OH2 | H2O | 459 | 28.609 | -2.440 | -4.898 | 1.00 | 51.27 | 8 |
| ATOM | 1564 | OH2 | H2O | 460 | 21.144 | 13.102 | -3.025 | 1.00 | 40.18 | 8 |
| ATOM | 1565 | OH2 | H2O | 461 | 7.943 | 6.305 | 5.294 | 1.00 | 43.25 | 8 |
| ATOM | 1566 | OH2 | H2O | 462 | 18.492 | 17.296 | 13.251 | 1.00 | 46.62 | 8 |
| ATOM | 1567 | OH2 | H2O | 463 | 17.640 | -1.627 | -20.591 | 1.00 | 39.89 | 8 |
| ATOM | 1568 | OH2 | H2O | 464 | 6.565 | 7.288 | 2.462 | 1.00 | 47.92 | 8 |
| ATOM | 1569 | OH2 | H2O | 465 | 10.328 | 10.891 | 37.435 | 1.00 | 56.02 | 8 |
| ATOM | 1570 | OH2 | H2O | 466 | 11.846 | 22.232 | 0.831 | 1.00 | 48.01 | 8 |
| ATOM | 1571 | OH2 | H2O | 467 | 11.258 | 27.340 | 17.664 | 1.00 | 34.07 | 8 |
| ATOM | 1572 | OH2 | H2O | 468 | 16.457 | 11.320 | -10.224 | 1.00 | 47.65 | 8 |
| ATOM | 1573 | OH2 | H2O | 469 | 8.282 | 8.824 | 3.936 | 1.00 | 45.65 | 8 |
| ATOM | 1574 | OH2 | H2O | 470 | 7.579 | 14.421 | 10.131 | 1.00 | 47.53 | 8 |
| ATOM | 1575 | OH2 | H2O | 471 | 14.685 | -1.128 | -18.599 | 1.00 | 46.26 | 8 |
| ATOM | 1576 | OH2 | H2O | 472 | 4.242 | 0.218 | -14.721 | 1.00 | 51.45 | 8 |
| ATOM | 1577 | OH2 | H2O | 473 | -0.073 | 24.190 | 26.842 | 1.00 | 72.56 | 8 |
| ATOM | 1578 | OH2 | H2O | 474 | 15.303 | 8.537 | -5.659 | 1.00 | 67.01 | 8 |
| ATOM | 1579 | OH2 | H2O | 475 | 6.058 | 10.179 | 22.696 | 1.00 | 72.61 | 8 |
| ATOM | 1580 | OH2 | H2O | 476 | -1.848 | -4.081 | 2.350 | 1.00 | 69.42 | 8 |
| ATOM | 1581 | OH2 | H2O | 477 | 3.081 | 21.176 | 26.354 | 1.00 | 58.94 | 8 |
| ATOM | 1582 | OH2 | H2O | 478 | 17.220 | 29.562 | 10.821 | 1.00 | 67.03 | 8 |
| ATOM | 1583 | OH2 | H2O | 479 | 21.792 | 23.038 | 8.845 | 1.00 | 74.48 | 8 |
| ATOM | 1584 | OH2 | H2O | 480 | 8.362 | 24.067 | 4.621 | 1.00 | 66.61 | 8 |
| ATOM | 1585 | OH2 | H2O | 481 | 6.127 | 35.122 | 30.044 | 1.00 | 52.00 | 8 |
| ATOM | 1586 | OH2 | H2O | 482 | 22.342 | 30.927 | 19.913 | 1.00 | 75.03 | 8 |
| ATOM | 1587 | OH2 | H2O | 483 | 7.015 | 22.843 | 26.787 | 1.00 | 81.17 | 8 |
| ATOM | 1588 | OH2 | H2O | 484 | 19.425 | 20.640 | 6.559 | 1.00 | 62.59 | 8 |
| ATOM | 1589 | OH2 | H2O | 485 | 4.508 | 37.458 | 23.211 | 1.00 | 68.92 | 8 |
| ATOM | 1590 | OH2 | H2O | 486 | 24.768 | 4.994 | 23.879 | 1.00 | 75.79 | 8 |
| ATOM | 1591 | OH2 | H2O | 487 | 0.956 | 6.426 | 15.758 | 1.00 | 63.84 | 8 |

F i g. 4 7

```
ATOM   1592  OH2 H2O    488      19.443   3.465  31.772  1.00 73.90   8
ATOM   1593  OH2 H2O    489      12.182  30.561  17.821  1.00 68.90   8
ATOM   1594  OH2 H2O    490       7.333   3.558  31.674  1.00 64.91   8
END
```

# INTERNATIONAL SEARCH REPORT

| International application No. |
| --- |
| PCT/JP98/06022 |

**A. CLASSIFICATION OF SUBJECT MATTER**
Int.Cl⁶ C07K14/47, G01N33/15, G01N33/68, C12N15/12, C12P21/02

According to International Patent Classification (IPC) or to both national classification and IPC

**B. FIELDS SEARCHED**

Minimum documentation searched (classification system followed by classification symbols)
Int.Cl⁶ C07K14/47, G01N33/15, G01N33/68, C12N15/12, C12P21/02

Documentation searched other than minimum documentation to the extent that such documents are included in the fields searched

Electronic data base consulted during the international search (name of data base and, where practicable, search terms used)
WPI (DIALOG), BIOSIS (DIALOG)

**C. DOCUMENTS CONSIDERED TO BE RELEVANT**

| Category* | Citation of document, with indication, where appropriate, of the relevant passages | Relevant to claim No. |
| --- | --- | --- |
| X | SAIJO, Y. et al., "Production, crystallization, and preliminary X-ray analysis of rabbit skeletal muscle troponin complex consisting of troponin C and fragment (1-47) of troponin I", Protein Sci. (1997. Apr) Vol. 6, No. 4, p.916-918 | 1-21 |
| P, X | VASSYLYEV, D.G. et al., "Crystal structure of troponin C in complex with troponin I fragment at 2.3-Å resolution", Proc. Natl. Acad. Sci. USA (1998. Apr) Vol. 95, No, 9, p.4847-4852 | 1-21 |
| T | VASSYLYEV, D.G. et al., "The crystal structure of troponin C in complex with N-terminal fragment of troponin I. The mechanism of how the inhibitory action of troponin I is released by Ca²⁺-binding to troponin C", Adv. Exp. Med. Biol. (1998) Vol. 453, p.157-167 | 1-21 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
| --- | --- |
| 25 March, 1999 (25. 03. 99) | 6 April, 1999 (06. 04. 99) |

| Name and mailing address of the ISA/ Japanese Patent Office | Authorized officer |
| --- | --- |
| Facsimile No. | Telephone No. |

Form PCT/ISA/210 (second sheet) (July 1992)

INTERNATIONAL SEARCH REPORT

International application No.

PCT/JP98/06022

| C (Continuation). DOCUMENTS CONSIDERED TO BE RELEVANT | | |
|---|---|---|
| Category* | Citation of document, with indication, where appropriate, of the relevant passages | Relevant to claim No. |
| Y | PEARLSTONE, J.R. et al., "Interactions of structural C and regulatory N domains of troponin C with repeated sequence motifs in troponin I", Biochemistry (1997. Jun) Vol. 36, No. 24, p.7601-7606 | 6-21 |
| P, A | MCKAY, R.T. et al., "Structure and interaction site of the regulatory domain of troponin-C when complexed with the 96-148 region of troponin-I", Biochemistry (1998. Sep) Vol. 37, No. 36, p.12419-12430 | 1-21 |
| P, A | US, 5834210, A (Spectral Diagnostics, Inc.), 11 November, 1998 (11. 11. 98) (Family: none) | 1-21 |
| A | TAKEDA, S. et al., "Structural and functional domains of the troponin complex revealed by limited digestion", Eur. J. Biochem. (1997. Jun) Vol. 246, No. 3, p.611-617 | 1-21 |

Form PCT/ISA/210 (continuation of second sheet) (July 1992)